# EUROPEAN PATENT APPLICATION

(11) **EP 4 487 871 A1**
(43) Date of publication of application: **08.01.2025**
(21) Application number: 23762993.6
(22) Date of filing: 03.03.2023
(51) Int. Cl.: A61K 47/18, A61K 9/127, C07C 323/12, C07C 319/22

(54) **DEGRADABLE LIPOSOME FOR ACTIVE MOLECULE DELIVERY AND NANOCOMPOSITE THEREOF**

(30) Priority: 04.03.2022 CN 202210214110
(71) Applicant: BEIJING CARRIUS BIO LTD., Beijing 100094 (CN)
(72) Inventor: WANG, Ming, Beijing 100094 (CN); CAI, Weiqi, Beijing 100094 (CN)
(74) Representative: Mewburn Ellis LLP
(86) International application number: PCT/CN2023/079515
(87) International publication number: WO 2023/165595

(57) **Abstract**

The present invention relates to a lipid compound for forming a nanodrug carrier. The lipid compound can form a nanocomplex that selectively and efficiently delivers a pharmaceutically active molecule, and reduce or minimize treatment-associated toxicity. The present invention also relates to a nanocomplex comprising the lipid compound and a pharmaceutically active molecule, and a pharmaceutical composition comprising the nanocomplex.

## Description

### Technical Field

The present invention relates to a degradable lipid compound for delivering an active molecule, preferably a lipid compound for delivering a biologically active molecule. The lipid compound can efficiently deliver active molecules, including but not limited to nucleic acids, proteins, small molecule drugs, etc., and reduce or minimize treatment-associated toxicity. The present invention relates to a method for delivering a nucleic acid by using the degradable lipid compound or the use of the degradable lipid compound in the delivery of a nucleic acid. The present invention further relates to a nanocomplex comprising the lipid compound and an active molecule, and a pharmaceutical composition comprising the nanocomplex.

### Background Art

Targeted delivery of a drug can improve the therapeutic selectivity of the drug towards diseased cells, and minimize the toxicity of delivery carriers and bioactive molecules to normal cells, the toxic and side effects of the drug, etc. Biological macromolecular drugs, such as nucleic acids and proteins, have presented broad application prospects in the treatment and/or prevention of major and difficult diseases. However, biological macromolecular drugs easily degrade and cannot enter mammalian cells autonomously, so novel drug delivery technologies are very important for biotherapeutic applications thereof. Developing novel drug delivery carriers to introduce a biological macromolecular drug efficiently and safely into target cells and allow for enrichment in lesion tissues is very important to improve the therapeutic effect of the biological macromolecular drug. For example, a messenger RNA (mRNA) needs to enter the cytoplasm of a target cell for translation into a functional protein. However, mRNA easily degrades, has a large molecular weight and strong hydrophilicity, and cannot enter cells autonomously. Therefore, the biotherapeutic application of an mRNA drug depends on a safe, effective and stable delivery system to protect the nucleic acid from degradation, promote the cellular uptake of the mRNA, and control the efficient and selective release of the mRNA from the carrier in cells. Viral vectors, which have been widely concerned and applied in gene drug delivery and gene therapies, are one of carriers for delivering biological macromolecular drugs. However, viral vectors have problems in the delivery of gene drugs, such as limited capacity of packageable gene drugs, strong immunogenicity, and cytotoxicity. Nanomaterials, such as non-viral vectors, have been widely used in the delivery of biological macromolecular drugs due to their low toxicity, high delivery efficiency, etc., and lipids, inorganic nanomaterials, metal-organic framework materials, macromolecular polymers, proteins, peptides, etc. have all been used in the delivery of nucleic acid and protein drugs. Lipids have been widely used in the delivery of biological macromolecules such as DNAs and RNAs due to their excellent delivery efficiency and good biocompatibility. At present, there have been several lipid preparations that have been approved in clinical application for delivering small interfering RNAs, among which mRNA vaccines encapsulated by lipid nanoparticles have been used to prevent novel coronavirus infection. Lipids encapsulating nucleic acids generally enter cells through endocytosis and need to further escape from lysosomes to release the nucleic acids into cytoplasm to express corresponding proteins. At present, it has been found that there are two main mechanisms of lysosomal escape for lipid nanoparticles: one is that lipids interact with the lysosomal membrane, which makes the lysosomal membrane unstable; and the other is that lipids act as proton sponges, so that for the sake of charge balance and concentration balance, negative ions and water flow in the lipids in large quantities, resulting in lysosome swelling and rupture.

It is very important to efficiently regulate and control the release of biological macromolecular drugs in target cells to improve the efficiency of drug delivery and biotherapy. Therefore, by developing a delivery carrier suitable for novel bioactive molecules, especially nucleic acids, which can not only more efficiently deliver bioactive molecules to targets, such as target cells or target organs, to protect normal tissues or cells from dosage-caused damage, but can also release drugs in diseased cells or lesion tissues efficiently, the delivery efficiency of biological macromolecular drugs will be further improved, and by developing targeted treatment strategies, the risk of treatment-associated toxicity will be reduced or minimized.

### Summary of the Invention

The present invention provides a nanodrug carrier, which is a degradable lipid compound based on a response to reactive oxygen species (ROS) and can deliver a pharmaceutically active molecule to a target diseased cell more efficiently and selectively. The nanodrug carrier not only improves the efficiency of the release of the pharmaceutically active molecule in the diseased cell, but also further reduces the drug toxicity to normal cells by targeting lesion tissues.

In a first aspect, the present invention provides a lipid compound of formula (I), 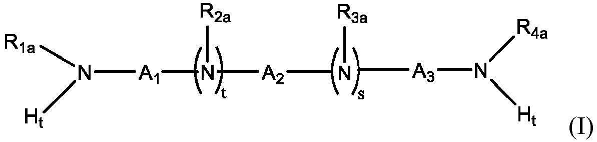 wherein
R₁ₐ, R₂ₐ, R₃ₐ, and R₄ₐ are each independently hydrogen, a monovalent aliphatic group, a monovalent heteroaliphatic group, a monovalent aromatic group, a monovalent heteroaromatic group, or Ht;
t and s are each independently 0 or 1, and when t or s is zero, it means that the part is directly a single bond;
A₁, A₂, and A₃ are each independently a single bond, a divalent aliphatic group, a divalent heteroaliphatic group, a divalent aromatic group, or a divalent heteroaromatic group, or a combination of two of the above;
each Ht is independently at each occurrence -R₁-X-R₂-**Y**-R₃-**Z**-R₄,
   wherein
   each R₁ is independently at each occurrence a divalent aliphatic group, a divalent heteroaliphatic group, a divalent aromatic group, or a divalent heteroaromatic group;
   each X is independently at each occurrence 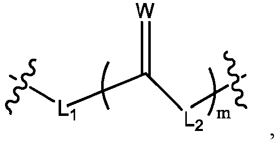 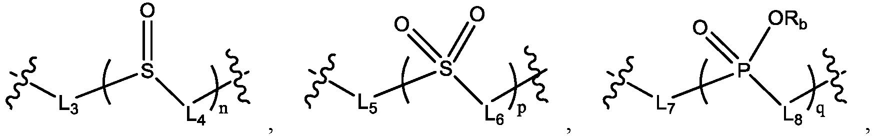 or 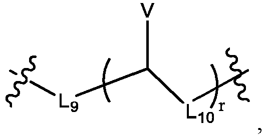 wherein
      m, n, p, q, and r are each independently 1-6;
      W is O, S, or NR_{c};
      L₁, L₃, L₅, L₇, and L₉ are directly connected to R₁ or R₂ and are each independently a single bond, O, S, or NR_{d};
      L₂, L₄, L₆, L₈, and L₁₀ are each independently a bond, O, S, or NRₑ;
      V is an aliphatic group, OR_{f}, SR_{g}, or NRₕRᵢ,
      wherein R_{b}, R_{c}, R_{d}, Rₑ, R_{f}, R_{g}, Rₕ, and Rᵢ are each independently hydrogen, hydroxyl, an oxyaliphatic group, a monovalent aliphatic group, a monovalent heteroaliphatic group, a monovalent aromatic group, or a monovalent heteroaromatic group;
   Y and Z are each independently at each occurrence S or O;
   each R₂ is independently at each occurrence a single bond, a divalent aliphatic group,
a divalent heteroaliphatic group, a divalent aromatic group, or a divalent heteroaromatic group;
   each R₃ is independently at each occurrence a single bond, a divalent aliphatic group, a divalent heteroaliphatic group, a divalent aromatic group, or a divalent heteroaromatic group; and
   each R₄ is independently at each occurrence a hydrophobic group;
and N atoms in the backbone structure of formula (I) are optionally cationized;
provided that at least one of R₁ₐ and R₄ₐ is hydrogen; and
provided that the compound is not one of the following compounds: 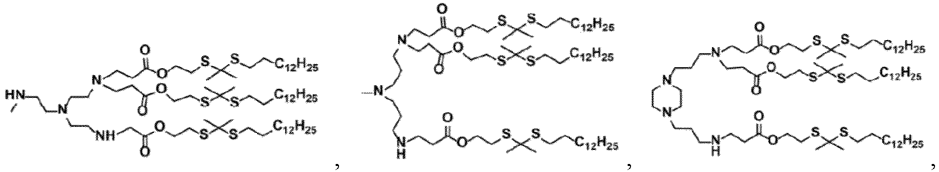 or

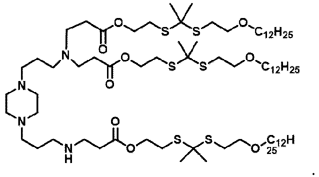

In some embodiments, the lipid compound of formula (I) is in an ionizable form, that is, one or more N atoms in the backbone structure of formula (I) can be ionized. In a further embodiment, the lipid compound of formula (I) is in an ionizable form, thereby forming the following structures: 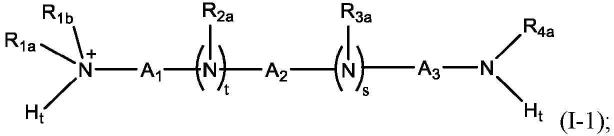 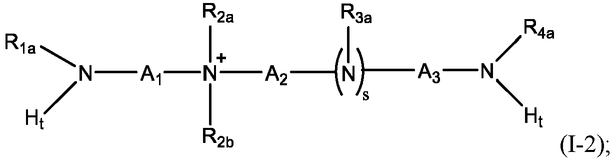 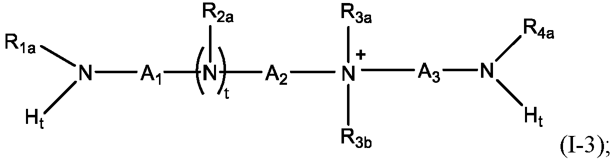 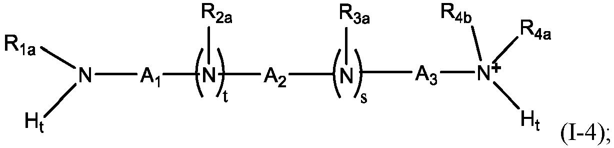 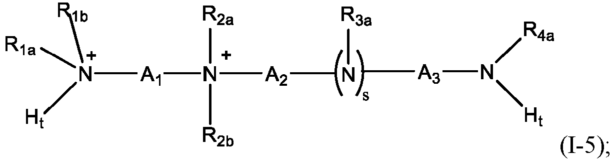 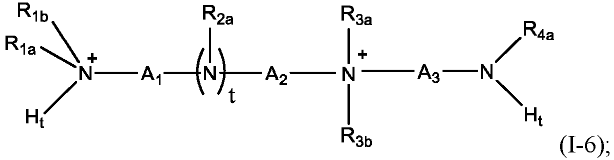 and 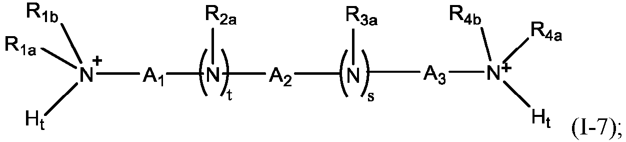 and contains corresponding counter ions, wherein R_{1b}, R_{2b}, R_{3b}, and R_{4b} are each independently hydrogen, a monovalent aliphatic group, a monovalent heteroaliphatic group, a monovalent aromatic group, a monovalent heteroaromatic group, or Ht, and the remaining variables are as defined in formula (I). In some embodiments, the counter ion is an anion that forms a salt with the positively charged N atom. Suitable anions include, but are not limited to, chloride ions, bromide ions, iodide ions, sulfate radicals, nitrate radicals, phosphate radicals, citrate radicals, mesylate radicals, trifluoroacetate radicals, acetate radicals, malate radicals, tosylate radicals, tartrate radicals, fumarate radicals, glutamate radicals, glucuronate radicals, lactate radicals, glutarate radicals, maleate radicals, etc.

In some embodiments, R₁ₐ or R₄ₐ is hydrogen; or R₁ₐ and R₄ₐ are each hydrogen.

In some embodiments, t and s are both 0; or t is 0 and s is 1; or t is 1 and s is 0.

In some embodiments, t and s are both 0; R₁ₐ is Ht, and R₄ₐ is hydrogen; or t and s are both 0; and R₁ₐ is hydrogen, and R₄ₐ is Ht.

In some embodiments, R₂ₐ and R₃ₐ, if present, are each independently hydrogen, a monovalent aliphatic group, or a monovalent heteroaliphatic group; preferably hydrogen, a monovalent C₁₋₆ alkyl, or a monovalent C₁₋₆ heteroalkyl, e.g., amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆alkylthio C₁₋₆ alkyl, or mercapto C₁₋₆ alkyl.

In some embodiments, the fragment 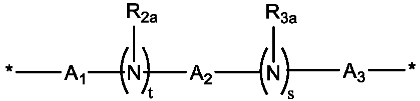 is
- A₁-A₃, wherein A₁ and A₃ are each independently a C₁-C₆ divalent aliphatic group (e.g., a C₁-C₄ divalent aliphatic group, e.g., a C₁-C₆ divalent alkyl, preferably a C₁-C₄ divalent alkyl); or
- A₁-(NR₂ₐ)-A₃, wherein A₁ and A₃ are each independently a C₁-C₆ divalent aliphatic group (e.g., a C₁-C₄ divalent aliphatic group, e.g., a C₁-C₆ divalent alkyl, preferably a C₁-C₄ divalent alkyl), and R₂ₐ is hydrogen, a monovalent aliphatic group, or a monovalent heteroaliphatic group; preferably hydrogen, a monovalent C₁₋₆ alkyl, or a monovalent C₁₋₆ heteroalkyl, e.g., amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆ alkylthio C₁₋₆ alkyl, or mercapto C₁₋₆ alkyl; or
- A₁-(NR₂ₐ)-A₂-(NR_{2b})-A₃, wherein A₁, A₂, and A₃ are each independently a C₁-C₆ divalent aliphatic group (e.g., a C₁-C₄ divalent aliphatic group, e.g., a C₁-C₆ divalent alkyl, preferably a C₁-C₄ divalent alkyl); and R₂ₐ and R_{2b} are each independently hydrogen, a monovalent aliphatic group, or a monovalent heteroaliphatic group; preferably hydrogen, a monovalent C₁₋₆ alkyl, or a monovalent C₁₋₆ heteroalkyl, e.g., amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆alkylthio C₁₋₆ alkyl, or mercapto C₁₋₆ alkyl; or
- A₁-A₂-A₃, wherein A₁ and A₃ are each independently a C₁-C₆ divalent aliphatic group (e.g., a C₁-C₄ divalent aliphatic group, e.g., a C₁-C₆ divalent alkyl, preferably a C₁-C₄ divalent alkyl); and A₂ is a divalent cyclic heteroaliphatic group, such as a 3- to 7-membered monocyclic heteroaliphatic group containing 1-3 heteroatoms selected from oxygen, nitrogen and sulfur, preferably divalent piperazinyl;
wherein the asterisk * represents the connection of the terminal N atom in formula (I) or (I-1) to (I-7).

In some embodiments, the fragment 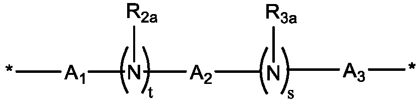 is
- -(CH₂)ₘ₁-, wherein m1 is an integer of 2-12, preferably 2-10 or 3-8; or an integer of 3; or
- -(CH₂)ₘ₂-(NR₂ₐ)-(CH₂)ₘ₃-, wherein m2 and m3 are each independently an integer of 1-6; preferably an integer of 2-6, or 2-4, or 2, or 3; R₂ₐ is hydrogen, a monovalent C₁₋₆ alkyl, amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆ alkylthio C₁₋₆ alkyl, or mercapto C₁₋₆ alkyl; or
- -(CH₂)ₘ₂-(NR₂ₐ)-(CH₂)ₘ₄-(NR_{2b})-(CH₂)ₘ₃, wherein m2, m3, and m4 are each independently an integer of 1-6; preferably an integer of 2-6, or 2-4, or 2, or 3; R₂ₐ and R_{2b} are each independently hydrogen, a monovalent C₁₋₆ alkyl, amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆ alkylthio C₁₋₆ alkyl, or mercapto C₁₋₆ alkyl; or
- -(CH₂)ₘ₂-A₂-(CH₂)ₘ₃, wherein m2 and m3 are each independently an integer of 1-6; preferably an integer of 2-6, or 2-4, or 2, or 3; and A₂ is a divalent cyclic heteroaliphatic group, such as a 3- to 7-membered monocyclic heteroaliphatic group containing 1-3 heteroatoms selected from oxygen, nitrogen and sulfur, preferably divalent piperazinyl;
wherein the asterisk * represents the connection of the terminal N atom in formula (I) or (I-1) to (I-7).

In some embodiments, R₁ is a C₁₋₆ (e.g., C₁₋₄) divalent aliphatic group or a C₁₋₆ (e.g., C₁₋₄) divalent heteroaliphatic group, preferably a C₁₋₄ divalent alkyl or a C₁₋₄ divalent heteroalkyl.

In some embodiments, X is 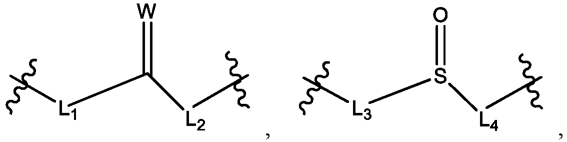 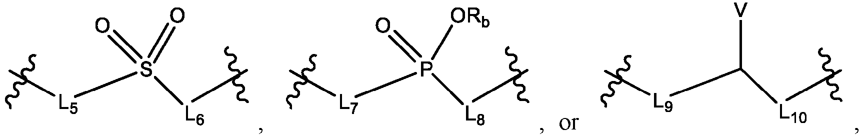 wherein each variable is as defined in formula (I). In a further embodiment, L₁, L₃, L₅, L₇, and L₉ are connected to R₁ and are each independently a single bond, O, S, or NH. In a further embodiment, X is 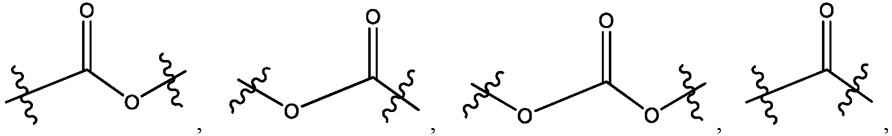 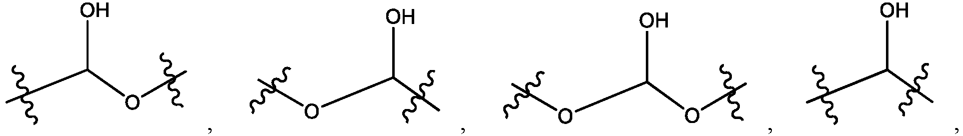 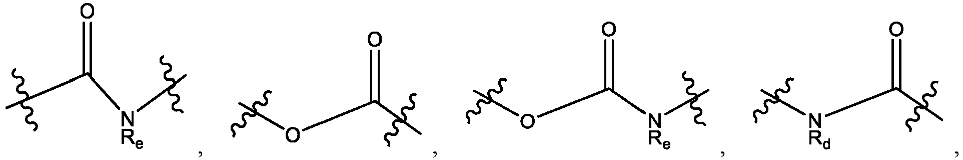 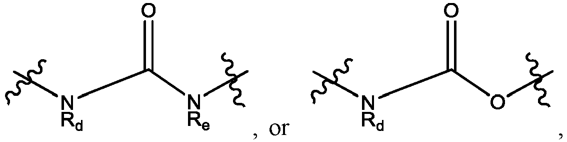 wherein R_{d} and Rₑ are as defined in formula (I). In some embodiments, R^{d} and R^{e} are each independently H or a C₁₋₄ monovalent aliphatic group, preferably H or C₁₋₄ monovalent alkyl.

In some embodiments, Y and Z are both S; or Y is S and Z is O; or Y is O and Z is S; or Y and Z are both O.

In some embodiments, each R₂ is independently at each occurrence a single bond or a C₁₋₆ divalent aliphatic group (e.g., a C₁₋₄ divalent aliphatic group, preferably a C₁₋₄ divalent alkyl, more preferably a C₁₋₂ divalent alkyl).

In some embodiments, each R₃ is independently at each occurrence a single bond or a C₁₋₆ divalent aliphatic group (e.g., a C₁₋₄ divalent aliphatic group, preferably a C₁₋₄ divalent alkyl). In a further embodiment, each R₃ is independently at each occurrence a single bond, or 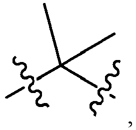 or methylene 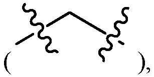 preferably

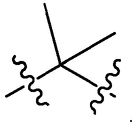

In some embodiments, each R₄ is independently at each occurrence a hydrophobic group selected from long-chain alkyl, alkenyl, aryl, alkylaryl, arylalkyl, arylalkenyl, a cyclic group, an alicyclic group, and a polycyclic group, and optionally having at least one heteroatom selected from nitrogen, oxygen, and sulfur. In a further embodiment, each R₄ is independently at each occurrence C₈-C₃₀ alkyl, C₈-C₃₀ alkenyl, or C₈-C₃₀ alkynyl. In a further embodiment, R₄ is

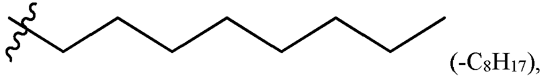

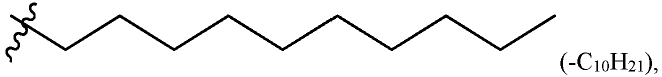

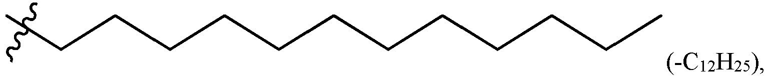

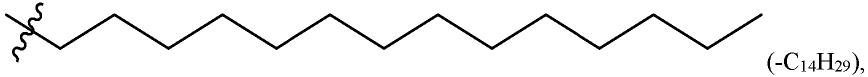

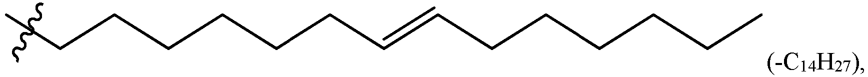

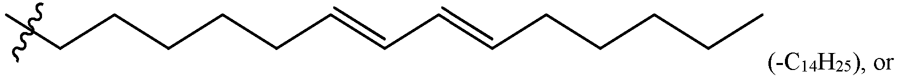

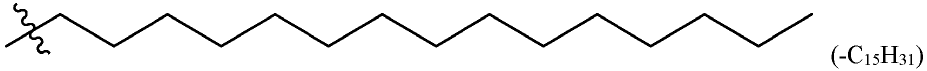

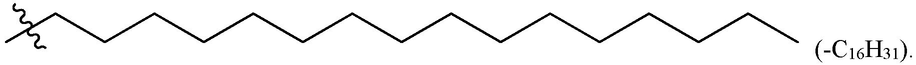

In a further embodiment, each R₄ is independently at each occurrence -(CH₂CH₂O)ₘ-C₈-C₃₀ alkyl, -(CH₂CH₂O)_{y}-C₈-C₃₀ alkenyl, or -(CH₂CH₂O)_{y}-C₈-C₃₀ alkynyl, in which y is 0, or 1, or 2, for example, R₄ is -CH₂CH₂O-C₈H₁₇, -CH₂CH₂O-C₁₀H₂₁, -CH₂CH₂O-C₁₂H₂₅, -CH₂CH₂O-C₁₄H₂₉, or -CH₂CH₂O-C₁₅H₃₁.

In some embodiments, the pKa value of the amine head is greater than 4, preferably greater than 6, and more preferably greater than 8.

In some embodiments, A₁ and A₃ are each independently a single bond or a divalent aliphatic group, and A₂ is a divalent aliphatic group or a divalent heteroaliphatic group. In a further embodiment, A₁ and A₃ are each independently a C₁-C₆ divalent aliphatic group (e.g., a C₁-C₄ divalent aliphatic group, preferably a C₁-C₄ divalent alkyl), and A₂ is a divalent cyclic heteroaliphatic group, such as a 3- to 7-membered monocyclic heteroaliphatic group containing 1-3 heteroatoms selected from oxygen, nitrogen, and sulfur. In a further embodiment, A₁ and A₃ are each independently ethydene, and A₂ is divalent piperazinyl.

In some embodiments, the lipid compound of formula (I) is 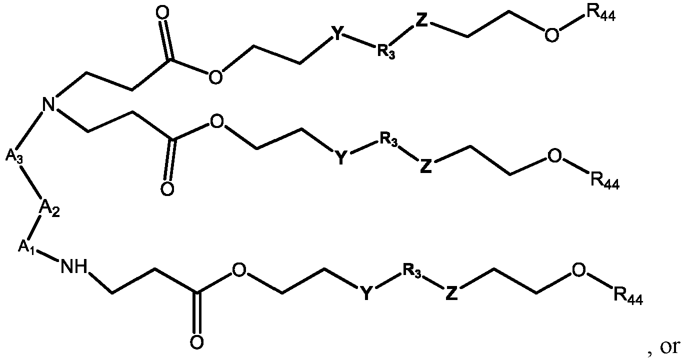 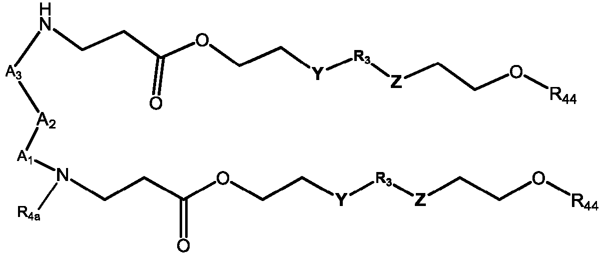 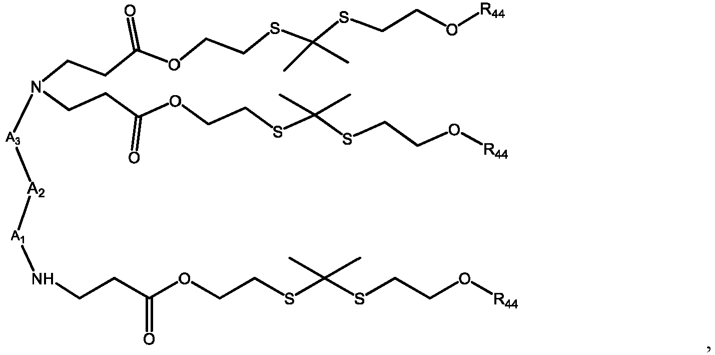 or 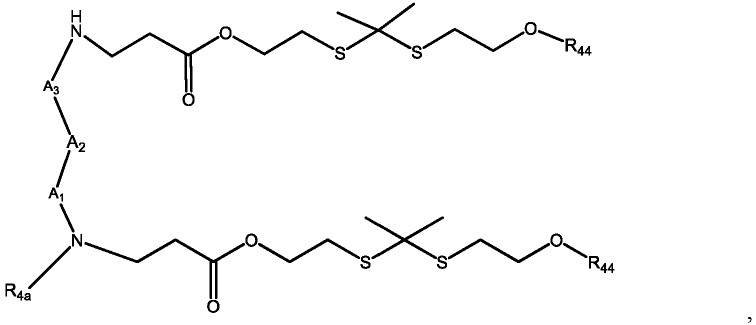 wherein A₁, A₂, A₃, and R₄ₐ are as defined in formula (I), and -(CH₂CH₂O)-R₄₄ is R₄ as defined in formula (I); Y and Z are both S; or Y is S and Z is O; or Y is O and Z is S; or Y and Z are both O; and each R₃ is independently at each occurrence a single bond, or 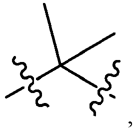 or methylene 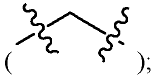 preferably wherein A₁ and A₃ are each independently -(CH₂)ₘ-; A₂ is -(CH₂)ₘ- or a divalent cyclic heteroaliphatic group, such as a 3- to 7-membered monocyclic heteroaliphatic group containing 1-3 heteroatoms selected from oxygen, nitrogen and sulfur, preferably divalent piperazinyl, wherein m is independently at each occurrence an integer from 1 to 6; preferably an integer of 2-6, or 2-4, or 2, or 3. In a further embodiment, each R₄₄ is independently at each occurrence alkyl CᵤH₂ᵤ₊₁ or alkenyl CᵤH₂ᵤ₋₁, wherein u is 8-30, preferably 8, 12, 14, or 16. In a further embodiment, A₂ is a divalent cyclic heteroaliphatic group, preferably divalent piperazinyl, and A₁ and A₃ are each independently C₁₋₄ divalent alkyl, e.g., methylene, ethylene, and propylene.

In some embodiments, the lipid compound of formula (I) is 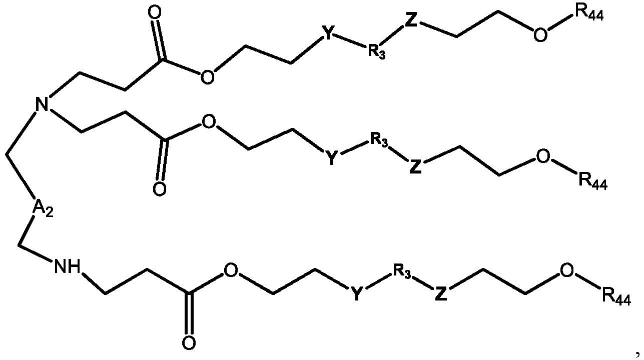 or 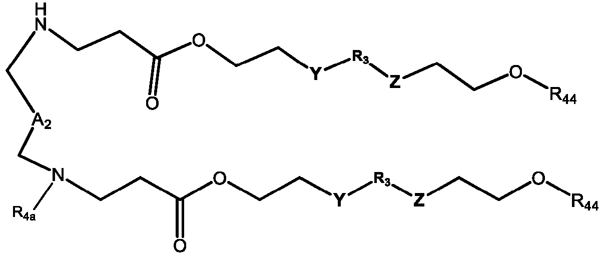 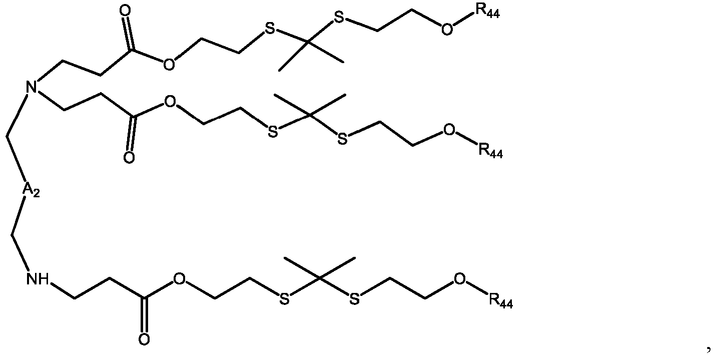 or 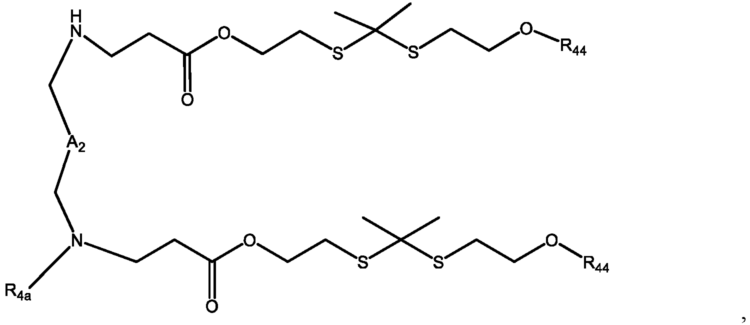 wherein A₂ and R₄ₐ are as defined in formula (I), and -(CH₂CH₂O)-R₄₄ is R₄ as defined in formula (I); Y and Z are both S; or Y is S and Z is O; or Y is O and Z is S; or Y and Z are both O; and each R₃ is independently at each occurrence a single bond, or 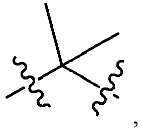 or methylene 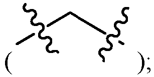 preferably A₂ is -(CH₂)ₘ- or a divalent cyclic heteroaliphatic group, such as a 3-to 7-membered monocyclic heteroaliphatic group containing 1-3 heteroatoms selected from oxygen, nitrogen and sulfur, preferably divalent piperazinyl, wherein m is independently at each occurrence an integer from 1 to 6; preferably an integer of 2-6, or 2-4, or 2, or 3. In a further embodiment, each R₄₄ is independently at each occurrence alkyl CᵤH₂ᵤ₊₁ or alkenyl CᵤH₂ᵤ₋₁, wherein u is 8-30, preferably 8, 12, 14, or 16. In a further embodiment, A₂ is a divalent cyclic heteroaliphatic group, preferably divalent piperazinyl.

In some embodiments, the lipid compound of formula (I) is 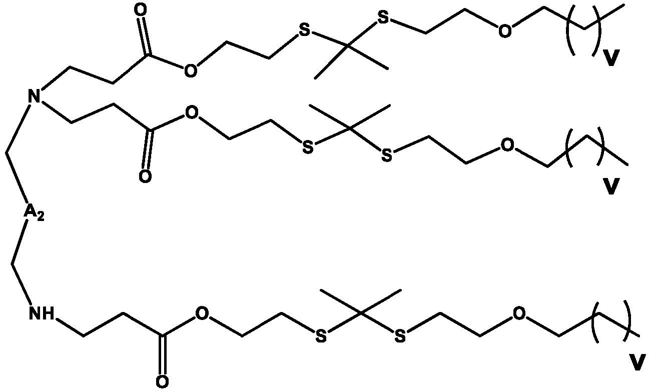 or 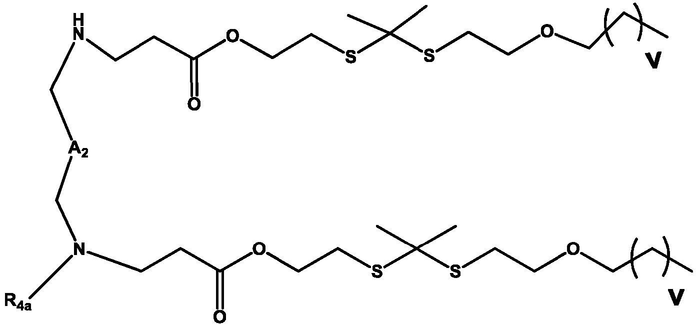 wherein v is 6-28, and preferably v is 10; A₂ and R₄ₐ are as defined in formula (I); preferably A₂ is -(CH₂)ₘ- or a divalent cyclic heteroaliphatic group, such as a 3- to 7-membered monocyclic heteroaliphatic group containing 1-3 heteroatoms selected from oxygen, nitrogen and sulfur, preferably divalent piperazinyl, wherein m is independently at each occurrence an integer from 1 to 6; preferably an integer of 2-6, or 2-4, or 2, or 3. In a further embodiment, A₂ is a divalent cyclic heteroaliphatic group, preferably divalent piperazinyl.

In some embodiments, the lipid compound of formula (I) is 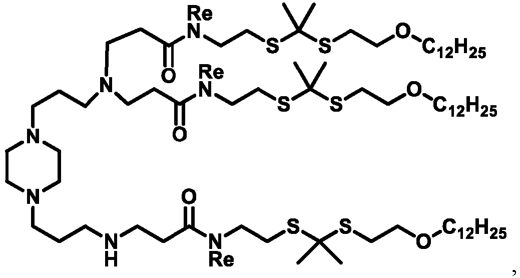 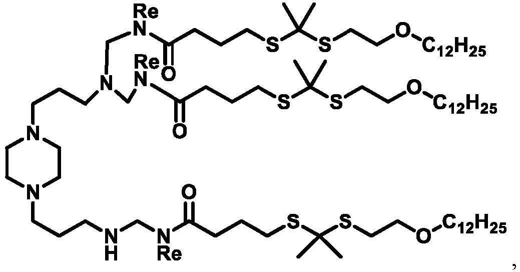 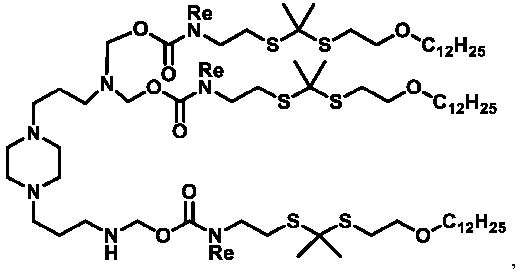 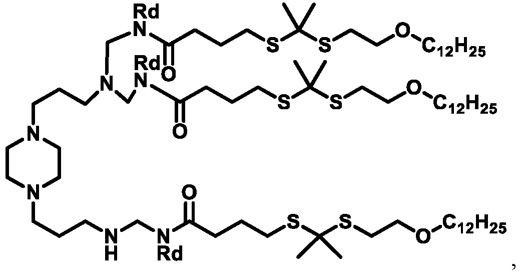 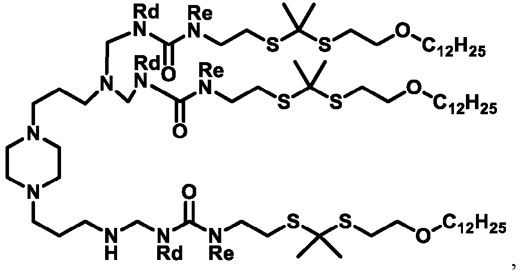 or 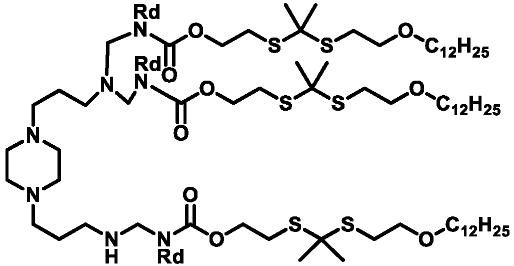 wherein R_{d} and Rₑ are as defined in formula (I), and preferably, each is independently hydrogen or a monovalent aliphatic group, such as C₁₋₄ alkyl.

In some embodiments, exemplary lipid compounds are as follow:

| | |
|---|---|
| TK-2 | 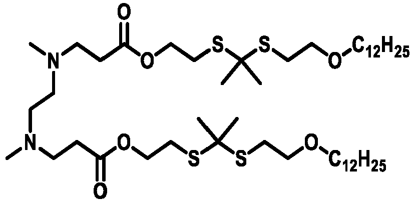 |
| TK-3 | 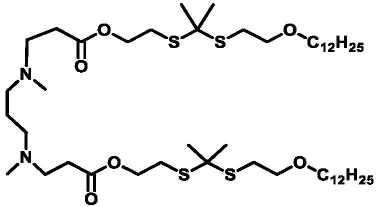 |
| TK-5 | 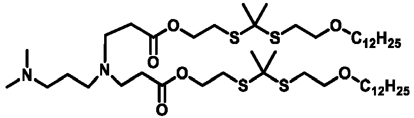 |
| TK-6 | 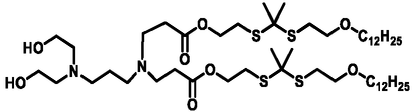 |
| TK-7 | 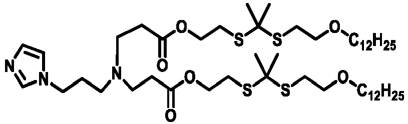 |
| TK-8 | 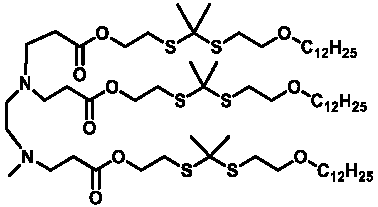 |
| TK-9 | 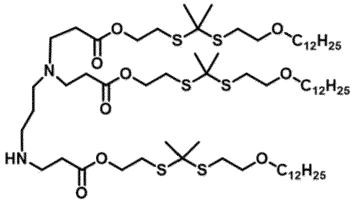 |
| TK-10 | 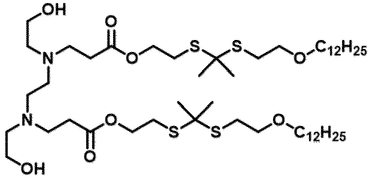 |
| TK-11 | 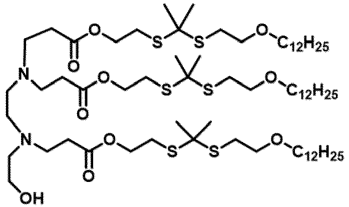 |
| TK-12 | 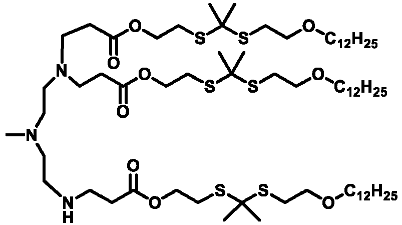 |
| TK-13 | 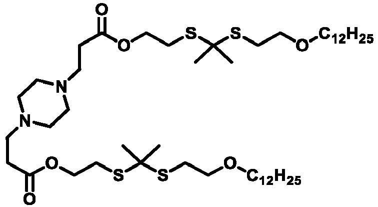 |
| TK-14 | 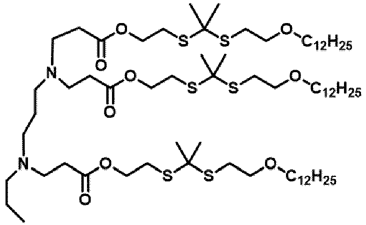 |
| TK-15 | 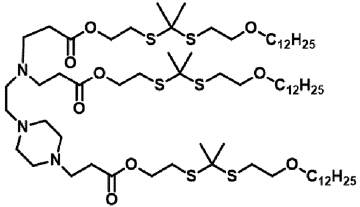 |
| TK-16 | 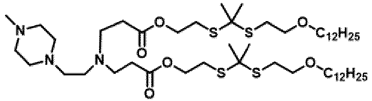 |
| TK-17 | 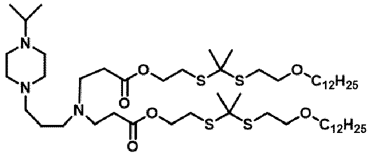 |
| TK-18 | 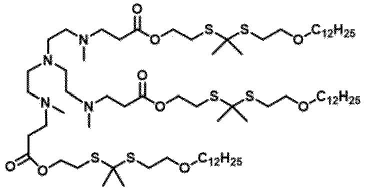 |
| TK-19 | 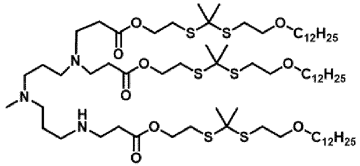 |
| TK-20 | 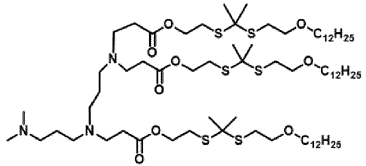 |
| TK-21 (also known as BAmP-TK-12 or TK-12) | 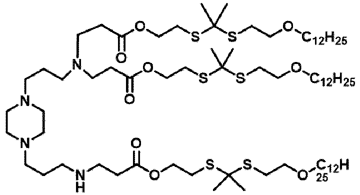 |
| TK-21-4 | 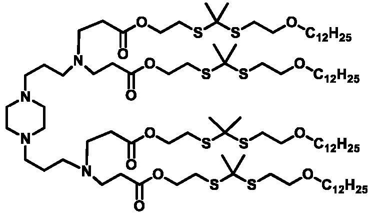 |
| TK-22 | 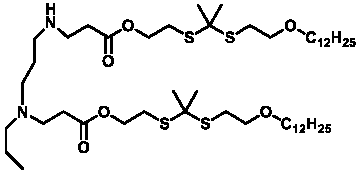 |
| TK-23 | 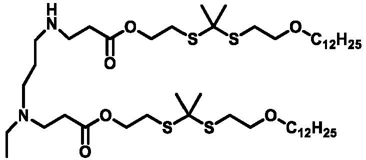 |
| TK-25 | 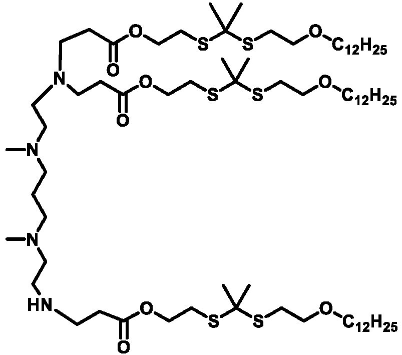 |
| TK-26 | 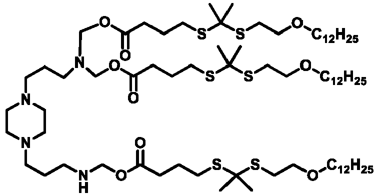 |
| TK-27 | 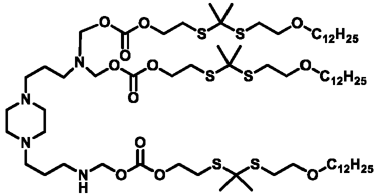 |
| TK-28 | 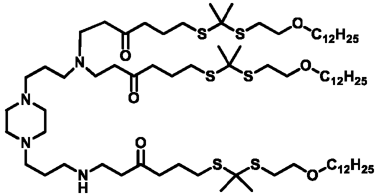 |
| TK-29 | 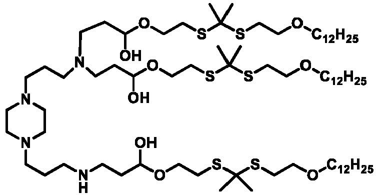 |
| TK-30 | 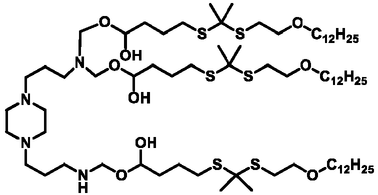 |
| TK-31 | 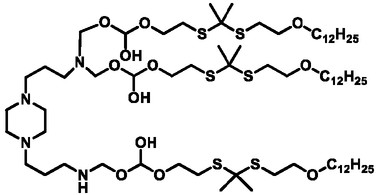 |
| TK-32 | 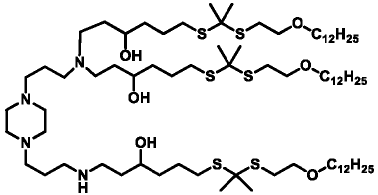 |

The lipid compound of formula (I) of the present invention is biodegradable due to the fact that the Ht group contains a ketal corresponding unit (especially a thioketal corresponding unit when Y and Z are both S). Such a lipid can efficiently bind an mRNA and assemble to form a nanostructure, and after entering a cell, the lipid selectively responds to an oxidative environment in the cell and degrades, thus resulting in efficient release of the mRNA and expression for a corresponding protein. Furthermore, the inventors of the present invention have found that by retaining at least one hydrogen atom on the N atom in the backbone structure of the compound of formula (I) (i.e., not replaced by Ht or other groups), the delivery efficiency is further improved, and the drug can be delivered to the lesion tissue in a targeted manner, thereby reducing dosage-associated toxicities, such as drug toxicity to normal cells and liver toxicity.

In a second aspect, the present invention provides a method for preparing a lipid compound of formula (I) in which R₁ is ethylene, the method comprising: mixing and reacting an acrylic compound of formula (II), i.e., CH₂=CH-X-R₂-**Y**-R₃-**Z**-R₄ (II), in which each variable is as defined in formula (I), with a hydrophilic amine at a molar ratio, wherein the hydrophilic amine contains at least one primary amino group (-NH₂) or at least two secondary amino groups (-NH-), and the molar ratio of the acrylate hydrate of formula (II) to the hydrophilic amine is less than the number of moles of free hydrogen atoms on amino groups in the hydrophilic amine, but greater than 1; for example, the molar ratio of the acrylate hydrate of formula (II) to a hydrophilic amine containing 2 (or 2 moles of) free hydrogen atoms on amino groups is less than 2, such as 1.1-1.8, still for example 1.5; the molar ratio of the acrylate hydrate of formula (II) to a hydrophilic amine containing 3 free hydrogen atoms on amino groups is less than 3, such as 1.1-2.7, still for example 1.5-2.4, yet for example 2.4; the molar ratio of the acrylate hydrate of formula (II) to a hydrophilic amine containing 4 free hydrogen atoms on amino groups is less than 4, e.g., 1.1-3.5, such as 1.1-1.8, or 2.1-3.3, still for example 3.3; and then optionally purifying the product by chromatography to obtain the desired lipid compound of formula (I);
wherein the variables R₂, X, **Y**, R₃, **Z**, and R₄ are as defined in formula (I). In some embodiments, X is 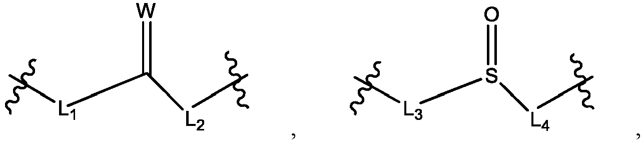 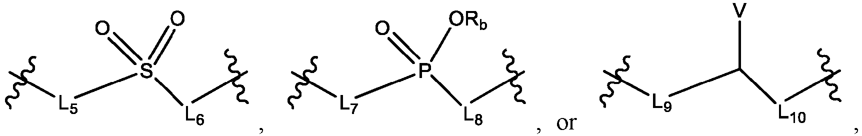 wherein each variable is as defined in formula (I). In a further embodiment, L₁, L₃, L₅, L₇, and L₉ are connected to R₁ and are each independently a single bond, O, S, or NH. In a further embodiment, X is 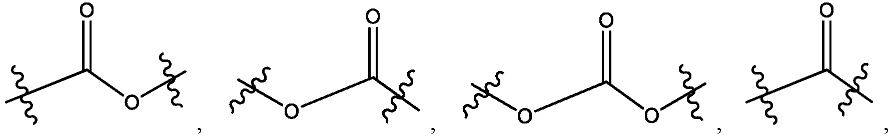 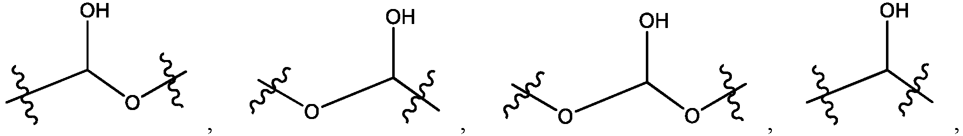 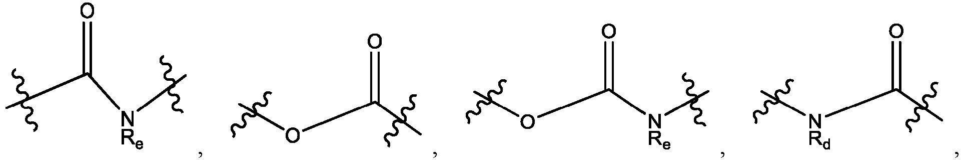 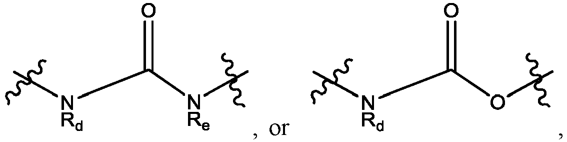 wherein R_{d} and Rₑ are as defined in formula (I). In some embodiments, R^{d} and R^{e} are each independently H or a C₁₋₄ monovalent aliphatic group, preferably H or C₁₋₄ monovalent alkyl.

Specifically, the present invention provides a method for preparing a lipid compound of formula (I) in which R₁ is ethylene and X is -C(O)-O-, the method comprising:
mixing and reacting a ketal-containing acrylate CH₂=CH-C(O)O-R₂-**Y**-R₃-**Z**-R₄, in which each variable is as defined in formula (I), with a hydrophilic amine at a molar ratio, wherein the hydrophilic amine contains at least one primary amino group (-NH₂) or at least two secondary amino groups (-NH-), and the molar ratio of the ketal-containing acrylate to the hydrophilic amine is less than the number of moles of free hydrogen atoms on amino groups in the hydrophilic amine, but greater than 1; for example, the molar ratio of the ketal-containing acrylate to a hydrophilic amine containing 2 (or 2 moles of) free hydrogen atoms on amino groups is less than 2, such as 1.1-1.8, still for example 1.5; the molar ratio of the ketal-containing acrylate to a hydrophilic amine containing 3 free hydrogen atoms on amino groups is less than 3, such as 1.1-2.7, still for example 1.5-2.4, yet for example 2.4; the molar ratio of the ketal-containing acrylate to a hydrophilic amine containing 4 free hydrogen atoms on amino groups is less than 4, e.g., 1.1-3.5, such as 1.1-1.8, or 2.1-3.3, still for example 3.3; and then optionally purifying the product by chromatography to obtain the desired lipid compound of formula (I);
wherein the variables R₂, **Y**, R₃, **Z**, and R₄ are as defined in formula (I).

In some embodiments, in the method, the molar ratio of the ketal-containing acrylate to the hydrophilic amine is 1 or 2 less than the number of moles of free hydrogen atoms on amino groups in the hydrophilic amine, but greater than 1. In some embodiments, the hydrophilic amine contains 2 primary amino groups (-NH₂), that is, it contains 4 free hydrogen atoms on amino groups, and the molar ratio of the ketal-containing acrylate to the hydrophilic amine is 3.3 or less, or 2.4 or less, but greater than 1; or in the method, the hydrophilic amine contains 1 primary amino group (-NH₂) and 1 secondary amino group (-NH-), and the molar ratio of the ketal-containing acrylate to the hydrophilic amine is 2.4 or less, but greater than 1; or in the method, the hydrophilic amine contains 2 secondary amino groups (-NH-), and the molar ratio of the ketal-containing acrylate to the hydrophilic amine is less than 1.5 or less, but greater than 1.

In some embodiments, in method (a), the product is purified by chromatography to obtain the desired lipid compound of formula (I), wherein the chromatographic purification method is well known in the art.

In some embodiments, the pKa value of the amine head is greater than 4, preferably greater than 6, and more preferably greater than 8. The hydrophilic amine in the method is selected from 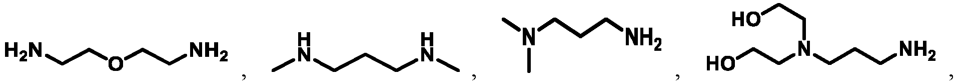 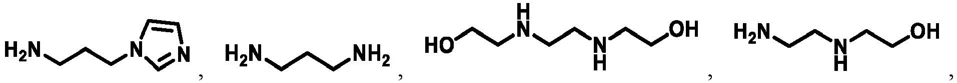 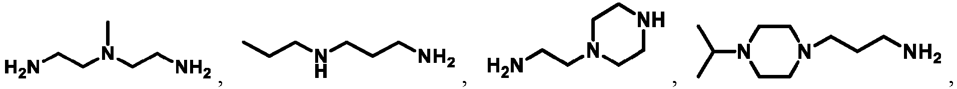 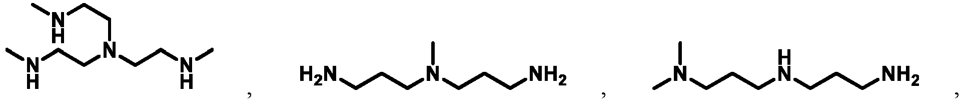 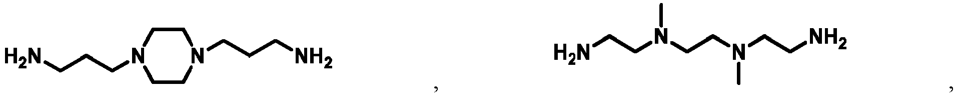 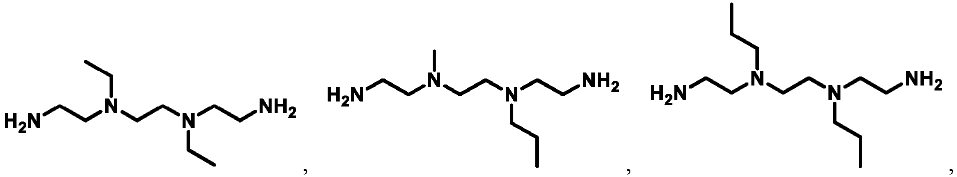 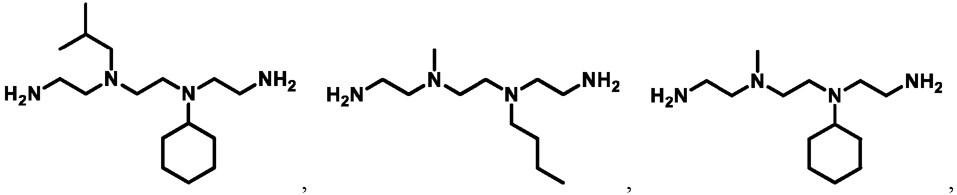 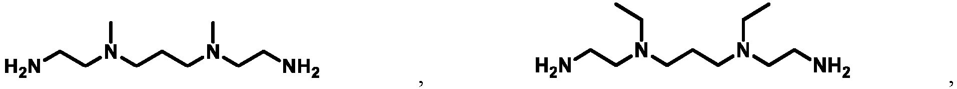 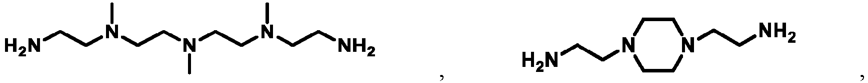 or

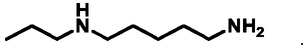

In some embodiments, the ketal-containing acrylate CH₂=CH-C(O)O-R₂-**Y**-R₃-**Z**-R₄ is 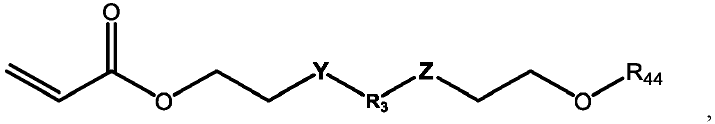 and the remaining variables are as defined in formula (I).

In some embodiments, the hydrophilic amine is 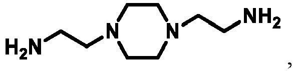 the ketal-containing acrylate is 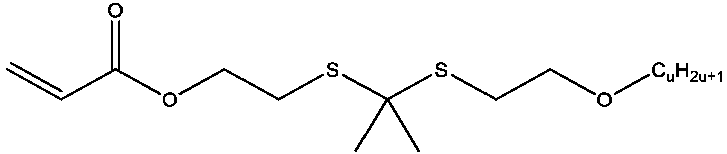 or 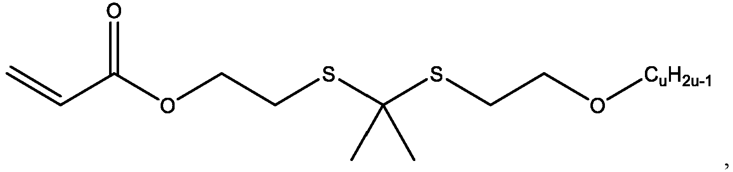 **wherein** CᵤH₂ᵤ₊₁ represents alkyl, CᵤH₂ᵤ₋₁ represents alkenyl, and u is 8-30, preferably 8, 12, 14, or 16. In a specific embodiment, the ketal-containing acrylate is 2-((2-((2-(dodecyloxy)ethyl)thio)propan-2-yl)thio)ethyl acrylate.

In some embodiments, the reaction is an addition reaction, preferably a Michael addition reaction.

In some embodiments, the reaction is carried out at a temperature of 60-85°C.

The ketal-containing acrylate described in the present invention can be synthesized according to a method known in the art, such as a method disclosed in CN 110101665 A. The ketal (thioketone)-containing acrylate is an ester compound obtained by subjecting an acrylic acid and an alcohol compound containing ketal (thioketone) to an esterification reaction.

Other lipid compounds of the present invention can be prepared by the above synthesis routes and other routes known in the art using other suitable starting materials. The methods listed above may include one or more additional steps to add or remove appropriate protecting groups to finally allow for the synthesis of lipid compounds. In addition, various synthesis steps can be carried out in an alternative order or sequence to obtain the desired materials. Synthetic chemical transformation and protective group methods (protection and deprotection) which can be used to synthesize suitable lipid compounds are known in the art.

In a third aspect, the present invention provides a pharmaceutical composition comprising a pharmaceutical carrier and a nanocomplex, wherein the nanocomplex comprises the lipid compound of formula (I) according to the present invention and a pharmaceutically active molecule.

In some embodiments, the pharmaceutically active molecule is a small molecule drug, a protein, a peptide, a nucleic acid, a saccharide, or a combination thereof, preferably a nucleic acid. In a further embodiment, the pharmaceutically active molecule is an mRNA. In a more further embodiment, the mRNA can be a RFP mRNA expressing a red fluorescent protein, a GFP mRNA expressing a green fluorescent protein, or a DUF5 mRNA expressing RAS protease DUF5. In a further embodiment, the pharmaceutically active molecule may also be a plasmid DNA, such as a plasmid DNA expressing GFP.

In some embodiments, the nanocomplex has a particle size of 50 to 500 nm (e.g., 50 to 300 nm and 50 to 180 nm).

In some embodiments, the lipid compound of formula (I) is ionizable.

In some embodiments, the lipid compound and the pharmaceutically active molecule are bound via a non-covalent interaction, a covalent bond or both.

In some embodiments, the nanocomplex further comprises other lipids selected from cholesterol, dioleoylphosphatidylethanolamine (DOPE, CAS: 4004-05-1), and distearoylphosphatidylethanolamine-polyethylene glycol 2000 (DSPE-PEG2000, CAS: 147867-65-0), or a combination thereof. In a further embodiment, the nanocomplex further contains cholesterol, dioleoylphosphatidylethanolamine, and distearoylphosphatidylethanolamine-polyethylene glycol 2000. In one embodiment, the mass ratio of lipid : cholesterol : DOPE : DSPE-PEG2000 in the examples of the present invention is 4 : 1 : 1 : 1 or 4 : 1 : 1 : 8).

In some embodiments, the nanocomplex of the present invention is prepared by separately dissolving the lipid compound of the present invention, cholesterol, and DOPE in trichloromethane to prepare mixed solutions, allowing the mixed solutions to volatilize to form a film, then ultrasonically dissolving the film with anhydrous ethanol, then dropwise adding the dissolved material to a sodium acetate buffer solution containing DSPE-PEG2000, and stirring the solution at a high speed to prepare lipid nanoparticles.

In some embodiments, the pharmaceutical composition of the present invention that comprises the nanocomplex is prepared by mixing pharmaceutically active molecules with the nanocomplex, the mass of which is 5-20 times that of the pharmaceutically active molecules (the range of the N/P ratio of lipid to nucleic acid is 3 : 1-20 : 1) and incubating the mixture. In a further embodiment, the pharmaceutically active molecule is an mRNA.

In a fourth aspect, the present invention provides a method for delivering a nucleic acid, which comprises delivering the nucleic acid to a target cell by using the lipid compound of formula (I) of the present invention to intracellularly release the nucleic acid drug in the target cell; or provides the use of the lipid compound of formula (I) as a drug delivery carrier, wherein the drug is a nucleic acid; or provides the use of a lipid compound of formula (I) as a drug delivery carrier for preparing a nucleic acid drug, wherein the drug delivery carrier delivers the nucleic acid drug to a target cell for the intracellular release of the nucleic acid drug in the target cell.

In some embodiments, the lipid compound of formula (I) is 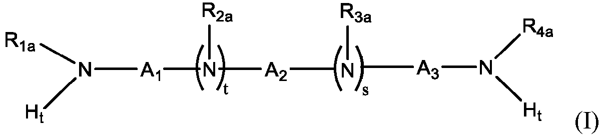 wherein
R₁ₐ, R₂ₐ, R₃ₐ, and R₄ₐ are each independently hydrogen, a monovalent aliphatic group, a monovalent heteroaliphatic group, a monovalent aromatic group, a monovalent heteroaromatic group, or Ht;
t and s are each independently 0 or 1, and when t or s is zero, it means that the part is directly a single bond;
A₁, A₂, and A₃ are each independently a single bond, a divalent aliphatic group, a divalent heteroaliphatic group, a divalent aromatic group, or a divalent heteroaromatic group, or a combination of two of the above;
each Ht is independently at each occurrence -R₁-X-R₂-**Y**-R₃-**Z**-R₄,
   wherein
   each R₁ is independently at each occurrence a divalent aliphatic group, a divalent heteroaliphatic group, a divalent aromatic group, or a divalent heteroaromatic group;
   each X is independently at each occurrence 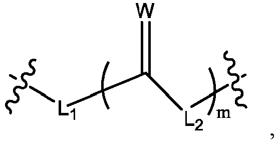 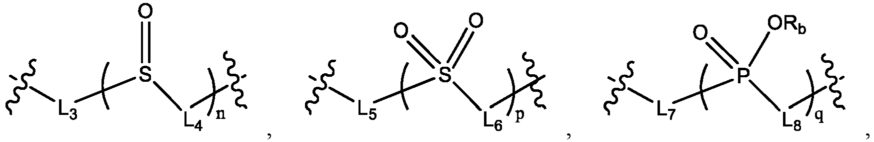 or 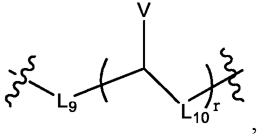 wherein
      m, n, p, q, and r are each independently 1-6;
      W is O, S, or NR_{c};
      L₁, L₃, L₅, L₇, and L₉ are directly connected to R₁ or R₂ and are each independently a single bond, O, S, or NR_{d};
      L₂, L₄, L₆, L₈, and L₁₀ are each independently a bond, O, S, or NRₑ;
      V is an aliphatic group, OR_{f}, SR_{g}, or NRₕRᵢ,
      wherein R_{b}, R_{c}, R_{d}, Rₑ, R_{f}, R_{g}, Rₕ, and Rᵢ are each independently hydrogen, hydroxyl, an oxyaliphatic group, a monovalent aliphatic group, a monovalent heteroaliphatic group, a monovalent aromatic group, or a monovalent heteroaromatic group;
   Y and Z are each independently at each occurrence S or O;
   each R₂ is independently at each occurrence a single bond, a divalent aliphatic group, a divalent heteroaliphatic group, a divalent aromatic group, or a divalent heteroaromatic group;
   each R₃ is independently at each occurrence a single bond, a divalent aliphatic group, a divalent heteroaliphatic group, a divalent aromatic group, or a divalent heteroaromatic group; and
   each R₄ is independently at each occurrence a hydrophobic group;
and N atoms in the backbone structure of formula (I) are optionally cationized;
provided that at least one of R₁ₐ and R₄ₐ is hydrogen.

In some embodiments, the lipid compound of formula (I) is in an ionizable form, that is, one or more N atoms in the backbone structure of formula (I) can be ionized. In a further embodiment, the lipid compound of formula (I) is in an ionizable form, thereby forming the following structures: 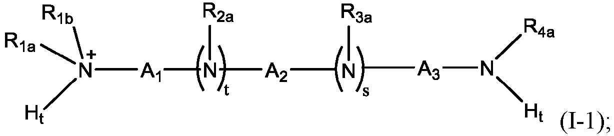 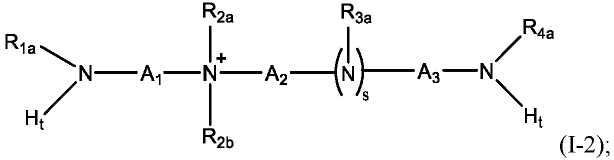 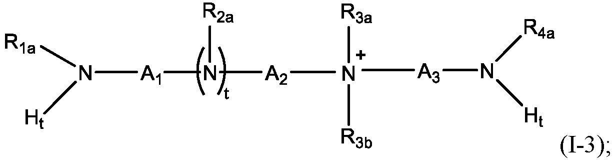 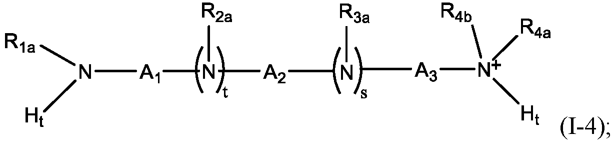 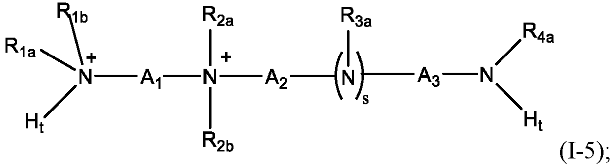 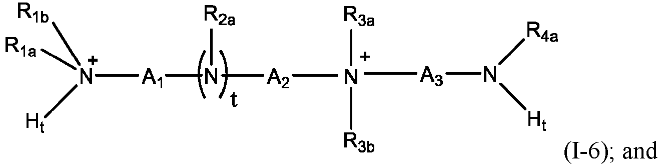 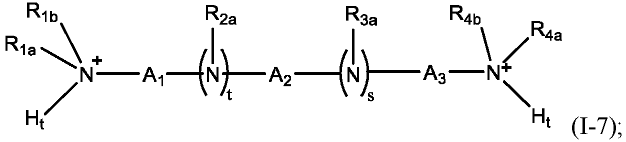 and contains corresponding counter ions, wherein R_{1b}, R_{2b}, R_{3b}, and R_{4b} are each independently hydrogen, a monovalent aliphatic group, a monovalent heteroaliphatic group, a monovalent aromatic group, a monovalent heteroaromatic group, or Ht, and the remaining variables are as defined in formula (I). In some embodiments, the counter ion is an anion that forms a salt with the positively charged N atom. Suitable anions include, but are not limited to, chloride ions, bromide ions, iodide ions, sulfate radicals, nitrate radicals, phosphate radicals, citrate radicals, mesylate radicals, trifluoroacetate radicals, acetate radicals, malate radicals, tosylate radicals, tartrate radicals, fumarate radicals, glutamate radicals, glucuronate radicals, lactate radicals, glutarate radicals, maleate radicals, etc.

In some embodiments, R₁ₐ or R₄ₐ is hydrogen; or R₁ₐ and R₄ₐ are each hydrogen.

In some embodiments, t and s are both 0; or t is 0 and s is 1; or t is 1 and s is 0.

In some embodiments, t and s are both 0; R₁ₐ is Ht, and R₄ₐ is hydrogen; or t and s are both 0; and R₁ₐ is hydrogen, and R₄ₐ is Ht.

In some embodiments, R₂ₐ and R₃ₐ, if present, are each independently hydrogen, a monovalent aliphatic group, or a monovalent heteroaliphatic group; preferably hydrogen, a monovalent C₁₋₆ alkyl, or a monovalent C₁₋₆ heteroalkyl, e.g., amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆alkylthio C₁₋₆ alkyl, or mercapto C₁₋₆ alkyl.

In some embodiments, the fragment 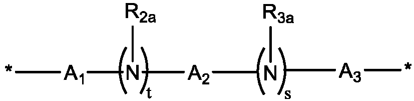 is
- A₁-A₃, wherein A₁ and A₃ are each independently a C₁-C₆ divalent aliphatic group (e.g., a C₁-C₄ divalent aliphatic group, e.g., a C₁-C₆ divalent alkyl, preferably a C₁-C₄ divalent alkyl); or
- A₁-(NR₂ₐ)-A₃, wherein A₁ and A₃ are each independently a C₁-C₆ divalent aliphatic group (e.g., a C₁-C₄ divalent aliphatic group, e.g., a C₁-C₆ divalent alkyl, preferably a C₁-C₄ divalent alkyl), and R₂ₐ is hydrogen, a monovalent aliphatic group, or a monovalent heteroaliphatic group; preferably hydrogen, a monovalent C₁₋₆ alkyl, or a monovalent C₁₋₆ heteroalkyl, e.g., amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆ alkylthio C₁₋₆ alkyl, or mercapto C₁₋₆ alkyl; or
- A₁-(NR₂ₐ)-A₂-(NR_{2b})-A₃, wherein A₁, A₂, and A₃ are each independently a C₁-C₆ divalent aliphatic group (e.g., a C₁-C₄ divalent aliphatic group, e.g., a C₁-C₆ divalent alkyl, preferably q C₁-C₄ divalent alkyl); and R₂ₐ and R_{2b} are each independently hydrogen, a monovalent aliphatic group, or a monovalent heteroaliphatic group; preferably hydrogen, a monovalent C₁₋₆ alkyl, or a monovalent C₁₋₆ heteroalkyl, e.g., amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆alkylthio C₁₋₆ alkyl, or mercapto C₁₋₆ alkyl; or
- A₁-A₂-A₃, wherein A₁ and A₃ are each independently a C₁-C₆ divalent aliphatic group (e.g., a C₁-C₄ divalent aliphatic group, e.g., a C₁-C₆ divalent alkyl, preferably a C₁-C₄ divalent alkyl); and A₂ is a divalent cyclic heteroaliphatic group, such as a 3- to 7-membered monocyclic heteroaliphatic group containing 1-3 heteroatoms selected from oxygen, nitrogen and sulfur, preferably divalent piperazinyl;
wherein the asterisk * represents the connection of the terminal N atom in formula (I) or (I-1) to (I-7).

In some embodiments, the fragment 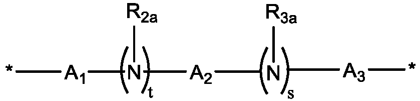 is
- -(CH₂)ₘ₁-, wherein m1 is an integer of 2-12, preferably 2-10 or 3-8; or an integer of 3; or
- -(CH₂)ₘ₂-(NR₂ₐ)-(CH₂)ₘ₃-, wherein m2 and m3 are each independently an integer of 1-6; preferably an integer of 2-6, or 2-4, or 2, or 3; R₂ₐ is hydrogen, a monovalent C₁₋₆ alkyl, amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆ alkylthio C₁₋₆ alkyl, or mercapto C₁₋₆ alkyl; or
- -(CH₂)ₘ₂-(NR₂ₐ)-(CH₂)ₘ₄-(NR_{2b})-(CH₂)ₘ₃, wherein m2, m3, and m4 are each independently an integer of 1-6; preferably an integer of 2-6, or 2-4, or 2, or 3; R₂ₐ and R_{2b} are each independently hydrogen, a monovalent C₁₋₆ alkyl, amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆ alkylthio C₁₋₆ alkyl, or mercapto C₁₋₆ alkyl; or
- -(CH₂)ₘ₂-A₂-(CH₂)ₘ₃, wherein m2 and m3 are each independently an integer of 1-6; preferably an integer of 2-6, or 2-4, or 2, or 3; and A₂ is a divalent cyclic heteroaliphatic group, such as a 3- to 7-membered monocyclic heteroaliphatic group containing 1-3 heteroatoms selected from oxygen, nitrogen and sulfur, preferably divalent piperazinyl;
wherein the asterisk * represents the connection of the terminal N atom in formula (I) or (I-1) to (I-7).

In some embodiments, R₁ is a C₁₋₆ (e.g., C₁₋₄) divalent aliphatic group or a C₁₋₆ (e.g., C₁₋₄) divalent heteroaliphatic group, preferably a C₁₋₄ divalent alkyl or a C₁₋₄ divalent heteroalkyl.

In some embodiments, X is 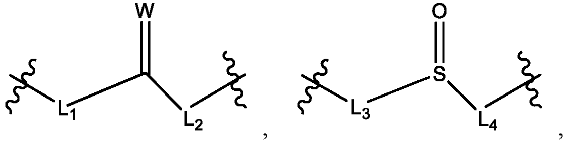 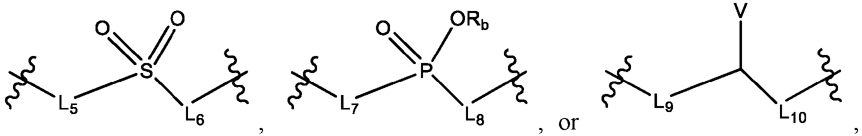 wherein each variable is as defined in formula (I). In a further embodiment, L₁, L₃, L₅, L₇, and L₉ are connected to R₁ and are each independently a single bond, O, S, or NH. In a further embodiment, X is 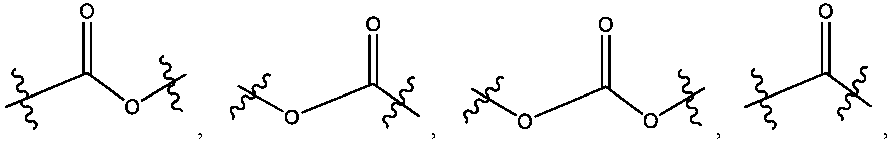 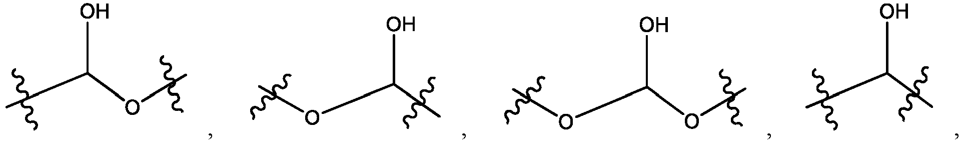 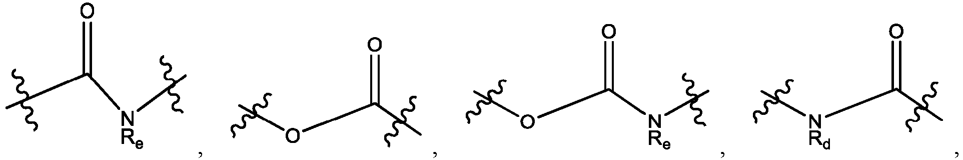 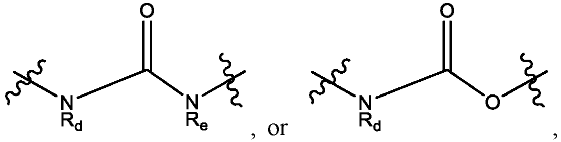 wherein R_{d} and Rₑ are as defined in formula (I). In some embodiments, R^{d} and R^{e} are each independently H or a C₁₋₄ monovalent aliphatic group, preferably H or C₁₋₄ monovalent alkyl.

In some embodiments, Y and Z are both S; or Y is S and Z is O; or Y is O and Z is S; or Y and Z are both O.

In some embodiments, each R₂ is independently at each occurrence a single bond or a C₁₋₆ divalent aliphatic group (e.g., a C₁₋₄ divalent aliphatic group, preferably a C₁₋₄ divalent alkyl, more preferably a C₁₋₂ divalent alkyl).

In some embodiments, each R₃ is independently at each occurrence a single bond or a C₁₋₆ divalent aliphatic group (e.g., a C₁₋₄ divalent aliphatic group, preferably a C₁₋₄ divalent alkyl). In a further embodiment, each R₃ is independently at each occurrence a single bond, or 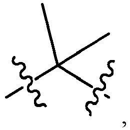 or methylene 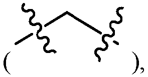 preferably

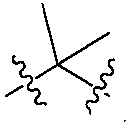

In some embodiments, each R₄ is independently at each occurrence a hydrophobic group selected from long-chain alkyl, alkenyl, aryl, alkylaryl, arylalkyl, arylalkenyl, a cyclic group, an alicyclic group, and a polycyclic group, and optionally having at least one heteroatom selected from nitrogen, oxygen, and sulfur. In a further embodiment, each R₄ is independently at each occurrence C₈-C₃₀ alkyl, C₈-C₃₀ alkenyl, or C₈-C₃₀ alkynyl. In a further embodiment, R₄ is

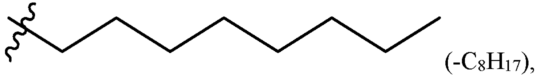

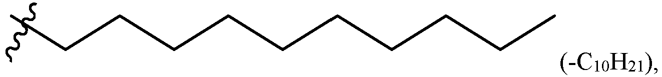

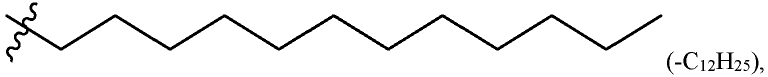

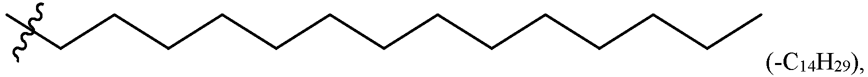

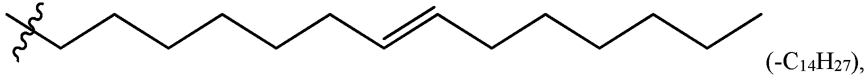

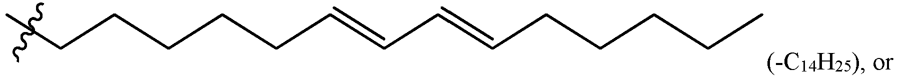

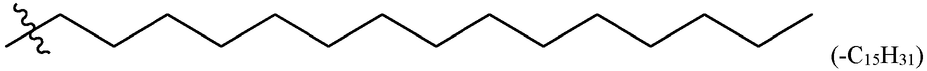

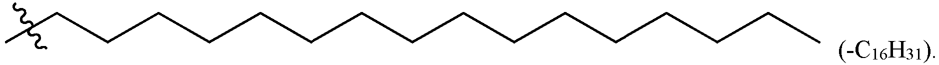

In a further embodiment, each R₄ is independently at each occurrence -(CH₂CH₂O)ₘ-C₈-C₃₀ alkyl, -(CH₂CH₂O)_{y}-C₈-C₃₀ alkenyl, or -(CH₂CH₂O)_{y}-C₈-C₃₀ alkynyl, in which y is 0, or 1, or 2, for example, R₄ is -CH₂CH₂O-C₈H₁₇, -CH₂CH₂O-C₁₀H₂₁, -CH₂CH₂O-C₁₂H₂₅, -CH₂CH₂O-C₁₄H₂₉, or -CH₂CH₂O-C₁₅H₃₁.

In some embodiments, the pKa value of the amine head is greater than 4, preferably greater than 6, and more preferably greater than 8.

In some embodiments, A₁ and A₃ are each independently a single bond or a divalent aliphatic group, and A₂ is a divalent aliphatic group or a divalent heteroaliphatic group. In a further embodiment, A₁ and A₃ are each independently a C₁-C₆ divalent aliphatic group (e.g., a C₁-C₄ divalent aliphatic group, preferably a C₁-C₄ divalent alkyl), and A₂ is a divalent cyclic heteroaliphatic group, such as a 3- to 7-membered monocyclic heteroaliphatic group containing 1-3 heteroatoms selected from oxygen, nitrogen, and sulfur. In a further embodiment, A₁ and A₃ are each independently ethydene, and A₂ is divalent piperazinyl.

In some embodiments, the lipid compound of formula (I) is 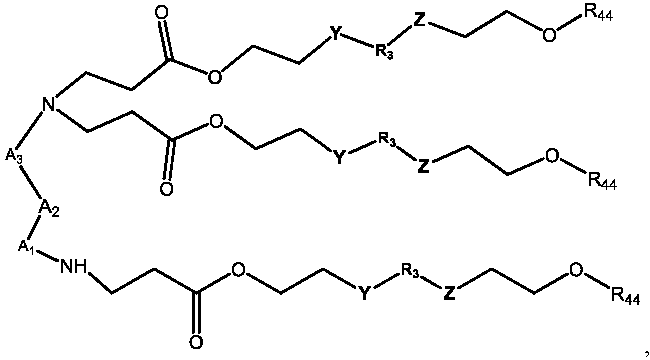 or 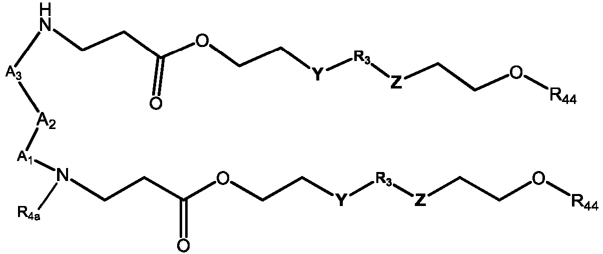 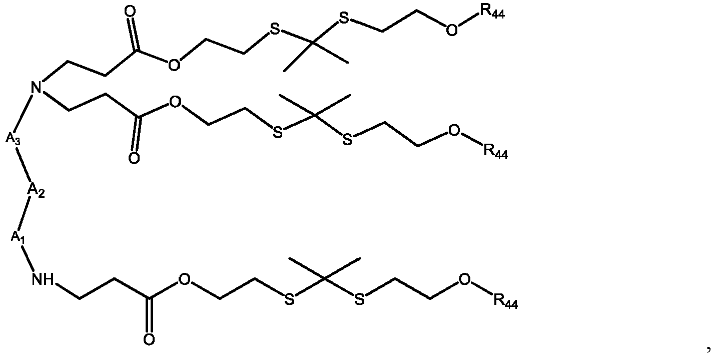 or 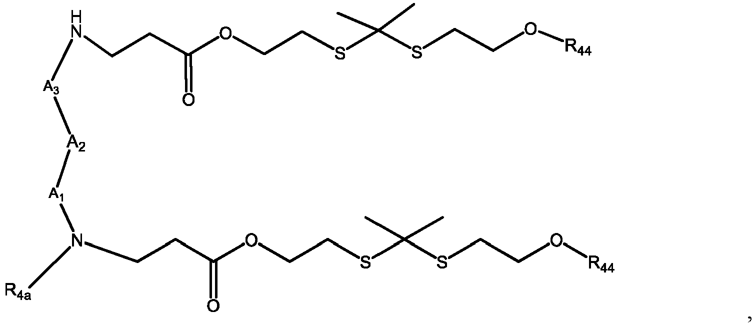 wherein A₁, A₂, A₃, and R₄ₐ are as defined in formula (I), and -(CH₂CH₂O)-R₄₄ is R₄ as defined in formula (I); Y and Z are both S; or Y is S and Z is O; or Y is O and Z is S; or Y and Z are both O; and each R₃ is independently at each occurrence a single bond, or 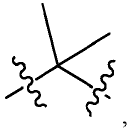 or methylene 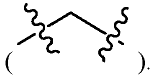 In a further embodiment, each R₄₄ is independently at each occurrence alkyl CᵤH₂ᵤ₊₁ or alkenyl CᵤH₂ᵤ₋₁, wherein u is 8-30, preferably 8, 12, 14, or 16. In a further embodiment, A₂ is a divalent cyclic heteroaliphatic group, preferably divalent piperazinyl, and A₁ and A₃ are each independently C₁₋₄ divalent alkyl, e.g., methylene, ethylene, and propylene.

In some embodiments, the lipid compound of formula (I) is 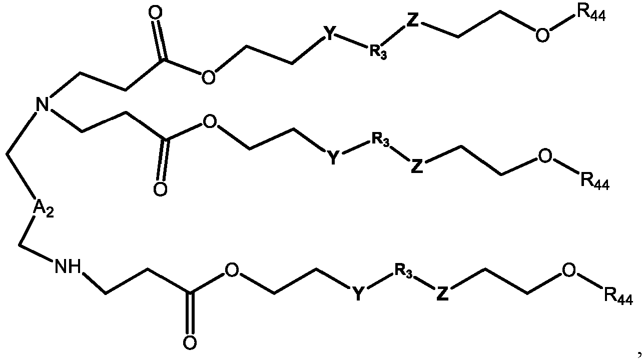 or 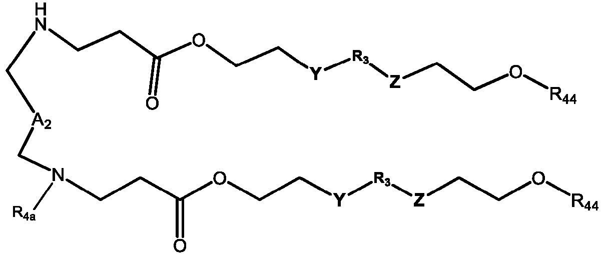 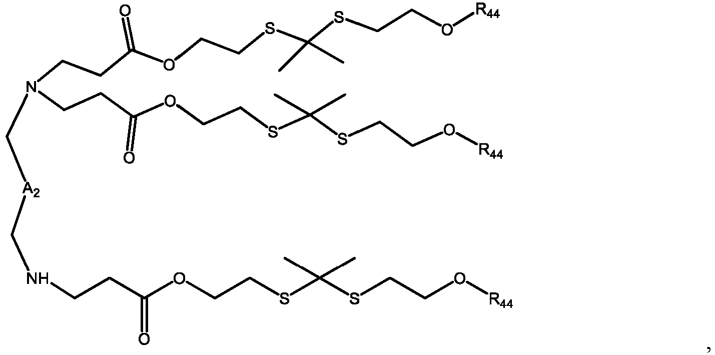 or 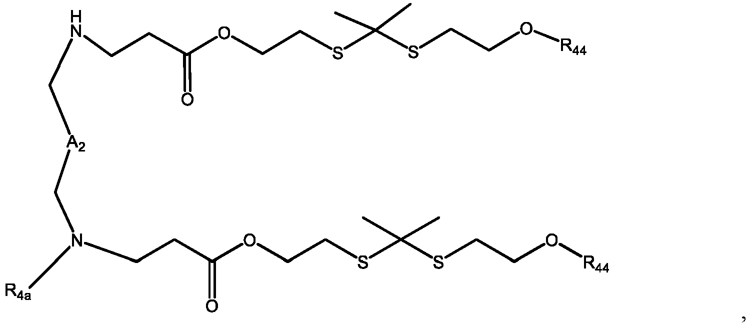 wherein A₂ and R₄ₐ are as defined in formula (I), and -(CH₂CH₂O)-R₄₄ is R₄ as defined in formula (I); Y and Z are both S; or Y is S and Z is O; or Y is O and Z is S; or Y and Z are both O; and each R₃ is independently at each occurrence a single bond, or 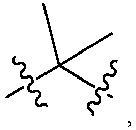 or methylene 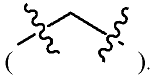 In a further embodiment, each R₄₄ is independently at each occurrence alkyl CᵤH₂ᵤ₊₁ or alkenyl CᵤH₂ᵤ₋₁, wherein u is 8-30, preferably 8, 12, 14, or 16. In a further embodiment, A₂ is a divalent cyclic heteroaliphatic group, preferably divalent piperazinyl.

In some embodiments, the lipid compound of formula (I) is 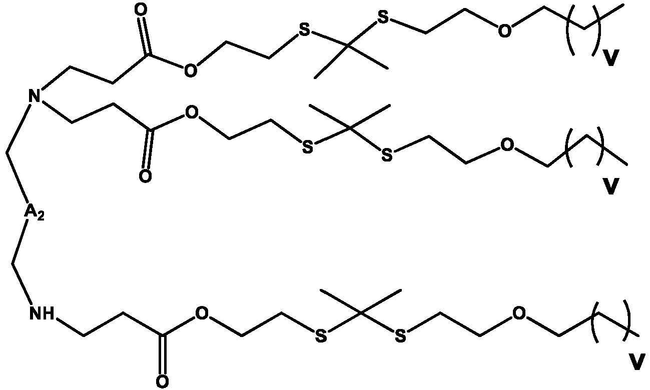 or 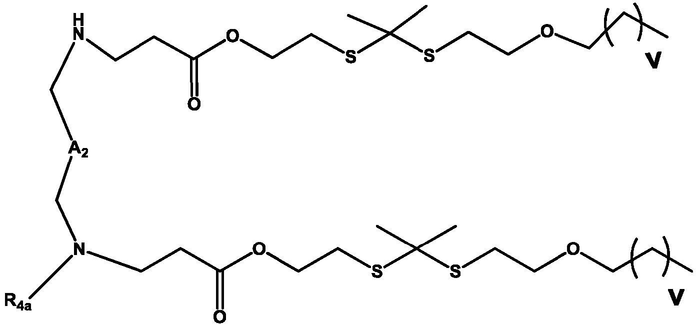 wherein v is 6-28, and preferably v is 10; A₂ and R₄ₐ are as defined in formula (I). In a further embodiment, A₂ is a divalent cyclic heteroaliphatic group, preferably divalent piperazinyl.

In some embodiments, the lipid compound of formula (I) is 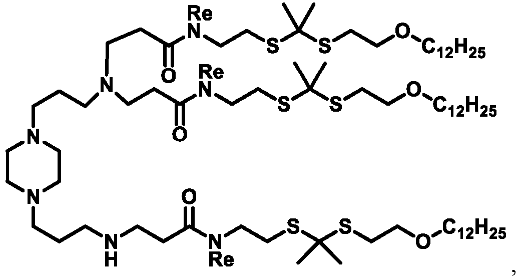 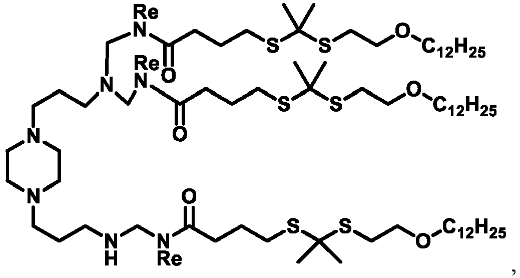 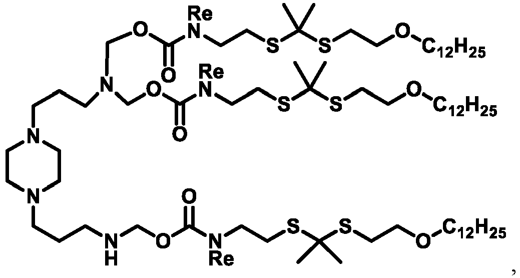 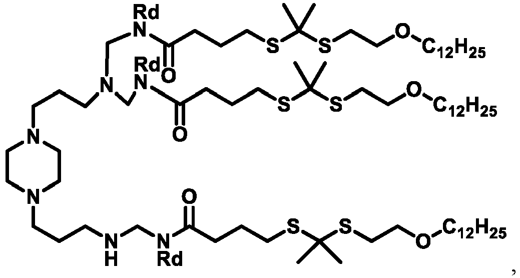 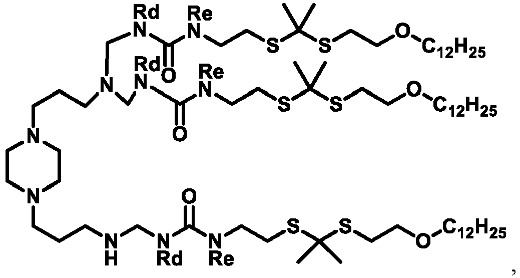 or 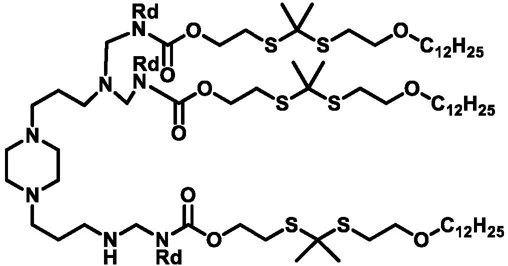 wherein R_{d} and Rₑ are as defined in formula (I), and preferably, each is independently hydrogen or a monovalent aliphatic group, such as C₁₋₄ alkyl.

In some embodiments, exemplary lipid compounds are as follow:

| | |
|---|---|
| TK-2 | 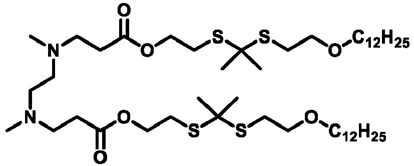 |
| TK-3 | 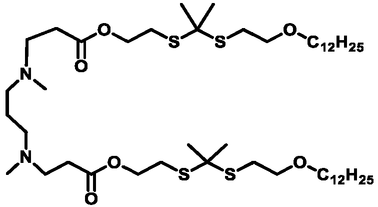 |
| TK-5 | 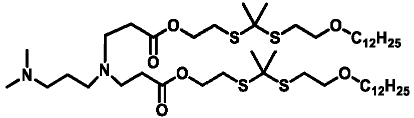 |
| TK-6 | 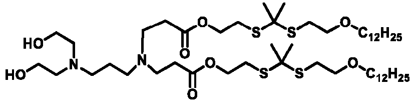 |
| TK-7 | 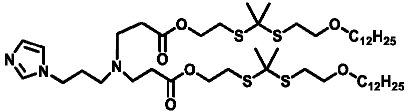 |
| TK-8 | 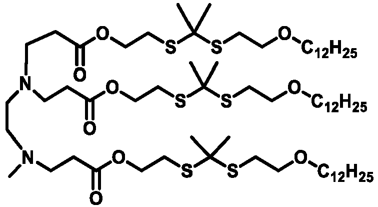 |
| TK-9 | 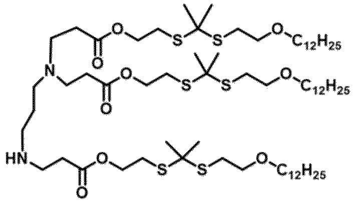 |
| TK-10 | 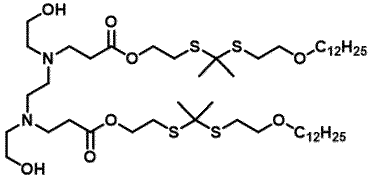 |
| TK-11 | 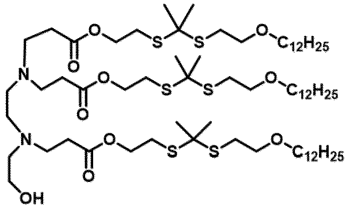 |
| TK-12 | 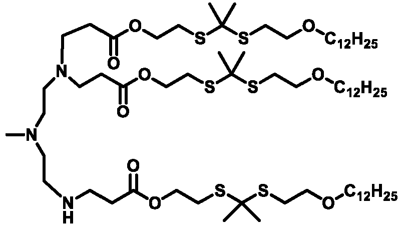 |
| TK-13 | 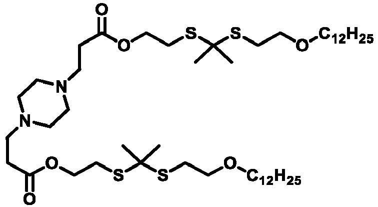 |
| TK-14 | 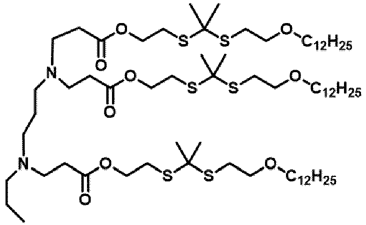 |
| TK-15 | 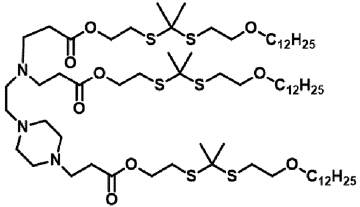 |
| TK-16 | 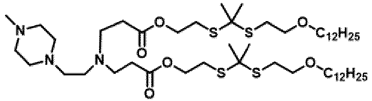 |
| TK-17 | 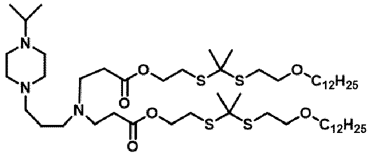 |
| TK-18 | 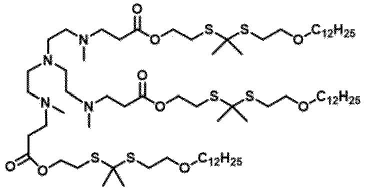 |
| TK-19 | 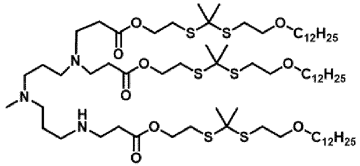 |
| TK-20 | 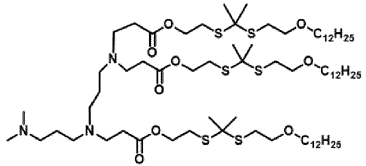 |
| TK-21 (also known as BAmP-TK-12) | 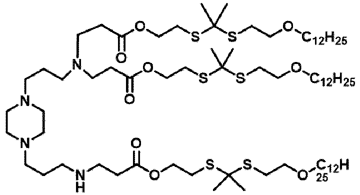 |
| TK-21-4 | 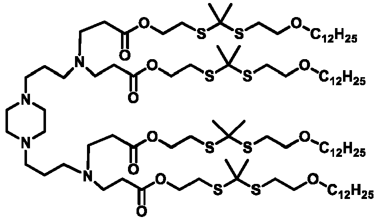 |
| TK-22 | 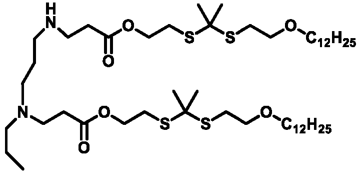 |
| TK-23 | 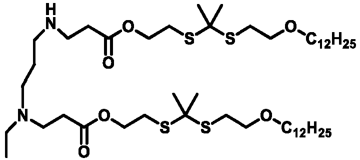 |
| TK-24 | 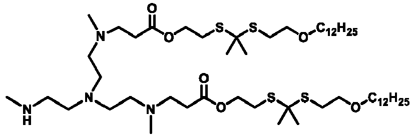 |
| TK-25 | 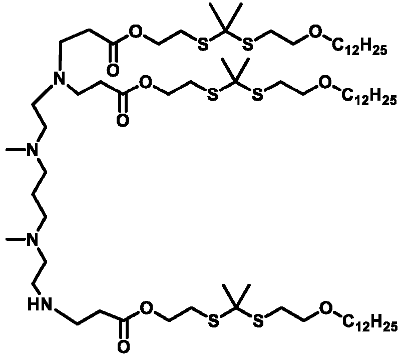 |

In some embodiments, the target cell is a cancer cell, a cell infected by a pathogen, or a cell mediating a disease, e.g., a cell selected from an endothelial cell, an epithelial cell, a muscle cell, a brain cell, a nerve cell, a skin cell, a hair cell, a progenitor cell, and a perithelial cell.

In some embodiments, the nucleic acid is selected from an oligonucleotide, an aptamer, a single-stranded DNA, a double-stranded DNA, a plasmid DNA (plasmid), a short isomer, an antisense molecule, a small interfering RNA (siRNA), an asymmetric interfering RNA (aiRNA), a microRNA (miRNA), a dsRNA (double-stranded RNA), an shRNA (small/short hairpin RNA), a transfer RNA (tRNA), a messenger RNA (mRNA), a small activating RNA, a circRNA, and other forms of RNA molecules known in the art.

In some embodiments, the nucleic acid exists in the form of a nanocomplex. In some embodiments, the nanocomplex further comprises other lipids.

In the present invention, A₁, A₂, and A₃, together with the N to which they are attached, form an amine head of the lipid compound of the present invention, which undergoes an addition reaction with a hydrophobic lipid tail to obtain a series of lipids. Since the amine head can be protonated, such lipid nanoparticles can be positively charged as a whole, so they can undergo an electrostatic interaction with negatively charged nucleic acids such as mRNAs, cell membranes and lysosomal membranes, such that such lipids can effectively encapsulate and deliver nucleic acids. The inventors of the present invention have found that the lipid compound with incomplete substitution of the nitrogen on the amine head has a stronger proton sponge effect than the lipid compound with complete substitution, and the lysosomal escape ability is improved, thus further improving the ability to deliver nucleic acid drugs; moreover, a specific response to the ROS species in lesion cells enhances the drug release, thus reducing dosage-associated toxicities. The inventors of the present invention have designed and synthesized a class of ROS-responsive lipid compounds, and have screened out lipid compounds with efficient plasmid delivery, e.g., BAmP-TK-12 of the present invention, which can realize the efficient and selective delivery of RAS protease DUF5 mRNA in various KRAS mutant cancer cells and live animals, thus solving the problem of difficult universal regulation due to tumor RAS mutations. The complex (BAmP-TK-12/DUF5 mRNA) formed by the inventors of the present invention by self-assembly using the lipid and DUF5 mRNA is expected to be capable of inhibiting abnormal RAS/RAF/MAPK/ERK and RAS/PI3K/AKT signaling pathways and tumor growth *in vitro* and *in vivo,* thus achieving the goal of targeting various mutant types of KRAS in cancer cells. The present invention is devoted to exploring the potential of the BAmP-TK-12/DUF5 mRNA complex to treat cancer and provides a new idea for cancer treatment strategies by targeted inhibition of RAS.

### Definitions

The term "aliphatic group" refers to a saturated or unsaturated, linear or branched, acyclic, cyclic or polycyclic hydrocarbon moiety. Examples include, but are not limited to, alkyl, alkenyl, alkynyl, cycloalkyl, cycloalkenyl, and cycloalkynyl moieties.

The term "alkyl" includes hydrocarbon groups selected from linear and branched saturated hydrocarbon groups containing 1 to 30, such as 1 to 24, 1 to 18, such as 1 to 12, further such as 1 to 10, and still further such as 1 to 8 or 1 to 6 or 1 to 4 carbon atoms. Examples of monovalent alkyl or alkyl include, but are not limited to, methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, triacontyl, etc. Examples of divalent alkyl, i.e., alkylene, include, but are not limited to, methylene, ethylene, propylene, butylene, pentylene, hexylene, heptylene, octylene, nonylene, decylene, undecylene, dodecylene, tridecylene, tetradecylene, pentadecylene, hexadecylene, heptadecylene, octadecylene, nonadecylene, eicosylene, triacotylene, etc.

A monovalent group is a group formed by removing a hydrogen atom from the corresponding hydrocarbon moiety. A divalent group is a group formed by removing two hydrogen atoms from the corresponding hydrocarbon moiety.

The term "alkenyl" includes hydrocarbon groups selected from linear and branched hydrocarbon groups containing at least one C=C double bond and 2 to 30, such as 2 to 24, 2 to 18, such as 2 to 8, further such as 2 to 6 carbon atoms. Examples of alkenyl, such as C2-6 alkenyl, include, but are not limited to, ethenyl/vinyl, prop-1-enyl, prop-2-enyl, 2-methylprop-1-enyl, but-1-enyl, but-2-enyl, but-3-enyl, buta-1,3-dienyl, 2-methylbuta-1,3-dienyl, hex-1-enyl, hex-2-enyl, hex-3-enyl, hex-4-enyl, hexa-1,3-dienyl, dodecenyl, tetradecenyl, hexadecenyl, octadecenyl, etc.

The term "alkynyl" includes hydrocarbon groups selected from linear and branched hydrocarbon groups containing at least one C≡C triple bond and 2 to 30, such as 2 to 24, 2 to 18, such as 2 to 8, further such as 2 to 6 carbon atoms. Examples of alkynyl, such as C2-6 alkynyl, include but are not limited to ethynyl, 1-propynyl, 2-propynyl (propargyl), 1-butynyl, 2-butynyl, and 3-butynyl.

The term "cycloalkyl" includes hydrocarbon groups selected from saturated cyclic hydrocarbon groups containing monocyclic and polycyclic (e.g., bicyclic and tricyclic) groups, including fused, bridged or spirocyclic alkyl. The cycloalkyl may contain 3 to 30, 3 to 12, for example 3 to 10, further for example 3 to 8, further for example 3 to 6, 3 to 5 or 3 to 4 carbon atoms. Furthermore, by way of example, the cycloalkyl may be selected from monocyclic groups containing 3 to 12, such as 3 to 10, further such as 3 to 8, 3 to 6 carbon atoms. Examples of monocyclic cycloalkyl include cyclopropyl, cyclobutyl, cyclopentyl, cyclohexyl, cycloheptyl, cyclooctyl, cyclononyl, cyclodecyl, cycloundecyl, and cyclododecyl. The term "cycloalkenyl" refers to a non-aromatic, cyclic hydrocarbon moiety containing at least one double bond, such as cyclohexenyl and cyclohexenylene. The term "cycloalkynyl" refers to a non-aromatic, cyclic hydrocarbon moiety containing at least one triple bond, such as cyclooctynyl and cyclooctynylene. Similarly, cycloalkylene, cycloalkenylene, and cycloalkynylene are corresponding divalent groups.

The term "heteroaliphatic group" refers to an aliphatic moiety containing at least one heteroatom selected from N, O, P, B, S, Si, Sb, Al, Sn, As, Se, and Ge. The heteroaliphatic group of the present invention includes alkyl, alkenyl or alkynyl containing at least one heteroatom selected from N, O, P, B, S, Si, Sb, Al, Sn, As, Se, and Ge, and a cycloalkyl, cycloalkenyl or cycloalkynyl moiety containing at least one heteroatom selected from N, O, P, B, S, Si, Sb, Al, Sn, As, Se, and Ge. The cyclic heteroaliphatic group includes a 3- to 7-membered monocyclic heteroaliphatic group and a 7- to 12-membered bicyclic heteroaliphatic group, and the cyclic heteroaliphatic groups contain 1-3 or more, for example, 1-3 heteroatoms selected from oxygen, nitrogen, and sulfur. In some embodiments, the cyclic heteroaliphatic group is a 3- to 7-membered monocyclic heteroaliphatic group containing 1-3 heteroatoms selected from oxygen, nitrogen and sulfur. Cyclic heteroaliphatic groups include, but are not limited to, pyrrolidinyl, imidazolidinyl, piperidinyl, piperazinyl, pyranyl, morpholinyl, oxiranyl, azirinyl, thiiranyl, azetidinyl, oxetanyl, thietanyl, dithietanyl, dihydropyridine, tetrahydropyridine, thiomorpholine, homopiperazinyl, homopiperidinyl, azepanyl, oxepanyl, thiepanyl, oxathianyl, dioxepanyl, oxathiepanyl, oxazepanyl, dithiepanyl, thiazepanyl, diazepanyl, thiazinanyl, oxazepine, diazepine, thiazepine, dihydrothienyl, dihydropyranyl, dihydrofuranyl, tetrahydrofuranyl, tetrahydrothienyl, tetrahydropyranyl, tetrahydrothiapyranyl, pyrrolinyl, indolinyl, dioxanyl, dioxolanyl, pyrazolinyl, pyrazolidinyl, dithianyl, dithiolanyl, pyrimidonyl, dioxo-thiomorpholinyl, azabicyclo[3.1.0]hexyl, azabicyclo[4.1.0]heptyl, azabicyclo[2.2.2]hexyl, etc. Typical heteroaliphatic groups are heteroalkyl groups, i.e., alkyl containing at least one heteroatom of N, O, or S, for example, C₁₋₆ alkyl containing one N atom (e.g., aminoalkyl and alkylaminoalkyl, still for example, amino C₁₋₆ alkyl, di(C₁₋₆ alkyl)aminoalkyl, and mono(C₁₋₆ alkyl)aminoalkyl), C₁₋₆ alkyl containing one O atom (e.g., alkoxyalkyl and hydroxyalkyl, still for example, C₁₋₆ alkoxy C₁₋₆ alkyl and hydroxy C₁₋₆ alkyl), or C₁₋₆ alkyl containing one S atom (e.g., alkylthioalkyl and mercaptoalkyl, still for example, C₁₋₆alkylthio C₁₋₆ alkyl and mercapto C₁₋₆ alkyl); or C₁₋₄ heteroalkyl containing one N atom, C₁₋₄ heteroalkyl containing one O atom, or C₁₋₄ heteroalkyl containing one S atom.

The term "oxyaliphatic group" refers to a -O-aliphatic group. Examples of the oxyaliphatic group include methoxy, ethoxy, n-propoxy, isopropoxy, n-butoxy, isobutoxy, sec-butoxy, and tert-butoxy.

The term "aryl (aromatic group)" refers to a C6 monocyclic, C10 bicyclic, C14 tricyclic, C20 tetracyclic, or C24 pentacyclic aromatic ring system. Examples of aryl include phenyl, phenylene, naphthyl, naphthylene, anthryl, anthrylene, pyrenyl, and pyrenylene.

The term "heteroaryl (heteroaromatic group)" refers to aromatic 5- to 8-membered monocyclic, 8- to 12-membered bicyclic, 11- to 14-membered tricyclic, and 15- to 20-membered tetracyclic ring systems having one or more heteroatoms (such as O, N, S, or Se). Examples of heteroaryl include furanyl, furanylene, fluorenyl, fluorenylene, pyrrolyl, pyrrolylene, thienyl, thienylene, oxazolyl, azolylene, imidazolyl, imidazolylene, benzimidazolyl, benzimidazolylene, thiazolyl, thiazolylene, pyridyl, pyridylene, pyrimidinyl, pyrimidinylene, quinazolinyl, quinazolinylene, quinolinyl, quinolinylene, isoquinolinyl, isoquinolinylene, indolyl, and indolylene.

Unless otherwise specified, the aliphatic group, heteroaliphatic group, oxyaliphatic group, alkyl, alkylene, alkenyl, alkenylene, alkynyl, alkynylene, cycloalkyl, cycloalkylene, cycloalkenyl, cycloalkenylene, cycloalkynyl, cycloalkynylene, heterocycloalkyl, heterocycloalkylene, heterocycloalkenyl, heterocycloalkenylene, aryl, and heteroaryl mentioned in the present application include both substituted and unsubstituted moieties. Possible substituents on the cycloalkyl, cycloalkylene, cycloalkenyl, cycloalkenylene, cycloalkynyl, cycloalkynylene, heterocycloalkyl, heterocycloalkylene, heterocycloalkenyl, heterocycloalkenylene, aryl, and heteroaryl include, but are not limited to, C₁-C₁₀ alkyl, C₂-C₁₀ alkenyl, C₂-C₁₀ alkynyl, C₃-C₂₀ cycloalkyl, C₃-C₂₀ cycloalkenyl, C₂-C₂₀ heterocycloalkyl, C₃-C₂₀ heterocycloalkenyl, C₁-C₁₀ alkoxy, aryl, aryloxy, heteroaryl, heteroaryloxy, amino, C₁-C₁₀ alkylamino, C₂-C₂₀ dialkylamino, arylamino, diarylamino, C₁-C₁₀ alkylsulfonamido, arylsulfonamido, C₁-C₁₀ alkylimino, arylimino, C₁-C₁₀ alkylsulfonimido, arylsulfonimido, hydroxyl, halo, thio, C1-C10 alkylthio, arylthio, C₁-C10 alkylsulfonyl, arylsulfonyl, acylamino, aminoacyl, aminothioacyl, amido, amidino, guanidine, ureido, thioureido, cyano, nitro, nitroso, azido, acyl, thioacyl, acyloxy, carboxyl, and carboxylate. In another aspect, possible substituents on the aliphatic group, heteroaliphatic group, oxyaliphatic group, alkyl, alkylene, alkenyl, alkenylene, alkynyl, and alkynylene include all the substituents listed above, except C₁-C₁₀ alkyl. Cycloalkyl, cycloalkylene, cycloalkenyl, cycloalkenylene, heterocycloalkyl, heterocycloalkylene, heterocycloalkenyl, heterocycloalkenylene, aryl, and heteroaryl may also be fused with one another.

The term "pharmaceutically active molecule" refers to any chemical substance intended for medical diagnosis, cure, treatment, or prevention of diseases, including small molecules and macromolecular drugs.

The term "small molecular substance" refers to organic compounds with a molecular weight of less than 800 Daltons (e.g., less than 500 Daltons), including oligopeptides, oligosaccharides, and oligonucleotides.

The term "macromolecular drug" includes "peptide" or "protein" and refers to a polymer of natural or unnatural amino acids linked together by amide bonds, which has a molecular weight of 800 Daltons or more.

The term "nucleic acid" refers to a polymer of nucleotides linked together by phosphodiester bonds. These polymers can all be chemically modified. Specifically, the nucleic acid includes any form of nucleic acid molecules, including but not limited to an oligonucleotide, an aptamer, a single-stranded DNA, a double-stranded DNA, a plasmid DNA (plasmid), a short isomer, an antisense molecule, a small interfering RNA (siRNA), an asymmetric interfering RNA (aiRNA), a microRNA (miRNA), a dsRNA (Dicer-substrate RNA), an shRNA (small/short hairpin RNA), a transfer RNA (tRNA), a messenger RNA (mRNA), a small activating RNA, a circRNA, and other forms of RNA molecules known in the art. The term "nucleic acid drug" refers to the above-mentioned nucleic acid molecules with disease treatment functions. The nucleic acid molecules as nucleic acid drugs may also comprise any other pharmaceutically acceptable carriers or excipients.

The term "drug delivery system" refers to a composition for administering a drug (especially a nucleic acid) to a subject in need thereof, such as a human or an animal. In some embodiments, the drug delivery system comprises the lipid compound of the present invention and/or another lipid compound as a drug delivery carrier and can deliver the lipid encapsulating a drug (nucleic acid) to a site of interest in a human or an animal, preferably selectively to target cells, such as cancer cells, rather than to other sites of the body or organs; more preferably to target cells by means of endocytosis.

The term "drug delivery carrier" refers to a substance that releases a drug into the body, such as target cells, during drug delivery to enhance the selectivity, efficacy, safety and/or pharmacokinetics of the drug.

The term "target cell" refers to a cell to which a drug is delivered through a drug delivery system, and generally refers to a cancer cell, a cell infected by a pathogen, or a cell mediating a disease, e.g., a cell selected from an endothelial cell, an epithelial cell, a muscle cell, a brain cell, a nerve cell, a skin cell, a hair cell, a progenitor cell, and a perithelial cell.

The term "non-covalent interaction" refers to any non-covalent bonding, including ionic interaction, hydrogen bonding, van der Waals force interaction, and hydrophobic interaction.

### Brief Description of the Drawings

Fig. 1 shows the delivery of RFP mRNA by lipids.
Fig. 2 shows a study on the structure-activity relationship of BAmP-TK-12 lipid. In the figure, a) the chemical structures of BAmP-TK-12 and BAmP-NTK; b) the RFP mRNA encapsulation efficiency of BAmP-TK-12 and BAmP-NTK; c) the mRNA release of BAmP-TK-12 and BAmP-NTK upon ROS stimulation; d) the Cy3-mRNA delivery efficiency of BAmP-TK-12 and BAmP-NTK in HeLa cells; and e) the RFP mRNA delivery efficiency of BAmP-TK-12 and BAmP-NTK in HeLa cells.
Fig. 3 shows the effect of the number of hydrophobic lipid tails in lipid s with 21TK (i.e., BAmP) as an amine head on delivery.
Fig. 4 shows the efficiency of the lipids of the present invention to deliver different doses of GFP mRNA in HeLa cells.

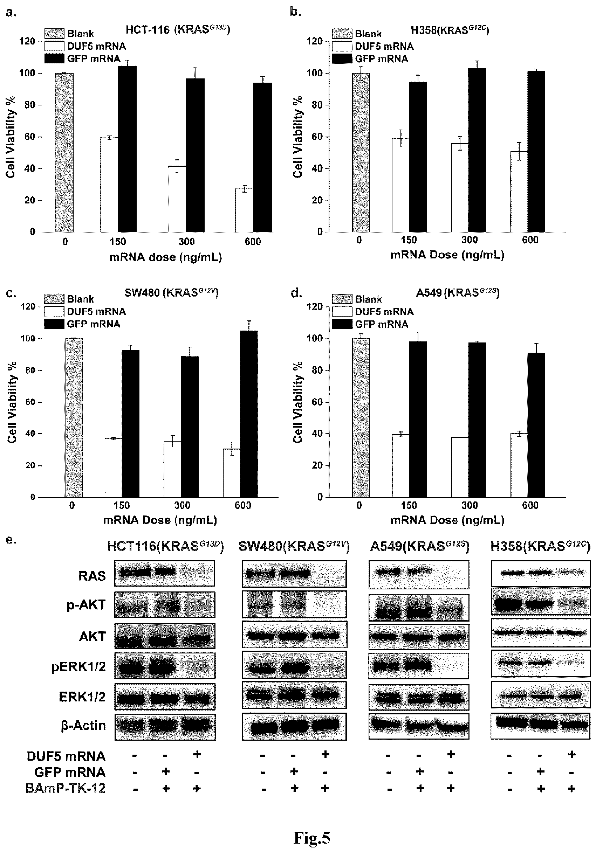

Fig. 5 shows that BAmP-TK-12 delivers DUF5 mRNA to inhibit the proliferation of tumor cells, wherein a) BAmP-TK-12 delivers DUF5 mRNA to inhibit the proliferation of colorectal cancer cell line HCT-116 containing KRAS*^{G13D}* mutation; b) BAmP-TK-12 delivers DUF5 mRNA to inhibit the proliferation of colorectal cancer cell H358 containing KRAS*^{G13C}* mutation; c) BAmP-TK-12 delivers DUF5 mRNA to inhibit the proliferation of colorectal cancer cell SW480 containing KRAS*^{G12V}* mutation; c) BAmP-TK-12 delivers DUF5 mRNA to inhibit the proliferation of non-small cell lung cancer cell line A549 containing KRAS*^{G12S}* mutation; and d) Western blot experiment, wherein for HCT-116, H358, SW480, and A549 cell lines, the DUF5 mRNA delivered by BAmP-TK-12 inhibits the phosphorylation of AKT protein and ERK protein by cleaving RAS protein to inhibit the proliferation of tumor cells.
Fig. 6 shows the *in vivo* delivery of DUF5 mRNA by BAmP-TK-12 lipid and tumor inhibition. a) *In vivo* distribution of BAmP-TK-12/DUF5 mRNA. b) A relative growth curve of the tumor volume in HCT-116 tumor-bearing mice. c) Tumor tissues in mice at the end of the experiment. d) Western blot experiment for tumor tissues.
Fig. 7 shows an NMR spectrum of compound TK-21 (BAmP-TK-12).
Fig. 8 shows an NMR spectrum of compound TK-21-4 (BAmP with 4 TK-12).
Fig. 9 shows an experiment of the delivery of pDHFR-GFP plasmid (5185 bp) by BAmP-TK-12.
Fig. 10 shows an experiment of the delivery of pDNMT3A-GFP (9746 bp) plasmid by BAmP-TK-12.
Fig. 11 shows an experiment of the delivery of DUF5 mRNA and GFP mRNA by the lipid of the present invention.
Fig. 12 shows an experiment of the delivery of P53 mRNA by BAmP-TK-12.
Fig. 13 shows an experiment of the delivery of GFP protein and GFP mRNA by BAmP-TK-12.

### Detailed Description of Embodiments

The present invention will be further described in detail in conjunction with specific embodiments, and examples are given only to illustrate the present invention, but not to limit the scope of the present invention. The examples provided below can be used as a guide for further improvement by those of ordinary skill in the art and are not intended to limit the present invention in any way.

For all the quantitative experiments in the following examples, three repeated experiments are set up and the results are averaged.

The experimental methods in the following examples are all conventional unless otherwise specified. The materials, reagents, etc., used in the following examples can all be obtained from commercial sources unless otherwise specified.

In the following examples, cholesterol is a product from Macklin, with article number C10006595, CAS: 57-88-5.

In the following examples, DOPE (dioleoylphosphatidylethanolamine) is a product from Ponsure Biotechnology, with CAS: 4004-05-1.

In the following examples, DSPE-PEG2000 is a product from Ponsure Biotechnology, with article number E05275 and CAS: 147867-65-0.

In the following examples, plasmids pDHFR-GFP and pDNMT3A-GFP are products from Wuhan Miaoling Biotechnology Co., Ltd.

In the following examples, GFP protein is a product from Beijing Solarbio Science & Technology Co., Ltd.

In the following examples, HeLa cells, HCT-116 cells, SW480 cells, and A549 cells are products from Beijing Xiehe Cell Center.

In the following examples, Nu/Nu mice are products from Beijing Vital River Laboratory Animal Technology Co., Ltd.

### Examples

### Example 1: Synthesis of lipid compounds and preparation of complexes

### Example 1a: Synthesis of lipid compounds

A lipid compound was synthesized by a Michael addition reaction between a hydrophilic amine compound and a hydrophobic tail, wherein the hydrophobic tail was a ketal-containing acrylate, specifically a thioketal-containing acrylate. The thioketal-containing acrylate was 2-((2-((2-(dodecyloxy)ethyl)thio)propan-2-yl)thio)ethyl acrylate as prepared by the methods in Examples 1 and 2 in CN 110101665 A. The ¹H NMR of the prepared 2-((2-((2-(dodecyloxy)ethyl)thio)propan-2-yl)thio)ethyl acrylate was as shown below: ¹H NMR (300 MHz, DMSO) δ 6.18 (s, 1H), 5.97 (d, J = 10.2 Hz, 1H), 4.26 (s, 2H), 3.51 (s, 4H), 3.34 (s, 6H), 2.87 (s, 1H), 2.74 (s, 1H), 1.55 (s, 4H), 1.50 - 1.39 (m, 1H), 1.25 (s, 6H), 0.86 (s, 1H).

The prepared 2-((2-((2-(dodecyloxy)ethyl)thio)propan-2-yl)thio)ethyl acrylate was then mixed with a hydrophilic amine compound 1,4-bis(3-aminopropyl)piperazine (CAS: 7209-38-3, abbreviated as BAmP) at a molar ratio of 3.3 : 1 and heated at 80°C for 24 h, and the crude product was purified on a silica gel column with dichloromethane/methanol as an eluent to obtain lipid compound TK-21 (also known as BAmP-TK-12).

In a similar way, corresponding lipid compounds were prepared using appropriate proportions of corresponding hydrophilic amines (for a hydrophilic amine containing four free hydrogen atoms on amino groups, the molar ratio of 2-((2-((2-(dodecyloxy)ethyl)thio)propan-2-yl)thio)ethyl acrylate to hydrophilic amine was 3.3 : 1; and for a hydrophilic amine containing three free hydrogen atoms on amino groups, the molar ratio of 2-((2-((2-(dodecyloxy)ethyl)thio)propan-2-yl)thio)ethyl acrylate to hydrophilic amine was 2.4 : 1).

| No. | TK No. | Structural formula | NMR | Molecular weight |
|---|---|---|---|---|
| 1) | TK-2 | 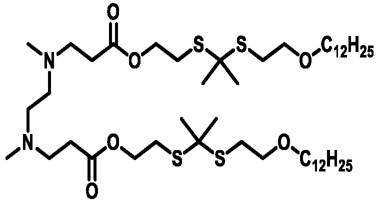 | ¹H NMR (300 MHz, CDCl3) δ 4.23 (s, 4H), 3.58 (s, 4H), 3.43 (s, 4H), 2.84 (s, 7H), 2.73 (s, 4H), 2.49 (s, 7H), 2.25 (s, 5H), 1.79 (d, J = 16.9 Hz, 1H), 1.60 (s, 16H), 1.26 (s, 37H), 0.88 (s, 6H). | MS Calc. 925.6 |
| | | | | Found.925.8 |
| 2) | TK-3 | 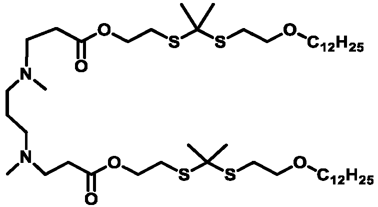 | ¹H NMR (300 MHz, CDCl3) δ 4.23 (s, 4H), 3.58 (s, 4H), 3.43 (s, 4H), 2.84 (s, 8H), 2.70 (s, 4H), 2.48 (s, 4H), 2.36 (s, 4H), 2.22 (s, 6H), 1.60 (s, 18H), 1.26 (s, 36H), 0.88 (s, 6H). | MS Calc. 939.6 |
| | | | | Found.939.8 |
| 3) | TK-5 | 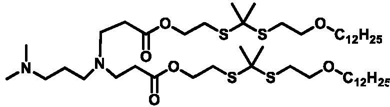 | ¹H NMR (300 MHz, CDCl3) δ 4.22 (s, 4H), 3.58 (s, 4H), 3.43 (s, 4H), 2.82 (s, 11H), 2.45 (s, 5H), 2.30 (s, 6H), 1.60 (s, 16H), 1.26 (s, 34H), 0.88 (s, 6H). | MS Calc. 939.6 |
| | | | | Found.939.8 |
| 4) | TK-6 | 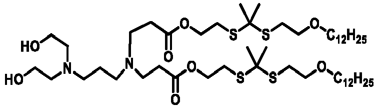 | ¹H NMR (300 MHz, CDCl3) δ 4.23 (s, 2H), 3.79 (s, 4H), 3.60 (s, 4H), 3.44 (s, 4H), 2.87 (s, 10H), 2.53 - 2.18 (m, 5H), 1.62 (s, 15H), 1.26 (s, 38H), 0.88 (s, 6H). | MS Calc. 999.6 |
| | | | | Found.999.9 |
| 5) | TK-7 | 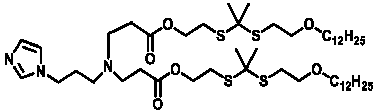 | ¹H NMR (300 MHz, CDCl3) δ 7.27 (s, 1H), 7.08 (d, J = 13.2 Hz, 1H), 6.96 (s, 1H), 4.23 (t, J = 6.9 Hz, 3H), 3.97 (t, J = 6.9 Hz, 1H), 3.58 (t, J = 6.7 Hz, 3H), 3.43 (t, J = 6.7 Hz, 3H), 2.81 (ddd, J = 23.7, 15.3, 6.8 Hz, 9H), 2.50 - 2.32 (m, 4H), 1.93 (dd, J = 13.2, 6.5 Hz, 1H), 1.72 - 1.46 (m, 14H), 1.26 (s, 32H), 0.88 (t, J = 6.4 Hz, 6H). | Calc. 962.6 |
| | | | | Found.962.9 |
| 6) | TK-8 | 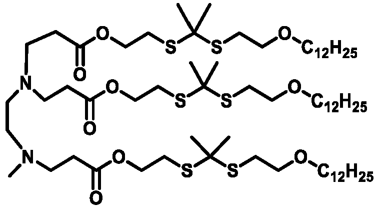 | ¹H NMR (300 MHz, CDCl3) δ 4.22 (s, 4H), 3.58 (s, 4H), 3.43 (s, 4H), 2.82 (s, 11H), 2.71 (s, 2H), 2.55 (s, 2H), 2.46 (s, 5H), 2.24 (s, 2H), 1.60 (s, 19H), 1.26 (s, 42H), 0.88 (s, 9H). | Calc. 1328.86 |
| | | | | Found.1329.86 |
| 7) | TK-9 | 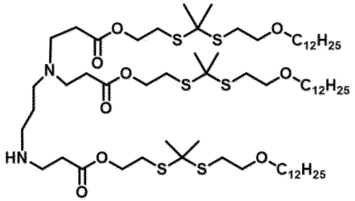 | ¹H NMR (300 MHz, CDCl3) δ 4.22 (s, 4H), 3.58 (s, 4H), 3.43 (s, 4H), 2.80 (ddd, J = 27.5, 16.4, 9.3 Hz, 15H), 2.45 (s, 5H), 1.60 (s, 19H), 1.26 (s, 50H), 0.88 (s, 9H). | Calc. 1328.86 |
| | | | | Found.1329.86 |
| 8) | TK-10 | 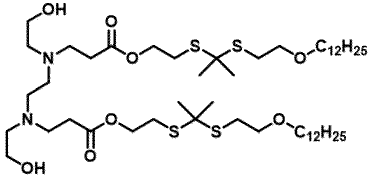 | ¹H NMR (300 MHz, CDCl3) δ 4.25 (s, 2H), 3.79 (s, 1H), 3.58 (s, 5H), 3.43 (s, 4H), 2.86 (s, 11H), 2.58 (s, 9H), 1.60 (s, 12H), 1.26 (s, 32H), 0.88 (s, 6H). | Calc. 985.6 |
| | | | | Found.985.9 |
| 9) | TK-11 | 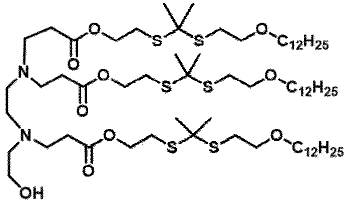 | ¹H NMR (300 MHz, CDCl3) δ 4.27 (s, 4H), 4.07 (s, 4H), 3.58 (s, 4H), 3.43 (s, 4H), 2.80 (ddd, J = 27.5, 16.4, 9.3 Hz, 15H), 2.45 (s, 6H), 1.60 (s, 19H), 1.26 (s, 48H), 0.88 (s, 9H). | Calc. 1358.87 |
| | | | | Found.1355.91 |
| 10) | TK-12 | 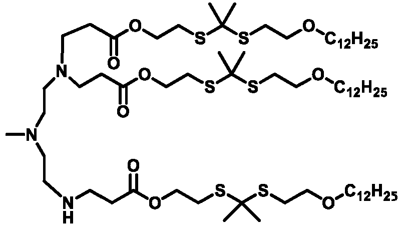 | ¹H NMR (300 MHz, CDCl3) δ 4.18 (dd, J = 27.0, 20.2 Hz, 4H), 3.60 (dd, J = 19.6, 12.9 Hz, 4H), 3.43 (t, J = 6.6 Hz, 4H), 2.85 (ddt, J = 20.0, 13.3, 6.5 Hz, 12H), 2.70 (t, J = 6.9 Hz, 3H), 2.61 - 2.35 (m, 12H), 2.23 (s, 3H), 1.99 (s, 1H), 1.72 - 1.49 (m, 16H), 1.26 (s, 47H), 0.88 (t, J = 6.3 Hz, 9H). | Calc.1344.87 |
| | | | | Found.1344.81 |
| 11) | TK-13 | 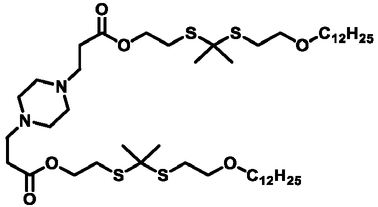 | ¹H NMR (300 MHz, CDCl3) δ 4.23 (s, 4H), 3.58 (s, 4H), 3.43 (s, 4H), 2.84 (s, 8H), 2.69 (s, 5H), 2.48 (s, 11H), 1.60 (s, 17H), 1.26 (s, 38H), 0.88 (s, 6H). | Calc.923.6 |
| | | | | Found.923.8 |
| 12) | TK-14 | 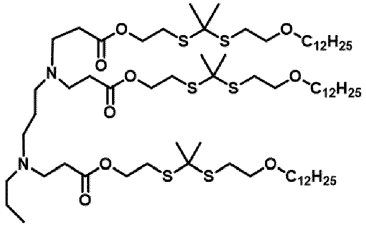 | ¹H NMR (300 MHz, CDCl3) δ 4.22 (s, 4H), 3.58 (s, 4H), 3.43 (s, 4H), 2.82 (s, 13H), 2.54 (dd, J = 14.5, 7.2 Hz, 1H), 2.45 (s, 9H), 1.60 (s, 18H), 1.26 (s, 45H), 0.88 (s, 9H). | Calc.1371.91 |
| | | | | Found.1371.91 |
| 13) | TK-15 | 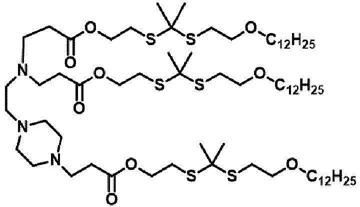 | ¹H NMR (300 MHz, CDCl3) δ 4.22 (s, 4H), 3.58 (s, 5H), 3.43 (s, 5H), 2.84 (s, 13H), 2.48 (s, 16H), 1.60 (s, 19H), 1.26 (s, 45H), 0.88 (s, 9H). | Calc.1370.90 |
| | | | | Found.1384.90 |
| 14) | TK-16 | 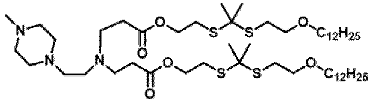 | ¹H NMR (300 MHz, CDCl3) δ 4.22 (s, 4H), 3.58 (s, 4H), 3.43 (s, 4H), 2.82 (s, 11H), 2.60 (s, 3H), 2.55 - 2.37 (m, 11H), 2.28 (s, 3H), 1.60 (s, 15H), 1.26 (s, 36H), 0.88 (s, 6H). | Calc.980.6 |
| | | | | Found.980.9 |
| 15) | TK-17 | 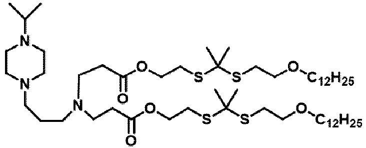 | ¹H NMR (300 MHz, CDCl3) δ 4.21 (s, 4H), 3.58 (s, 4H), 3.43 (s, 4H), 2.82 (s, 14H), 2.43 (s, 4H), 2.27 (s, 2H), 1.60 (s, 19H), 1.26 (s, 38H), 0.88 (s, 6H). | Calc.1023.7 |
| | | | | Found.1023.8 |
| 16) | TK-18 | 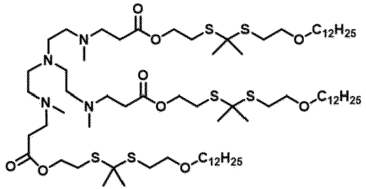 | ¹H NMR (300 MHz, CDCl3) δ 4.23 (s, 5H), 3.58 (s, 6H), 3.43 (s, 6H), 2.84 (s, 12H), 2.72 (s, 5H), 2.61 (s, 5H), 2.49 (s, 10H), 2.25 (s, 7H), 1.60 (s, 23H), 1.26 (s, 54H), 0.88 (s, 9H). | Calc.1443.98 |
| | | | | Found.1443.98 |
| 17) | TK-19 | 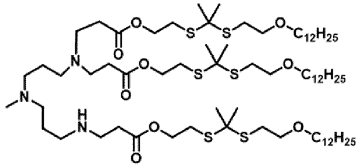 | ¹H NMR (300 MHz, CDCl3) δ 4.22 (s, 4H), 3.79 (s, 1H), 3.58 (s, 5H), 3.43 (s, 5H), 2.82 (s, 15H), 2.45 (s, 9H), 1.60 (s, 23H), 1.26 (s, 54H), 0.88 (s, 9H). | Calc.1400.93 |
| | | | | Found.1400.94 |
| 18) | TK-20 | 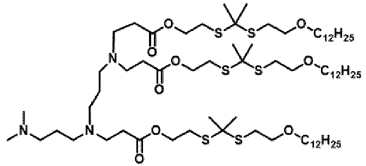 | ¹H NMR (300 MHz, CDCl3) δ4.21 (s, 6H), 3.57 (s, 6H), 3.42 (s, 6H), 2.80 (s, 19H), 2.39 (dt, J = 14.8, 7.6 Hz, 14H), 2.29 (s, 6H), 1.59 (s, 28H), 1.25 (s, 57H), 0.87 (s, 9H). | Calc. 1414.95 |
| | | | | Found.1414.95 |
| 19) | TK-21 | 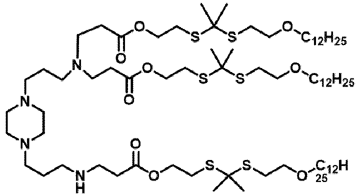 | ¹H NMR (300 MHz, CDCl3) δ 4.22 (s, 6H), 3.58 (s, 6H), 3.43 (s, 6H), 2.82 (s, 12H), 2.69 (s, 6H), 2.55 (s, 6H), 2.35 (s, 6H), 2.23 (s, 9H), 1.60 (s, 27H), 1.26 (s, 56H), 0.88 (s, 9H). | Calc. 1414.95 |
| | | | | Found. 1455.98 |
| 20) | TK- | 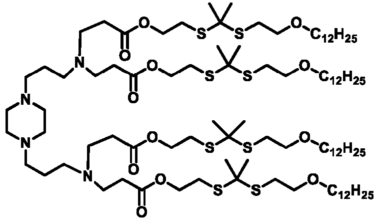 | ¹H NMR (300 MHz, CDCl3) δ 4.22 (s, 7H), 3.58 (s, 7H), 3.43 (s, 7H), 2.82 (s, 23H), 2.45 (s, 14H), 2.30 (s, 5H), 1.60 (s, 42H), 1.26 (s, 65H), 0.88 (s, 12H). | Calc. 1874.23 |
| | 21-4 | | | Found. 1874.24 |
| 21) | TK-22 | 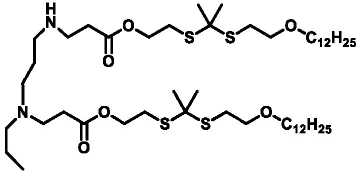 | ¹H NMR (300 MHz, CDCl₃) δ 4.25(q,4H), 3.60(s,4H), 3.45(s,4H), 2.84(m,12H), 2.66(s,2H), 2.55(s,2H),2.47(q,4H),2.38(t,2 H),1.62(s,13H),1.59(m,4H),1.4 6(q,2H),1.28(s,38H), 0.88(s,9H). | Calc. 953.6 |
| | | | | Found. 953.8 |
| 22) | TK-23 | 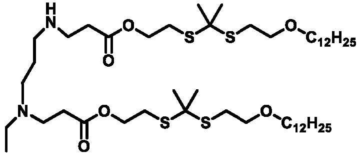 | ¹H NMR (300 MHz, CDCl₃) δ 4.22 (s,4H), 3.59(s,4H), 3.43(s,4H), 2.82(m,11H), 2.64(m,4H), 2.48(m,7H), 1.61(s, 18H), 1.26 (m,32H), 1.11(s,4H), 0.88(s,6H). | Calc.939.6 |
| | | | | Found. 939. 8 |
| 23) | TK-24 | 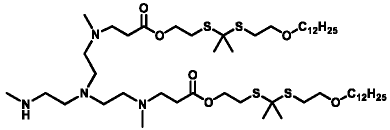 | 1H NMR (300 MHz, CDCl3) δ 4.23 (s, 4H), 3.58 (s, 4H), 3.43 (s, 4H), 2.94 - 2.77 (m, 7H), 2.71 (s, 4H), 2.61 (s, 7H), 2.46 (d, J = 12.0 Hz, 10H), 2.24 (s, 6H), 1.60 (s, 15H), 1.26 (s, 34H), 0.88 (s, 6H). | Calc. 1025.72 |
| | | | | Found. 1025.71 |
| 24) | TK-25 | 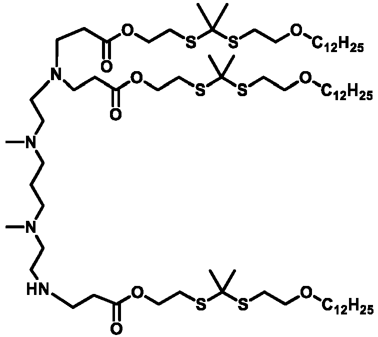 | 1H NMR (300 MHz, CDCl3) δ 5.30 (s, 2H), 4.22 (t, J = 6.3 Hz, 1H), 3.58 (t, J = 6.8 Hz, 1H), 3.43 (t, J = 6.7 Hz, 1H), 2.98 - 2.74 (m, 2H), 2.69 (s, 1H), 2.53 (s, 1H), 2.46 (s, 1H), 2.35 (s, 1H), 2.22 (d, J = 4.7 Hz, 1H), 1.60 (s, 3H), 1.26 (s, 4H), 0.88 (t, J = 6.6 Hz, 1H). | Calc. 1443.98 |
| | | | | Found. 1443.95 |

### Example 1b: Preparation of lipid complexes

In order to construct a delivery carrier with a high transfection efficiency and a good biocompatibility, the inventors prepared lipid complexes, and the specific method was as follows: the lipid compounds prepared in Example 1a, cholesterol, and DOPE were separately dissolved in trichloromethane to prepare mixed solutions with a concentration of 10 mg/mL; and the three mixed solutions were taken in proportion and evaporated to form a film, and the film was then ultrasonically dissolved with anhydrous ethanol, after which the dissolved film was dropwise added to the sodium acetate buffer solution containing 5 mg/mL DSPE-PEG2000 (200 mM, pH = 5.2) and stirred at 2000 rpm for 5 minutes to prepare a lipid complex. In this example, 16 lipid complexes with a mass ratio of lipid compound : cholesterol : DOPE : DSPE-PEG2000 of 16 : 4 : 4 : 1 in order were assembled according to the above method by selecting the lipid compounds prepared in Example 1a (TK-2, TK-3, TK-5, TK-6, TK-7, TK-8, TK-10, TK-11, TK-13, TK-14, TK-15, TK-16, TK-17, TK-18, TK-20, and TK-21).

The 16 lipid complexes prepared above were allowed to deliver RFP mRNA by the same method, and the RFP positive cell rate was detected for lipid compound screening. The specific method was as follows:
0.33 µg/mL RFP mRNA was added to a sodium acetate buffer solution (25 mM, pH = 5.2) and mixed with 15 times of its mass of the 16 lipid complexes prepared above, respectively, and after being mixed uniformly, the mixtures were incubated for 15 minutes, thus completing the self-assembly of lipid compound/RFP mRNA. 50 µL of each of the self-assembled systems was taken, added to HeLa cells, and incubated for 8 h, after which the medium was replaced with a fresh one. After 40 h, the RFP positive cell rate was analyzed by a flow cytometer.

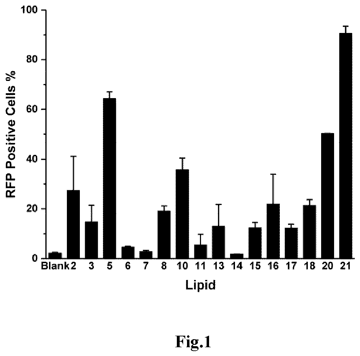

The results were seen in Fig. 1, which showed that all the 16 lipid compounds could deliver RFP mRNA, among which lipid compound TK-21 (also known as BAmP-TK-12) had the highest RFP positive cell rate (see Fig. 1).

### Example 2: Study on structure-activity relationship of BAmP-TK-12 lipid compound

### 1. Effect of thioketal structure on the ability of BAmP-TK-12 to respond to ROS stimulation

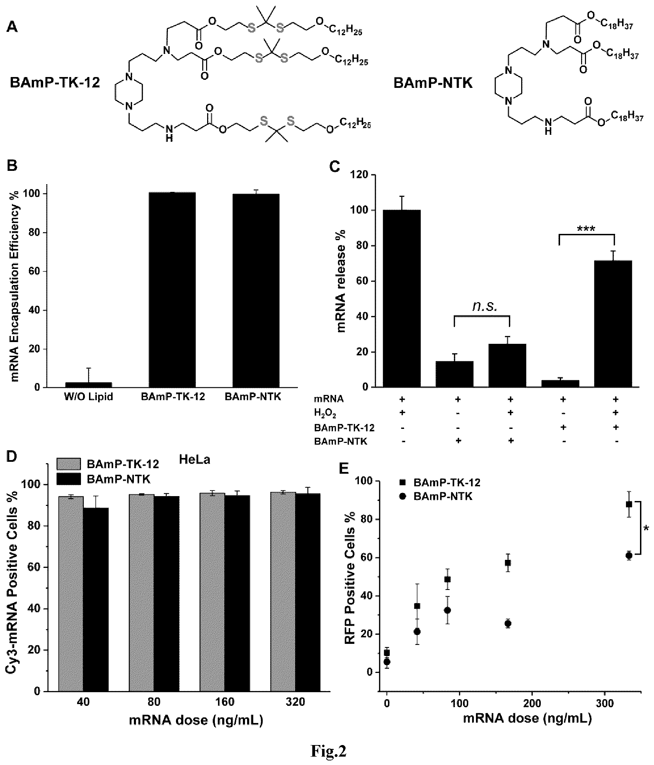

In the present invention, by introducing an ROS-cleavable thioketal structure into a hydrophobic lipid tail of a lipid compound, the lipid compound could be degraded under intracellular ROS stimulation to release the encapsulated mRNA. In order to verify the effect of the thioketal structure on the ability of BAmP-TK-12 to respond to ROS stimulation, a BAmP-NTK lipid compound with the same amine head structure BAmP but without thioketal in the hydrophobic tail was first synthesized (see Fig. 2A for the structural formula). That is, the amine head BAmP and the hydrophobic tail octadecyl acrylate (CAS: 4813-57-4) were mixed at a molar ratio of 1 : 3.3 and heated at 80°C for 24 h, and the crude product was purified on a silica gel column with dichloromethane/methanol as an eluent to obtain lipid compound BAmP-NTK.

In order to compare BAmP-TK-12 with BAmP-NTK in terms of the mRNA encapsulation efficiency and the ability to respond to ROS stimulation, BAmP-TK-12 and BAmP-TK were separately used to self-assemble with one fifteenth of its mass of RFP mRNA in a sodium acetate buffer solution (25 mM, pH = 5.2), and after incubation for 15 min, the concentration of unencapsulated mRNA was measured by using iQuant broad range RNA quantitation kit (GeneCopoeia). The measured results were normalized to a corresponding concentration obtained by directly dissolving an equal amount of mRNA in a buffer as 100%, and the results were seen in Fig. 2B. The mRNA encapsulation efficiencies of BAmP-TK-12 and BAmP-TK were both close to 100%. In order to simulate the process of ROS stimulating lipid compound particles to rupture and release mRNA in diseased cells, H₂O₂ at a final concentration of 25 mM was added to BAmP-TK-12/RFP mRNA and BAmP-TK/RFP mRNA, and after incubation at 37°C for 24 h, the released mRNA concentration was measured. The measured results were normalized to a corresponding concentration obtained by directly dissolving an equal amount of mRNA in a buffer as 100%, and the results were seen in Fig. 2C. It could be seen from the figure that after treatment with H₂O₂, BAmP-NTK only released a small amount of mRNA, whereas BAmP-TK-12 released mRNA significantly, which proved that the thioketal structure endowed BAmP-TK-12 with the ability to release mRNA in response to ROS stimulation.

In order to compare the mRNA delivery performances of BAmP-TK-12 and BAmP-NTK, different concentrations of BAmP-TK-12 and BAmP-NTK were used separately to self-assemble with one fifteenth of its mass of Cy3-labeled mRNA (Cy3-mRNA) in a sodium acetate buffer solution (25 mM, pH = 5.2), and after incubation for 15 min, they were added to HeLa cells. After incubation for 8 h, the Cy3 positive cell rate was analyzed by a flow cytometer. The experimental results were seen in Fig. 2D. The results showed that under various concentration conditions, the BAmP-TK-12 and BAmP-NTK complexes with mRNA were close in Cy3 positive cell rate, which proved that the two were similar in the efficiency of entering cells. Furthermore, BAmP-TK-12 and BAmP-NTK were applied to the delivery of RFP mRNA as described in the above method, and the results were as seen in Fig. 2E. By comparing the RFP positive cell rate, it could be seen that under various concentration conditions, since BAmP-TK-12 could be ruptured to release mRNA in response to a higher level of ROS in HeLa cells, the delivery efficiency thereof was higher than that of BAmP-NTK.

### 2. Effect of the number of hydrophobic long chains on delivery efficiency

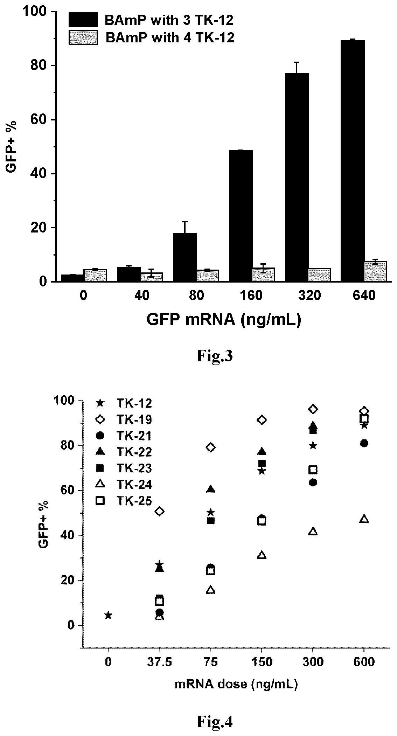

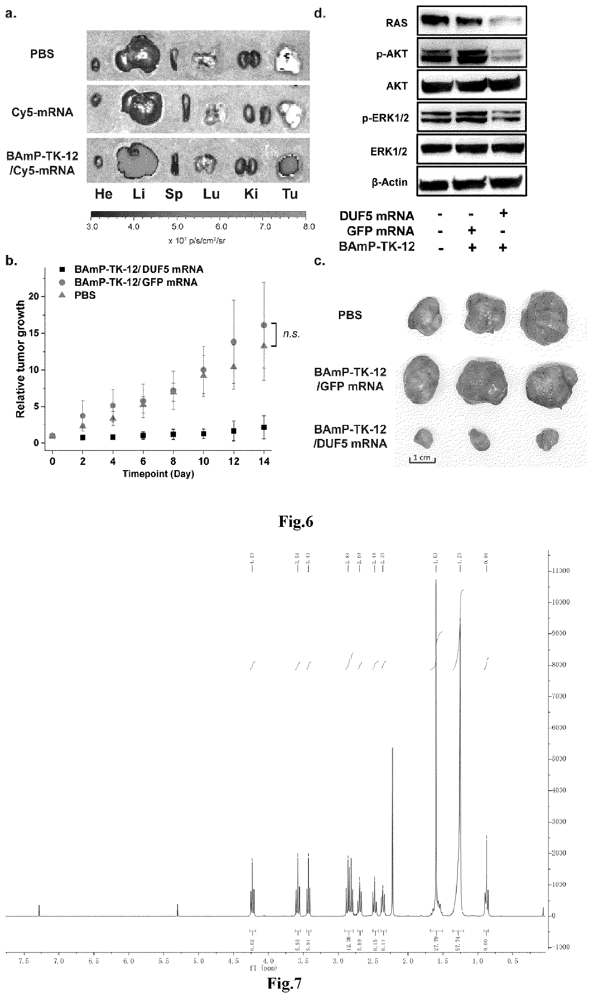

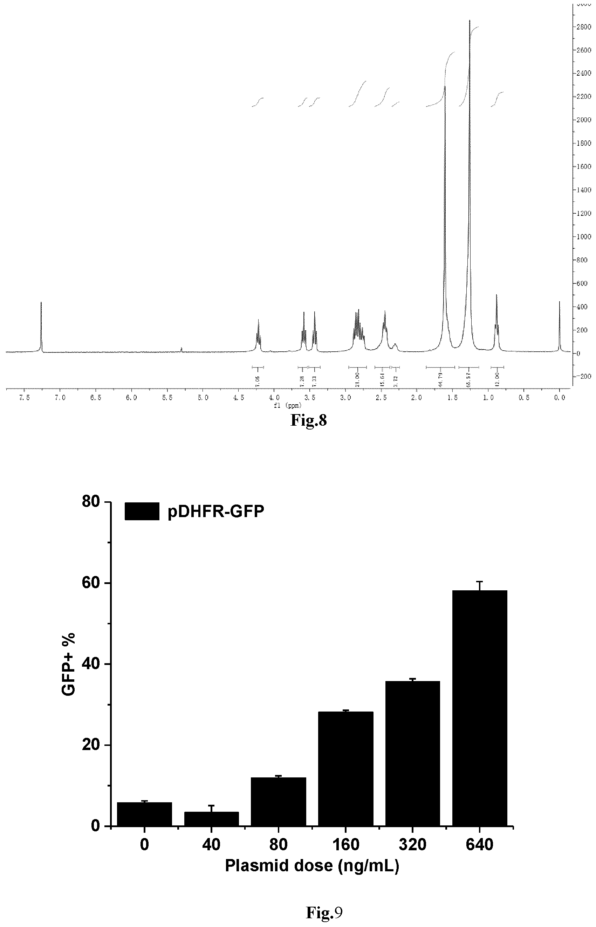

In the present invention, a series of lipid compounds were obtained by an addition reaction between different amine heads and hydrophobic lipid tails, and the N atoms in the amine heads acted as proton acceptors and could be protonated, so that such lipid compound nanoparticles were positively charged as a whole and could thus undergo an electrostatic interaction with negatively charged mRNAs, cell membranes, and lysosomal membranes, so the N structure that was not completely substituted with hydrophobic tails was an important reason for the lipid compound to be capable of effectively encapsulating and delivering mRNA. In order to study how different amine head structures affected the mRNA delivery ability of lipid compounds, BAmP-TK-12 with 3 thioketal hydrophobic tails and TK-21-4 with 4 thioketal hydrophobic tails (i.e., BAmP with 4 thioketal hydrophobic tails) were synthesized. An NMR spectrum of TK-21 (BAmP-TK-12) was seen in Fig. 7, and an NMR spectrum of TK-21-4 (BAmP with 4 TK-12) was seen in Fig. 8. The mRNA delivery abilities thereof were compared. Different concentrations of BAmP-TK-12 and TK-21-4 were separately used to self-assemble with one fifteenth of its mass of GFP mRNA in a sodium acetate buffer solution (25 mM, pH = 5.2), and after incubation for 15 min, they were added to HeLa cells. After incubation for 8 h, the medium was changed, and after 36 h, the GFP positive cell rate was analyzed by a flow cytometer. The experimental results were seen in Fig. 3. The results showed that under various concentration conditions, BAmP-TK-12 could efficiently deliver GFP mRNA, whereas TK-21-4 could not deliver GFP mRNA. The experiment proved that the N in the amine head that was not completely substituted with hydrophobic tails was an important reason for the lipid compound to be capable of effectively encapsulating and delivering mRNA.

### 3. Delivery efficiencies of different lipid compounds

In order to further prove that the N in the amine head that was not completely substituted with hydrophobic tails was an important reason for the lipid compound to be capable of effectively encapsulating and delivering mRNA, more lipid compounds with incomplete substitutions of N in the amine head were used for GFP mRNA delivery. Lipid compounds TK-12, TK-19, TK-21, TK-22, TK-23, TK-24, and TK-25, cholesterol, and DOPE were separately dissolved in trichloromethane to prepare mixed solutions with a concentration of 10 mg/mL; and the three mixed solutions were taken in proportion and evaporated to form a film, and the film was then ultrasonically dissolved with anhydrous ethanol, after which the dissolved film was dropwise added to the sodium acetate buffer solution containing 5 mg/mL DSPE-PEG2000 (200 mM, pH = 5.2) and stirred at 2000 rpm for 5 minutes to prepare a lipid complex. 7 lipid complexes with a mass ratio of lipid compound : cholesterol : DOPE : DSPE-PEG2000 of 16 : 4 : 4 : 1 in order were assembled according to the above method using one of the lipid compounds TK-12, TK-19, TK-21, TK-22, TK-23, TK-24, and TK-25 as prepared in this example. 37.5, 75, 150, 300, and 600 ng/mL GFP mRNA were added to sodium acetate buffer solutions (25 mM, pH = 5.2) and mixed with 15 times of its mass of the 7 lipid complexes prepared above, respectively, and after being mixed uniformly, the mixtures were incubated for 15 minutes, thus completing the self-assembly of lipid compound/GFP mRNA. 50 µL of each of the self-assembled systems was taken, added to HeLa cells, and incubated for 8 h, after which the medium was replaced with a fresh one. After 40 h, the GFP positive cell rate was analyzed by a flow cytometer. The results were seen in Fig. 4. The experiment proved that the lipid compounds with incomplete substitutions of N in the amine head could all efficiently deliver mRNA.

### Example 3: Intracellular delivery of DUF5 mRNA by BAmP-TK-12 lipid compound and inhibition of cancer cell proliferation

### 1. Inhibitory effect of BAmP-TK-12/DUF5 mRNA on colon cancer cells HCT-116

The gene encoding DUF5 protein was DUF5 gene, and the protein thereof was a fragment at 3596-4079 of *V. vulnificus* rtxA1 gene (GI: 27366913; VV2_0479). See PDB ID: 5W6L for the sequence. pcDNA3.1 was used as a vector for recombining and cloning DUF5 gene to obtain recombinant expression vector pcDNA3.1-DUF5 plasmid of DUF5 protein. pcDNA3.1-DUF5 plasmid was linearized with XhoI endonuclease (NEB), and the linearized plasmid as a template was transcribed using RiboMAX^{™} Large Scale RNA Production System-T7 (Promega) kit and then tailed with *E. coli* Poly(A) Polymerase (NEB) to obtain DUF5 mRNA.

As described in the above method, different doses of DUF5 mRNA or GFP mRNA were mixed uniformly with 15 times of its mass of BAmP-TK-12 lipid compound in sodium acetate buffer solutions (25 mM, pH = 5.2), and after incubation for 15 min, they were added to colon cancer cells, such that the final concentrations thereof were 0, 150, 300, and 600 ng/mL. Cell viability was detected after incubation for 8 h. CellTiter-Glo^{®} Luminescent Cell Viability Assay Kit (Promega) was used to detect cell viability. The principle thereof was that luciferase catalyzed an oxidation reaction of fluorescein in the presence of ATP and produced chemiluminescence at the same time, wherein the chemiluminescence intensity was directly proportional to the ATP content in living cells, so it could indicate the cell vitality. The chemiluminescence intensity was measured by a microplate reader, the percentage of the chemiluminescence intensity in cells after the experimental treatment relative to the blank control group was calculated, and the cell survival rate was obtained. The experimental results were as shown in Fig. 5a. Compared with cells treated with BAmP-TK-12/GFP mRNA, the viability of HCT-116 cells treated with BAmP-TK-12/DUF5 mRNA decreased significantly, and the killing effect gradually increased with the increase of the concentration of DUF5 mRNA. It was proved that BAmP-TK-12 successfully delivered DUF5 mRNA into the colon cancer cells HCT-116 cells and inhibited the proliferation thereof.

In order to explore the effect of DUF5 mRNA on RAS protein and its downstream signaling pathways in cancer cells, the lysate of the HCT-116 cells incubated with BAmP-TK-12/DUF5 mRNA or BAmP-TK-12/GFP mRNA for 8 h or those untreated was collected for a Western blot experiment. The primary antibodies used were anti-RAS (RAS Antibody, CST #3965), anti-Phospho-p44/42 MAPK (phosphorylated ERK1/2, Thr202/Tyr204, 197G2, CST #4377), anti-p44/42 MAPK (ERK1/2, L34F12, CST #4696), anti-Phospho-Akt (Phospho-Akt, Thr308, CST #9275), anti-AKT (anti-pan-AKT, Abcam #ab8805), and anti-β-Actin (anti-β-Actin, 8H10D10, CST #3700). The secondary antibodies used were anti-rabbit (anti-rabbit IgG, HRP-linked antibody, CST #7074), and anti-mouse (anti-mouse IgG, HRP-linked antibody, CST #7076). The experimental results were seen in Fig. 5e. Compared with the other control groups, after treatment with BAmP-TK-12/DUF5 mRNA, significant cleavage of RAS was found in the HCT-116 cells, which in turn led to a decrease in the level of p-ERK (phosphorylated ERK protein) (the expression level of ERK protein was not affected); in addition, the level of p-AKT (phosphorylated AKT protein) was also decreased (the expression level of AKT protein was not affected). It was proved here that after DUF5 mRNA was delivered by BAmP-TK-12 into HCT-116 cells with KRASG13D mutation, the translated DUF5 protein specifically recognized and cleaved RAS protein, blocking its activation on phosphorylation of ERK protein and AKT protein, thus inhibiting the cell viability and proliferation process of the colon cancer cells HCT-116, demonstrating the regulatory function of DUF5 on RAS/RAF/MEK/ERK and PI3K/AKT signaling pathways.

### 2. Inhibitory effect of BAmP-TK-12/DUF5 mRNA on colon cancer cells SW480 and human non-small cell lung cancer cells A549

The previous experiments had proved that BAmP-TK-12/DUF5 mRNA had an inhibitory effect on the proliferation of colon cancer HCT-116 cells with KRAS*^{G13D}* mutation. In order to further prove the therapeutic effect of this system on all kinds of cancerous cells caused by KRAS mutations, it was applied to colon cancer cells SW480 with KRAS*^{G12V}* mutation and human non-small cell lung cancer cells A549 with KRAS*^{G12S}* mutation.

As described in 1., different doses of DUF5 mRNA or GFP mRNA were mixed uniformly with 15 times of its mass of BAmP-TK-12 lipid compound in sodium acetate buffer solutions (25 mM, pH = 5.2), and after incubation for 15 min, they were added to colon cancer cells SW480 or non-small cell lung cancer cells A549, such that the final mRNA concentrations thereof were 0, 150, 300, and 600 ng/mL. Cell viability was detected after incubation for 8 h. In addition, the cell lysate after 8 hours of treatment was collected for a Western blot experiment. The experimental results were seen in Figs. 5b, c, and d. Compared with other control groups, after treatment with BAmP-TK-12/DUF5 mRNA, the viabilities of SW480 and A549 cells decreased significantly, and when the concentration of DUF5 mRNA was only 150 ng/mL, after 8 hours of rapid killing, the cell survival rate decreased to 40%. The intrinsic mechanism thereof was that RAS was cleaved by DUF5 protein, which in turn led to a decrease in the level of phosphorylated ERK protein (the expression level of ERK protein was not affected); in addition, the level of phosphorylated AKT protein was also decreased (the expression level of AKT protein was not affected).

It was proved here that DUF5 mRNA was delivered into cancer cells by BAmP-TK-12 and translated into DUF5 protein, it could efficiently cleave all of the RAS proteins containing G13D, G12V, and G12S mutations, inhibiting its activation on phosphorylation of downstream ERK and AKT Akt proteins, thus inhibiting the proliferation of different types of cancer cells, indicating that BAmP-TK-12/DUF5 mRNA had broad-spectrum therapeutic effects on various cancers caused by RAS mutations.

### Example 4: In vivo delivery of DUF5 mRNA by BAmP-TK-12 lipid compound and tumor inhibition

### 1. In vivo distribution of BAmP-TK-12/DUF5 mRNA

pcDNA3.1-DUF5 plasmid was linearized with XhoI endonuclease, and the linearized plasmid as a template was transcribed using HyperScribe^{™} T7 High Yield Cy5 RNA Labeling Kit (Apexbio) to obtain Cy5-labeled DUF5 mRNA (Cy5-DUF5 mRNA). BAmP-TK-12/Cy5-DUF5 mRNA complex was prepared as described in the above method.

In order to obtain a tumor-bearing mouse model heterotransplanted with colon cancer HCT-116 cells, 5.0 x 10⁶ HCT-116 cells (suspended in a PBS solution) were inoculated under the right armpit of female Nu/Nu mice aged 4-6 weeks. When the tumor volume reached 200 mm³ (the tumor volume calculation formula was volume (mm³) = (l x w²)/2, wherein l was the tumor length and w was the tumor width), the mice were injected with 200 µg of BAmP-TK-12/Cy5-DUF5 mRNA, or 200 µg of Cy5-DUF5 mRNA, or 200 µL of a PBS solution via the tail vein. 6 h after tail vein injection, the mice were euthanized and then dissected to obtain heart, liver, spleen, lung, kidney, and tumor tissues, which were imaged at an excitation wavelength of 620 nm and an emission wavelength of 660 nm. The experimental results were seen in Fig. 6a. As shown in the figure, Cy5-DUF5 mRNA not complexed with BAmP-TK-12 lipid compound had no distribution in any of the major organs and tumor tissues, and the signal intensity thereof was similar to that of the blank control group injected with PBS. In contrast, BAmP-TK-12/Cy5-DUF5 mRNA was not only accumulated in the liver, but also extensively distributed in tumor tissues. It was proved here that BAmP-TK-12/Cy5-DUF5 mRNA could accumulate in tumor tissues after *in vivo* circulation and had the potential to inhibit tumor tissues.

### 2. Inhibitory effect of BAmP-TK-12/DUF5 mRNA on in vivo tumors

A tumor-bearing mouse model heterotransplanted with colon cancer HCT-116 cells was established as described in 1. When the tumor volume reached 100 mm³, the mice were injected with BAmP-TK-12/DUF5 mRNA, or BAmP-TK-12/GFP mRNA, or a PBS solution via the tail vein, at a dose of 200 µg/mouse, at a dosage frequency of 2 days once, for six times in total. At the same time, the tumor volume and mouse weight were monitored. After reaching the end of the experiment, blood was collected, and serum was taken for blood biochemical detection. The mice were euthanized and dissected to obtain tumor tissues.

From the relative growth curve of the tumor volume in mice (see Fig. 6b), it could be seen that tumors proliferated rapidly in the mice treated with BAmP-TK-12/GFP mRNA or PBS solution by tail vein injection, whereas the tumor volume in the mice treated with BAmP-TK-12/DUF5 mRNA only increased slightly relative to the beginning of the experiment. From the photos of the tumor tissues in the mice (see Fig. 6c), it could be seen that at the end of the experiment, the tumor volume in the mice treated with BAmP-TK-12/DUF5 mRNA was much smaller than that in the control group, which proved that BAmP-TK-12/DUF5 mRNA effectively inhibited the proliferation of colorectal cancer malignancies *in vivo.*

A part at the periphery of the tumor tissues in the mice was cut and ground by a tissue homogenizer to obtain a tumor cell lysate for a Western blot experiment. The antibodies used were as mentioned above. The Western blot experiment (as shown in Fig. 6d) showed that BAmP-TK-12/GFP mRNA or PBS had no effect on the expression amount of RAS protein, its regulated AKT and ERK, and its phosphorylated proteins p-AKT and p-ERK in cells at the tumor site in the mice, whereas BAmP-TK-12/DUF5 mRNAresulted in a decreased expression amount of p-AKT and p-ERK by cleaving RAS protein. It was proved here that BAmP-TK-12/DUF5 mRNA mediated the enzymatic hydrolysis of RAS protein and the inactivation of the downstream signaling pathways *in vivo,* thus achieving the anti-tumor function.

### Example 5: Experiment of delivery of different plasmids by lipid compound of the present invention

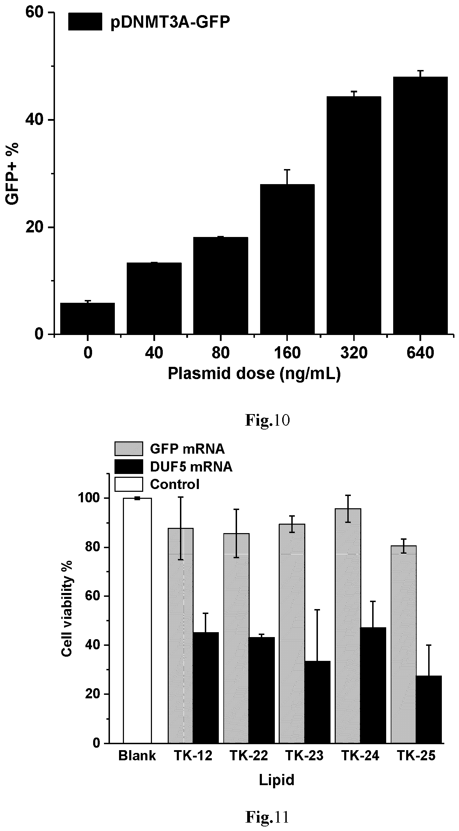

**1. Experiment of delivery of pDHFR-GFP (5185 bp) plasmid and pDNMT3A-GFP (9746 bp) plasmid:** Lipid compound TK-21, cholesterol, and DOPE were separately dissolved in trichloromethane to prepare mixed solutions with a concentration of 10 mg/mL; and the three mixed solutions were taken in proportion and evaporated to form a film, and the film was then ultrasonically dissolved with anhydrous ethanol, after which the dissolved film was dropwise added to the sodium acetate buffer solution containing 5 mg/mL DSPE-PEG2000 (200 mM, pH = 5.2) and stirred at 2000 rpm for 5 minutes to prepare a lipid complex. The mass ratio of lipid compound : cholesterol : DOPE : DSPE-PEG2000 in the lipid complex was 16 : 4 : 4 : 1 in order. 40, 80, 160, 320, and 640 ng/mL pDHFR-GFP (5185 bp) plasmid were added to sodium acetate buffer solutions (25 mM, pH = 5.2) and mixed with 15 times of its mass of the TK-21 lipid complex prepared above, respectively, and after being mixed uniformly, the mixtures were incubated for 15 minutes, thus completing the self-assembly of TK-21/pDHFR-GFP plasmid. 50 µL of each of the self-assembled systems was taken, added to HeLa cells, and incubated for 8 h, after which the medium was replaced with a fresh one. After 40 h, the GFP positive cell rate was analyzed by a flow cytometer. The results were seen in Fig. 9. Similarly, the TK-21 nanolipid complex prepared above was mixed with pDNMT3A-GFP (9746 bp) plasmid for delivery to HeLa cells, and the GFP positive cell rate was detected. The results were seen in Fig. 10. The results showed that the lipid compound of the present invention, such as TK-21, was suitable for effectively delivering plasmids with various lengths.

### Example 6: Experiment of delivery of different mRNAs by lipid compound of the present invention

**1. DUF5 mRNA delivery experiment:** The lipid compound, cholesterol, and DOPE were separately dissolved in trichloromethane to prepare mixed solutions with a concentration of 10 mg/mL; and the three mixed solutions were taken in proportion and evaporated to form a film, and the film was then ultrasonically dissolved with anhydrous ethanol, after which the dissolved film was dropwise added to the sodium acetate buffer solution containing 5 mg/mL DSPE-PEG2000 (200 mM, pH = 5.2) and stirred at 2000 rpm for 5 minutes to prepare a lipid complex. In this example, 5 lipid complexes with a mass ratio of lipid compound : cholesterol : DOPE : DSPE-PEG2000 of 16 : 4 : 4 : 1 in order were assembled according to the above method by selecting one of the prepared lipid compounds TK-12, TK-22, TK-23, TK-24, and TK-25. 0.33 µg/mL DUF5 mRNA or GFP mRNA was added to a sodium acetate buffer solution (25 mM, pH = 5.2) and mixed with 15 times of its mass of the prepared 5 lipid complexes, respectively, and after being mixed uniformly, the mixtures were incubated for 15 minutes, thus completing the self-assembly of lipid compound/mRNA. 50 µL of each of the self-assembled systems was taken and added to HeLa cells. After incubation for 8 h, the corresponding cell viability was determined, and the percentage of cell viability was normalized to the blank well. The results were seen in Fig. 11.

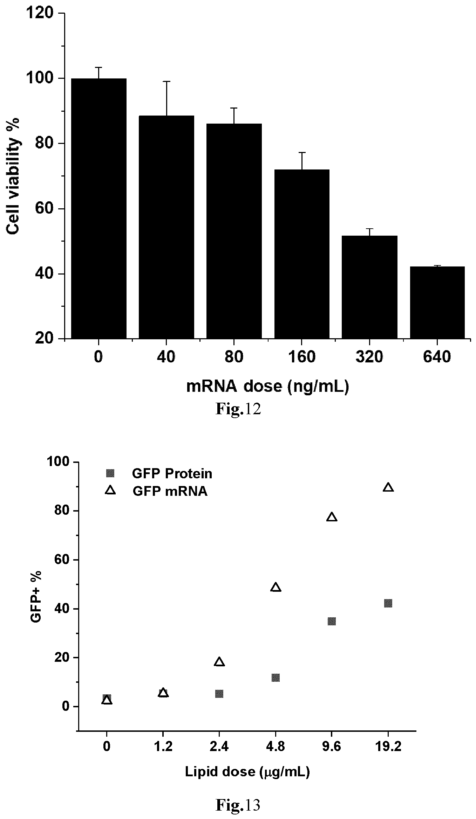

**2. Experiment of delivery of p53 mRNA by TK-21:** Similarly, TK-21 nanolipid complex was prepared and mixed with 40, 80, 160, 320, and 640 ng/mL p53 mRNA, which was 1/15 of the mass of TK-21, for delivery to HeLa cells. After incubation for 36 h, the cell viability was detected. The results were seen in Fig. 12, which proved that TK-21 could effectively deliver p53 mRNA and had a killing effect on the cells., and TK-21 was suitable for effectively delivering various functional mRNAs.

### Example 7: Experiment of delivery of nucleic acids and proteins by lipid compound of the present invention

**1. GFP protein delivery and its comparison with nucleic acid delivery:** TK-21, cholesterol, and DOPE were dissolved in trichloromethane to prepare mixed solutions with a concentration of 10 mg/mL; and the three mixed solutions were taken in proportion and evaporated to form a film, and the film was then ultrasonically dissolved with anhydrous ethanol, after which the dissolved film was dropwise added to the sodium acetate buffer solution containing 5 mg/mL DSPE-PEG2000 (200 mM, pH = 5.2) and stirred at 2000 rpm for 5 minutes to prepare a lipid complex, wherein the mass ratio of TK-21 : cholesterol : DOPE : DSPE-PEG2000 was 16 : 4 : 4 : 1 in order. 1.2, 2.4, 4.8, 9.6, and 19.2 µg/mL TK-21 lipid complexes prepared above were added to DMEM buffer solutions, which were respectively mixed with GFP protein, which was 0.6 times of its mass, and after being mixed uniformly, the mixtures were incubated for 15 minutes, thus completing the self-assembly of lipid compound/GFP protein. 50 µL of each of the self-assembled systems was taken and added to HeLa cells. After incubation for 6 h, the GFP positive cell rate was analyzed by a flow cytometer. The results were seen in Fig. 13, in which the lipid compound of the present invention had a stronger ability to deliver nucleic acids than deliver proteins at the same lipid complex concentration.

The above description is only preferred embodiments of the present invention; however, the scope of protection of the present invention is not limited thereto. Any changes or substitutions readily conceivable to those familiar with the technical field within the technical scope disclosed by the present invention should be covered by the scope of protection of the present invention. Therefore, the scope of protection of the present invention should be based on the scope of protection of the claims.

## Claims

1. Use of a lipid compound of formula (I) as a drug delivery carrier for preparing a nucleic acid drug, wherein the drug delivery carrier delivers the nucleic acid drug to a target cell for the intracellular release of the nucleic acid drug in the target cell,
wherein the lipid compound of formula (I) is 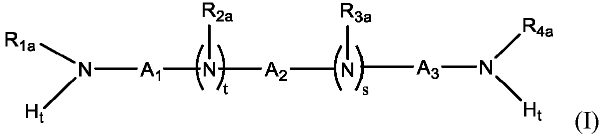 wherein
R₁ₐ, R₂ₐ, R₃ₐ, and R₄ₐ are each independently hydrogen, a monovalent aliphatic group, a monovalent heteroaliphatic group, a monovalent aromatic group, a monovalent heteroaromatic group, or Ht;
t and s are each independently 0 or 1, and when t or s is zero, it means that the part is directly a single bond;
A₁, A₂, and A₃ are each independently a single bond, a divalent aliphatic group, a divalent heteroaliphatic group, a divalent aromatic group, or a divalent heteroaromatic group, or a combination of two of the above;
each Ht is independently at each occurrence -R₁-X-R₂-Y-R₃-Z-R₄,
wherein
each R₁ is independently at each occurrence a divalent aliphatic group, a divalent heteroaliphatic group, a divalent aromatic group, or a divalent heteroaromatic group;
each X is independently at each occurrence 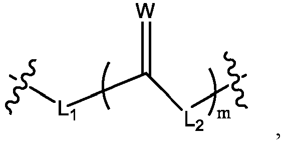 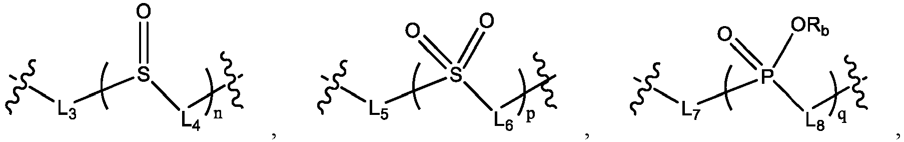
or 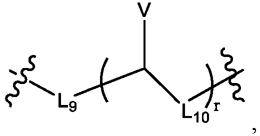 wherein
m, n, p, q, and r are each independently 1-6;
W is O, S, or NR_{c};
L₁, L₃, L₅, L₇, and L₉ are directly connected to R₁ or R₂ and are each independently a single bond, O, S, or NR_{d};
L₂, L₄, L₆, L₈, and L₁₀ are each independently a bond, O, S, or NRₑ;
V is an aliphatic group, OR_{f}, SR_{g}, or NRₕRᵢ,
wherein R_{b}, R_{c}, R_{d}, Rₑ, R_{f}, R_{g}, Rₕ, and Rᵢ are each independently hydrogen, hydroxyl, an oxyaliphatic group, a monovalent aliphatic group, a monovalent heteroaliphatic group, a monovalent aromatic group, or a monovalent heteroaromatic group;
Y and Z are each independently at each occurrence S or O;
each R₂ is independently at each occurrence a single bond, a divalent aliphatic group, a divalent heteroaliphatic group, a divalent aromatic group, or a divalent heteroaromatic group;
each R₃ is independently at each occurrence a single bond, a divalent aliphatic group, a divalent heteroaliphatic group, a divalent aromatic group, or a divalent heteroaromatic group; and
each R₄ is independently at each occurrence a hydrophobic group;
and N atoms in the backbone structure of formula (I) are optionally cationized;
provided that at least one of R₁ₐ and R₄ₐ is hydrogen.

2. The use according to claim 1, wherein the lipid compound is in an ionizable form, thereby forming the following structures: 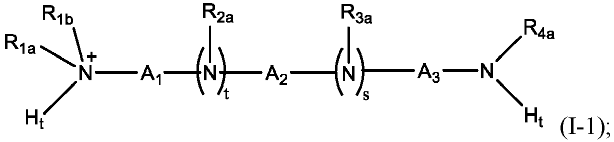 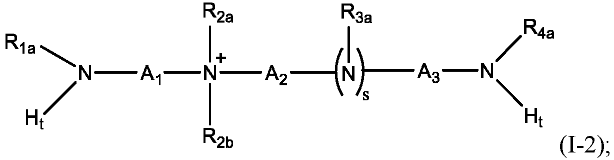 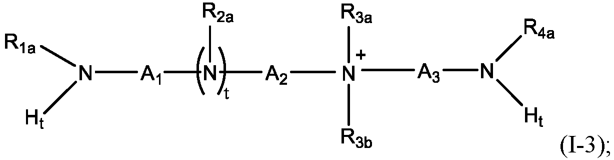 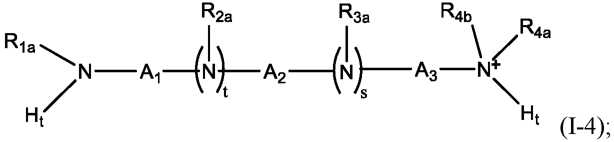 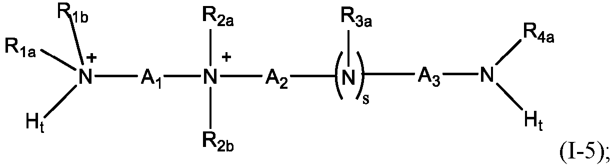 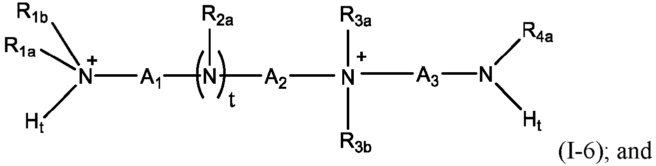 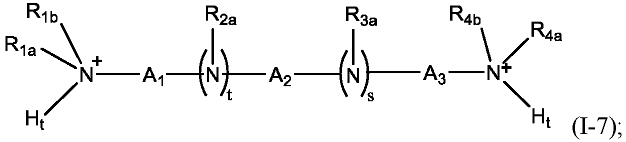 and contains corresponding counter ions, wherein R_{1b}, R_{2b}, R_{3b}, and R_{4b} are each independently hydrogen, a monovalent aliphatic group, a monovalent heteroaliphatic group, a monovalent aromatic group, a monovalent heteroaromatic group, or Ht, and the remaining variables are as defined in formula (I).

3. The use according to claim 1, wherein R₁ₐ or R₄ₐ is hydrogen; or R₁ₐ and R₄ₐ are each hydrogen.

4. The use according to claim 1, wherein t and s are both 0; or t is 0 and s is 1; or t is 1 and s is 0; preferably, t and s are both 0; R₁ₐ is Ht and R₄ₐ is hydrogen; or t and s are both 0; and R₁ₐ is hydrogen and R₄ₐ is Ht.

5. The use according to claim 1, wherein the fragment 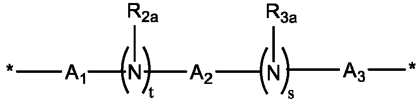 is
• A₁-A₃, wherein A₁ and A₃ are each independently a C₁-C₆ divalent aliphatic group (e.g., a C₁-C₄ divalent aliphatic group, e.g., a C₁-C₆ divalent alkyl, preferably a C₁-C₄ divalent alkyl); or
• A₁-(NR₂ₐ)-A₃, wherein A₁ and A₃ are each independently a C₁-C₆ divalent aliphatic group (e.g., a C₁-C₄ divalent aliphatic group, e.g., a C₁-C₆ divalent alkyl, preferably a C₁-C₄ divalent alkyl), and R₂ₐ is hydrogen, a monovalent aliphatic group, or a monovalent heteroaliphatic group; preferably hydrogen, a monovalent C₁₋₆ alkyl, or a monovalent C₁₋₆ heteroalkyl, e.g., amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆ alkylthio C₁₋₆ alkyl, or mercapto C₁₋₆ alkyl; or
• A₁-(NR₂ₐ)-A₂-(NR₂₆)-A₃, wherein A₁, A₂, and A₃ are each independently a C₁-C₆ divalent aliphatic group (e.g., a C₁-C₄ divalent aliphatic group, e.g., a C₁-C₆ divalent alkyl, preferably a C₁-C₄ divalent alkyl); and R₂ₐ and R_{2b} are each independently hydrogen, a monovalent aliphatic group, or a monovalent heteroaliphatic group; preferably hydrogen, a monovalent C₁₋₆ alkyl, or a monovalent C₁₋₆ heteroalkyl, e.g., amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆ alkylthio C₁₋₆ alkyl, or mercapto C₁₋₆ alkyl; or
• A₁-A₂-A₃, wherein A₁ and A₃ are each independently a C₁-C₆ divalent aliphatic group (e.g., a C₁-C₄ divalent aliphatic group, e.g., a C₁-C₆ divalent alkyl, preferably a C₁-C₄ divalent alkyl); and A₂ is a divalent cyclic heteroaliphatic group, such as a 3- to 7-membered monocyclic heteroaliphatic group containing 1-3 heteroatoms selected from oxygen, nitrogen and sulfur, preferably divalent piperazinyl;
wherein the asterisk * represents the connection of the terminal N atom in formula (I) or (I-1) to (I-7);
preferably, the fragment 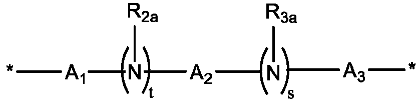 is
• -(CH₂)ₘ₁-, wherein m1 is an integer of 2-12, preferably 2-10 or 3-8; or an integer of 3; or
• -(CH₂)ₘ₂-(NR₂ₐ)-(CH₂)ₘ₃-, wherein m2 and m3 are each independently an integer of 1-6; preferably an integer of 2-6, or 2-4, or 2, or 3; R₂ₐ is hydrogen, a monovalent C₁₋₆ alkyl, amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆ alkylthio C₁₋₆ alkyl, or mercapto C₁₋₆ alkyl; or
• -(CH₂)ₘ₂-(NR₂ₐ)-(CH₂)ₘ₄-(NR₂₆)-(CH₂)ₘ₃, wherein m2, m3, and m4 are each independently an integer of 1-6; preferably an integer of 2-6, or 2-4, or 2, or 3; R₂ₐ and R_{2b} are each independently hydrogen, a monovalent C₁₋₆ alkyl, amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆ alkylthio C₁₋₆ alkyl, or mercapto C₁₋₆ alkyl; or
• -(CH₂)ₘ₂-A₂-(CH₂)ₘ₃, wherein m2 and m3 are each independently an integer of 1-6; preferably an integer of 2-6, or 2-4, or 2, or 3; and A₂ is a divalent cyclic heteroaliphatic group, such as a 3- to 7-membered monocyclic heteroaliphatic group containing 1-3 heteroatoms selected from oxygen, nitrogen and sulfur, preferably divalent piperazinyl;
wherein the asterisk * represents the connection of the terminal N atom in formula (I) or (I-1) to (I-7).

6. The use according to claim 1, wherein R₁ is a C₁₋₆ divalent aliphatic group or a C₁₋₆ divalent heteroaliphatic group.

7. The use according to claim 1, wherein X is 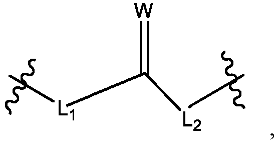 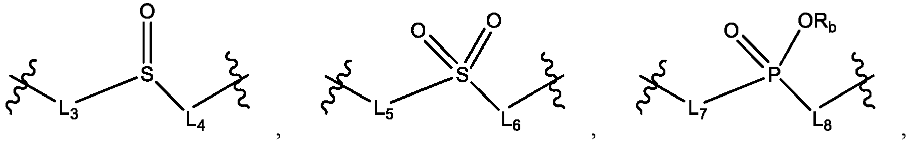 or 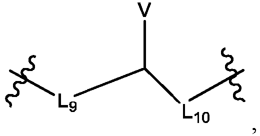 wherein each variable is as defined in formula (I).

8. The use according to claim 1, wherein X is 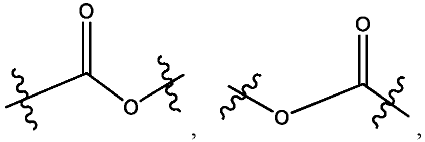 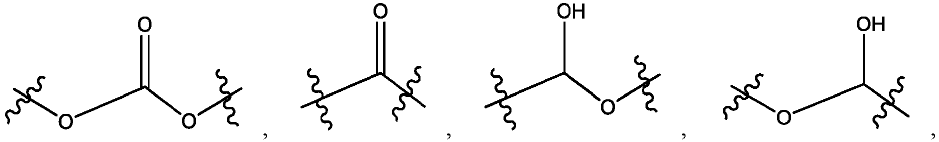 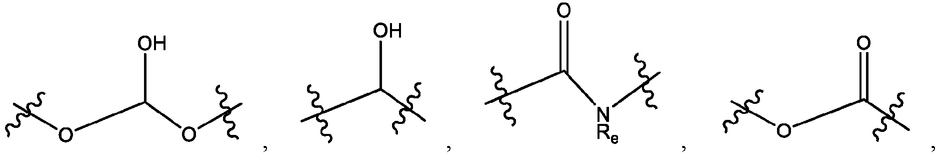 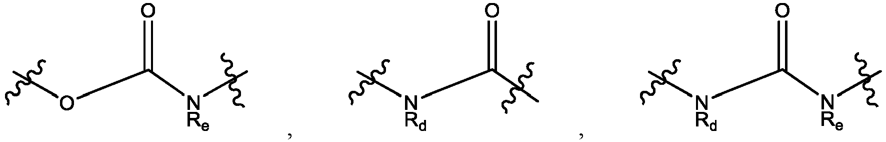 or 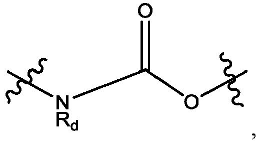 wherein R_{d} and Rₑ are as defined in formula (I).

9. The use according to claim 1, wherein Y and Z are both S; or Y is S and Z is O; or Y is O and Z is S; or Y and Z are both O.

10. The use according to claim 1, wherein each R₂ is independently at each occurrence a single bond, or a C₁₋₆ divalent aliphatic group, or a C₁₋₆ divalent heteroaliphatic group.

11. The use according to claim 1, wherein each R₃ is independently at each occurrence a single bond or a C₁₋₆ divalent aliphatic group.

12. The use according to claim 1, wherein each R₃ is independently at each occurrence a single bond, or 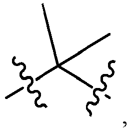 or methylene

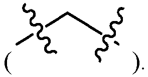

13. The use according to claim 1, wherein each R₄ is independently at each occurrence a hydrophobic group selected from long-chain alkyl, alkenyl, aryl, alkylaryl, arylalkyl, arylalkenyl, a cyclic group, an alicyclic group, and a polycyclic group, and optionally having at least one heteroatom selected from nitrogen, oxygen, and sulfur.

14. The use according to claim 1, wherein each R₄ is independently at each occurrence C₈-C₃₀ alkyl, C₈-C₃₀ alkenyl, or C₈-C₃₀ alkynyl; or each R₄ is independently at each occurrence - (CH₂CH₂O)ₘ-C₈-C₃₀ alkyl, -(CH₂CH₂O)_{y}-C₈-C₃₀ alkenyl, or -(CH₂CH₂O)_{y}-C₈-C₃₀ alkynyl, in which y is 0, or 1, or 2, for example, R₄ is -CH₂CH₂O-C₈H₁₇, -CH₂CH₂O-C₁₀H₂₁, -CH₂CH₂O-C₁₂H₂₅, -CH₂CH₂O-C₁₄H₂₉, or -CH₂CH₂O-C₁₅H₃₁.

15. The use according to claim 1, wherein A₁ and A₃ are each independently a single bond or a divalent aliphatic group, and A₂ is a divalent aliphatic group or a divalent heteroaliphatic group.

16. The use according to claim 1, wherein the lipid compound of formula (I) is 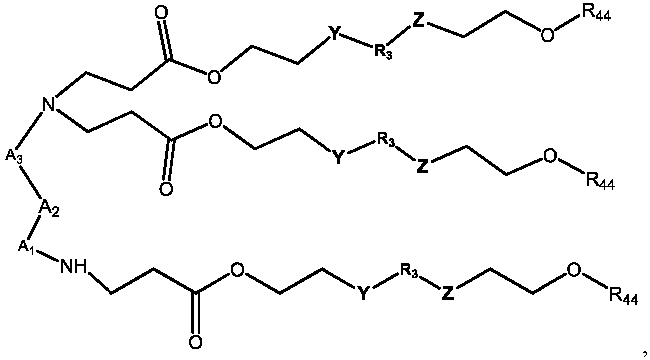 or 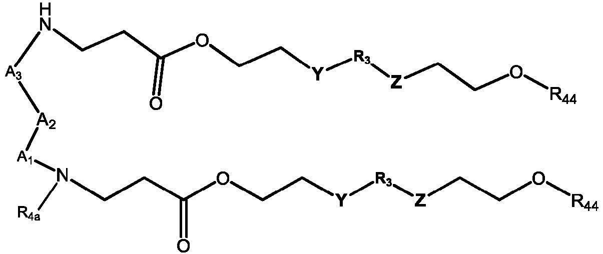 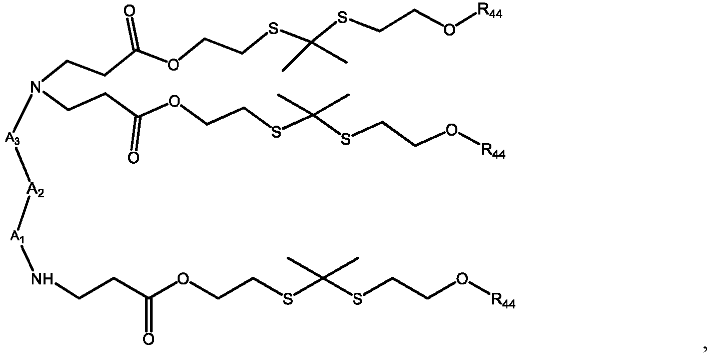 or 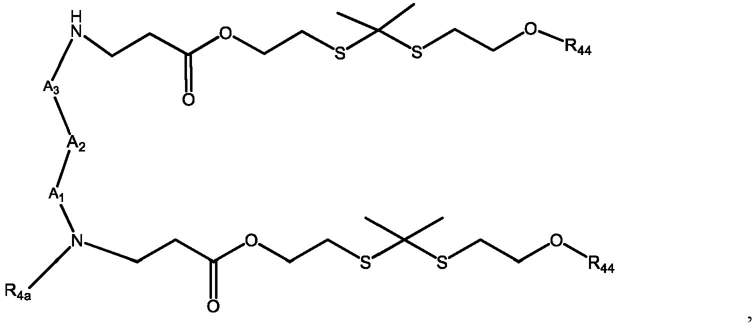 wherein A₁, A₂, A₃, and R₄ₐ are as defined in formula (I), and -(CH₂CH₂O)-R₄₄ is R₄ as defined in formula (I); Y and Z are both S; or Y is S and Z is O; or Y is O and Z is S; or Y and Z are both O; and each R₃ is independently at each occurrence a single bond, or 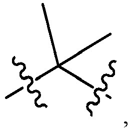 or methylene

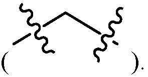

17. The use according to claim 16, wherein A₂ is a divalent cyclic heteroaliphatic group, and A₁ and A₃ are each independently C₁₋₄ divalent alkyl.

18. The use according to claim 1, wherein the lipid compound of formula (I) is 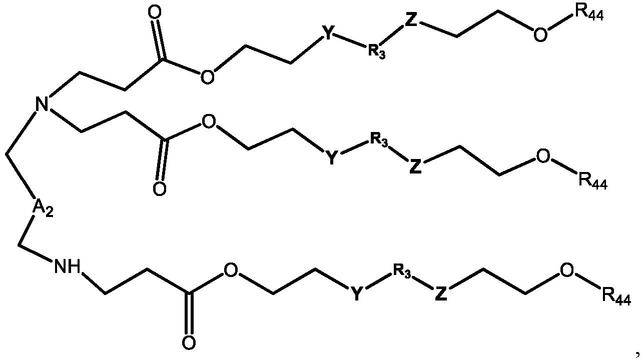 or 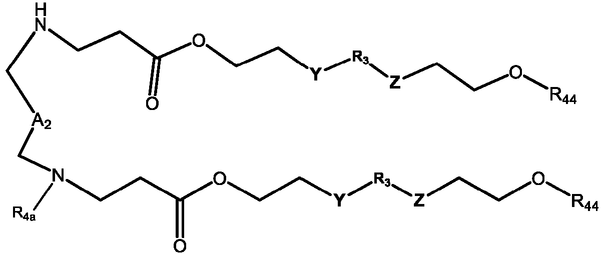 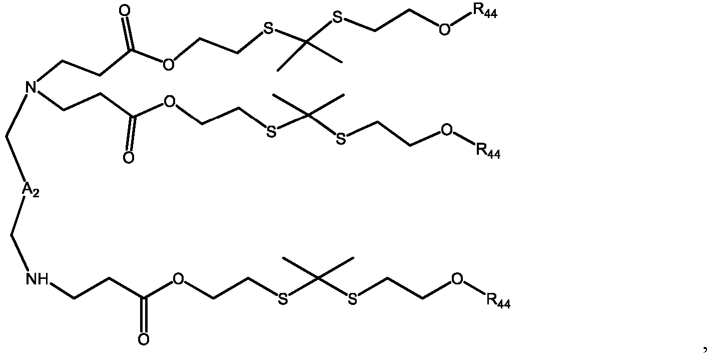 or 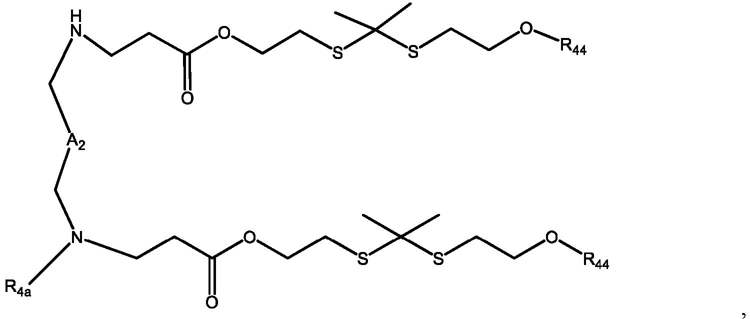 wherein A₂ and R₄ₐ are as defined in formula (I), and -(CH₂CH₂O)-R₄₄ is R₄ as defined in formula (I); Y and Z are both S; or Y is S and Z is O; or Y is O and Z is S; or Y and Z are both O; and each R₃ is independently at each occurrence a single bond, or 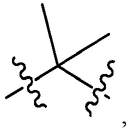 or methylene

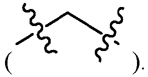

19. The use according to claim 1, wherein the lipid compound is 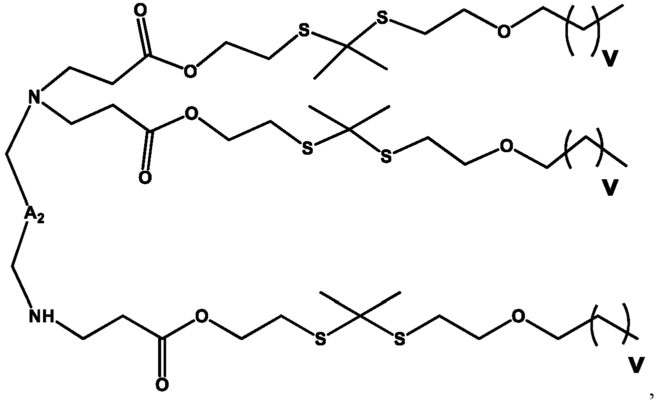 or 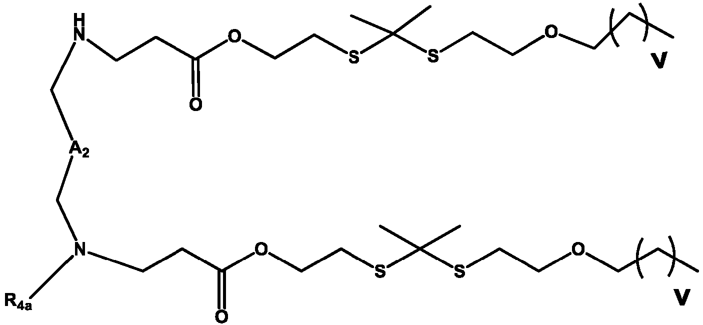 wherein v is 6-28; and A₂ and R₄ₐ are as defined in formula (I); or 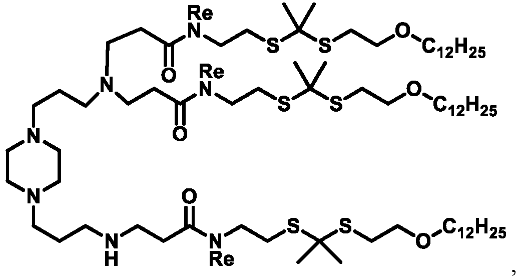 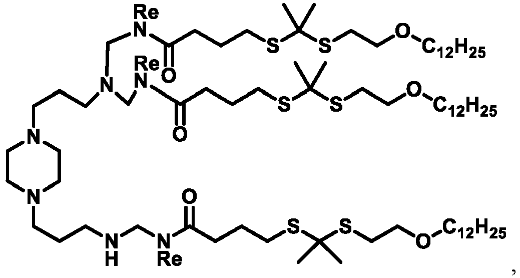 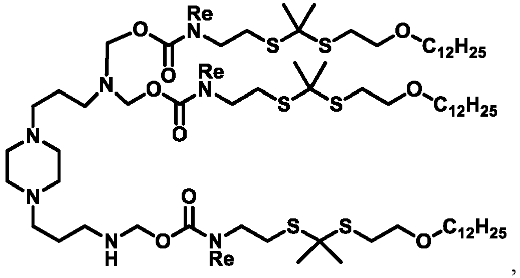 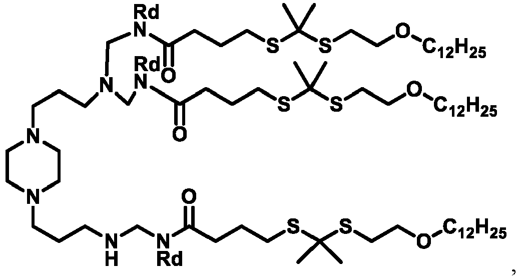 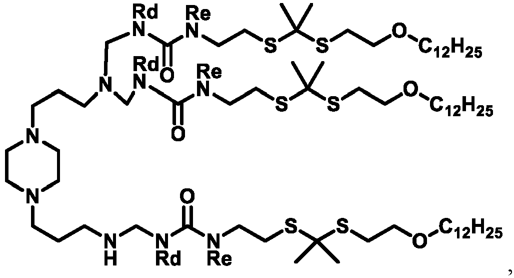 or 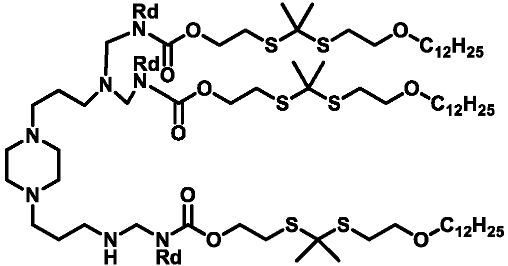 wherein R_{d} and Rₑ are as defined in formula (I).

20. The use according to claim 1, wherein the lipid compound is

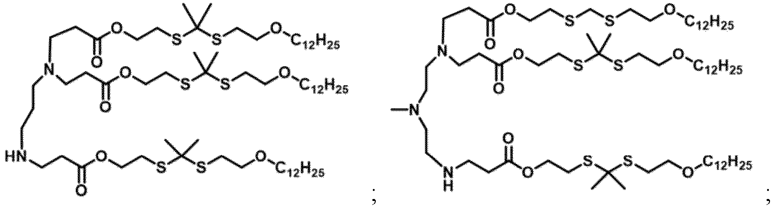

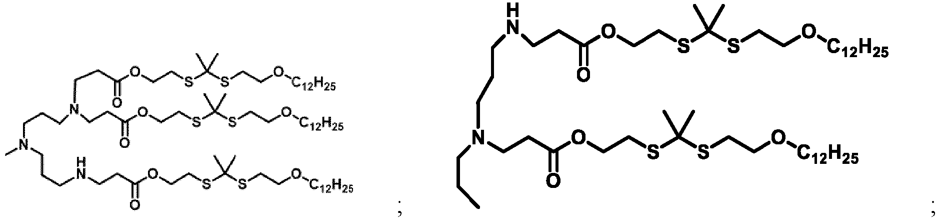

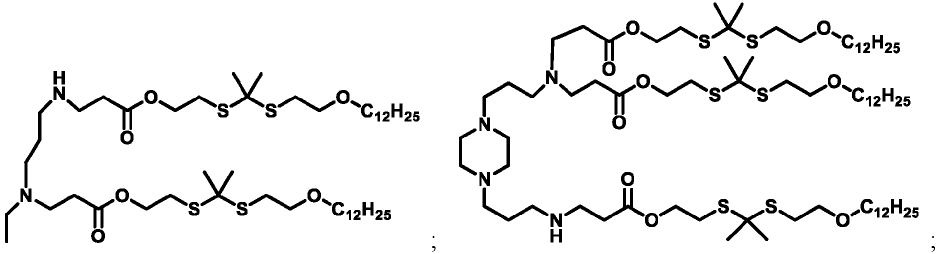

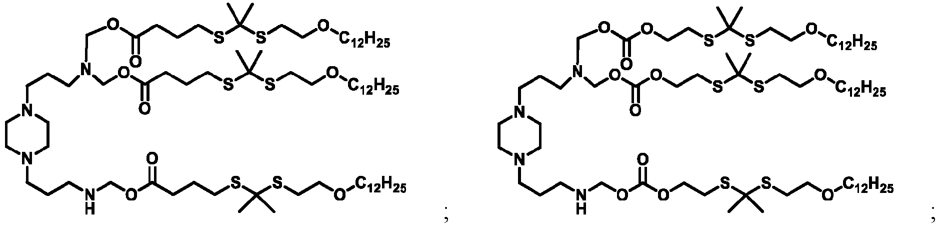

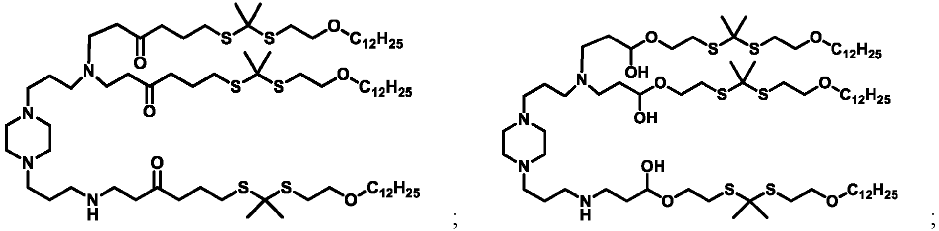

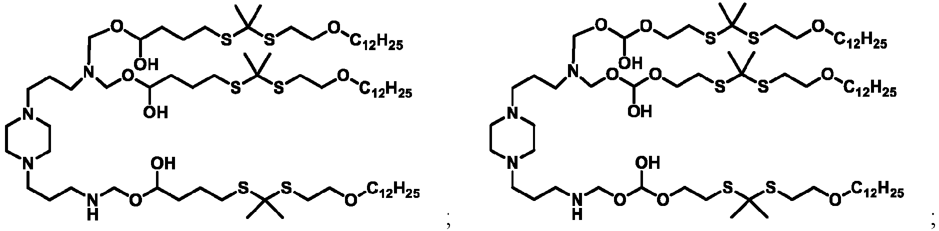

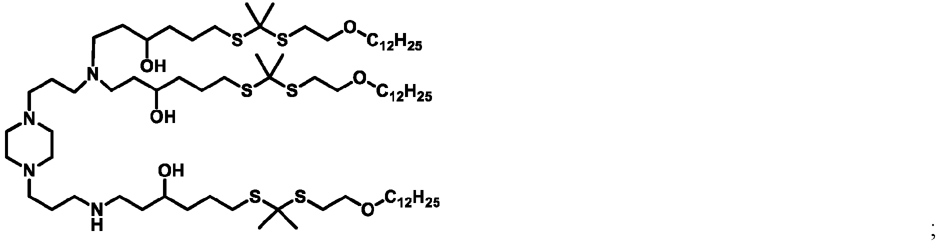

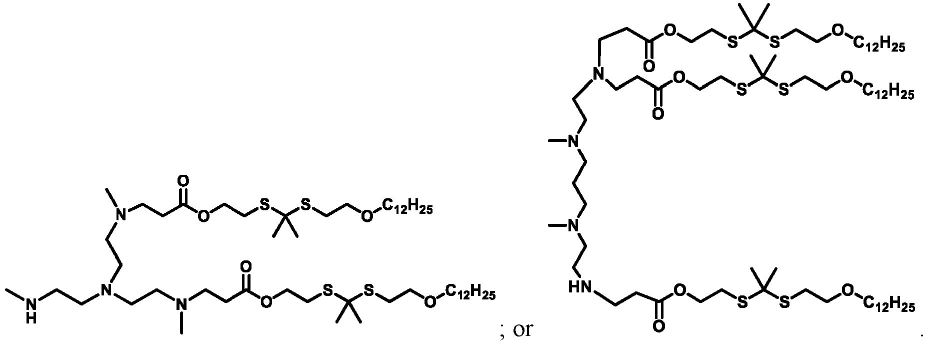

21. The use according to claim 1, wherein the target cell is a cancer cell, a cell infected by a pathogen, or a cell mediating a disease.

22. The method according to claim 1, wherein the nucleic acid is selected from an oligonucleotide, an aptamer, a single-stranded DNA, a double-stranded DNA, a plasmid DNA, a short isomer, an antisense molecule, a small interfering RNA (siRNA), an asymmetric interfering RNA (aiRNA), a microRNA (miRNA), a dsRNA (double-stranded RNA), an shRNA (small/short hairpin RNA), a transfer RNA (tRNA), a messenger RNA (mRNA), a small activating RNA, and a circRNA.

23. A lipid compound of formula (I), 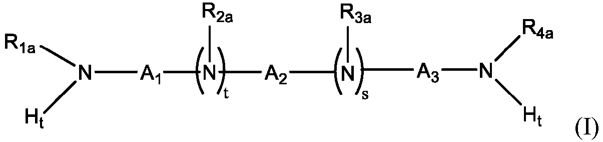 wherein
R₁ₐ, R₂ₐ, R₃ₐ, and R₄ₐ are each independently H, a monovalent aliphatic group, a monovalent heteroaliphatic group, a monovalent aromatic group, a monovalent heteroaromatic group, or Ht;
t and s are each independently 0 or 1, and when t or s is zero, it means that the part is directly a single bond;
A₁, A₂, and A₃ are each independently a single bond, a divalent aliphatic group, a divalent heteroaliphatic group, a divalent aromatic group, or a divalent heteroaromatic group, or a combination of two of the above;
each Ht is independently at each occurrence -R₁-X-R₂-Y-R₃-Z-R₄, wherein
each R₁ is independently at each occurrence a divalent aliphatic group, a divalent heteroaliphatic group, a divalent aromatic group, or a divalent heteroaromatic group;
each X is independently at each occurrence 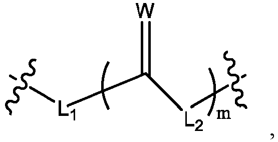 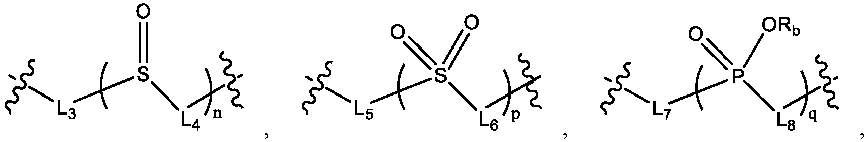
or 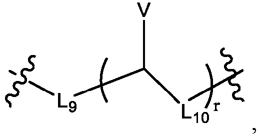 wherein
m, n, p, q, and r are each independently 1-6;
W is O, S, or NR_{c};
L₁, L₃, L₅, L₇, and L₉ are directly connected to R₁ or R₂ and are each independently a single bond, O, S, or NR_{d};
L₂, L₄, L₆, L₈, and L₁₀ are each independently a bond, O, S, or NRₑ;
V is an aliphatic group, OR_{f}, SR_{g}, or NRₕRᵢ,
wherein R_{b}, R_{c}, R_{d}, Rₑ, R_{f}, R_{g}, Rₕ, and Rⱼ are each independently H, OH, an oxyaliphatic group, a monovalent aliphatic group, a monovalent heteroaliphatic group, a monovalent aromatic group, or a monovalent heteroaromatic group;
Y and Z are each independently at each occurrence S or O;
each R₂ is independently at each occurrence a single bond, a divalent aliphatic group, a divalent heteroaliphatic group, a divalent aromatic group, or a divalent heteroaromatic group;
each R₃ is independently at each occurrence a single bond, a divalent aliphatic group, a divalent heteroaliphatic group, a divalent aromatic group, or a divalent heteroaromatic group;
each R₄ is independently at each occurrence a hydrophobic group;
and N atoms in the backbone structure of formula (I) are optionally cationized;
provided that at least one of R₁ₐ and R₄ₐ is hydrogen; and
provided that the compound is not one of the following compounds:

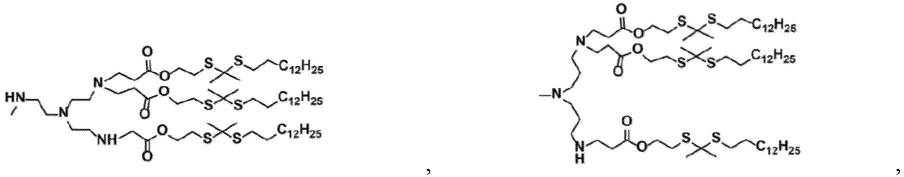

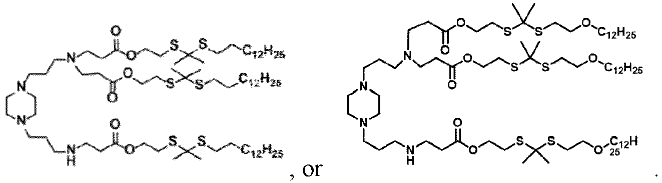

24. The lipid compound according to claim 23, wherein the lipid compound is in an ionizable form, thereby forming the following structures: 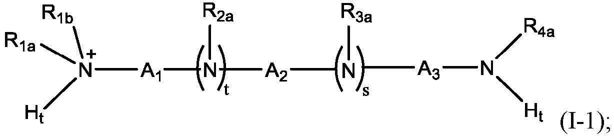 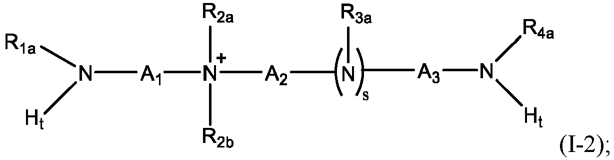 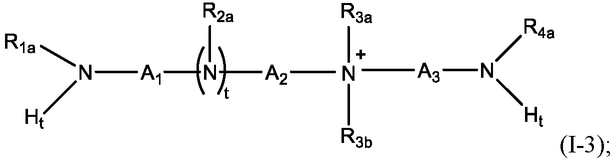 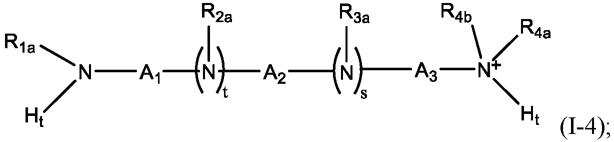 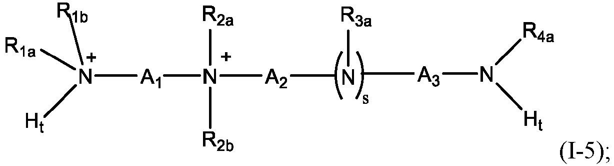 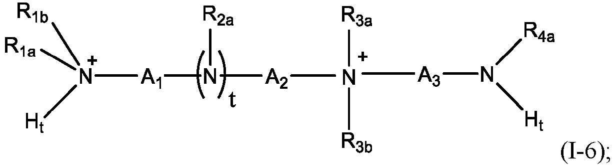 and 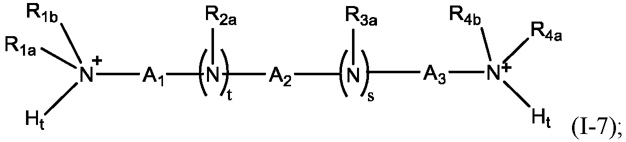 and contains corresponding counter ions, wherein R_{1b}, R_{2b}, R_{3b}, and R_{4b} are each independently hydrogen, a monovalent aliphatic group, a monovalent heteroaliphatic group, a monovalent aromatic group, a monovalent heteroaromatic group, or Ht, and the remaining variables are as defined in formula (I).

25. The lipid compound according to claim 23, wherein R₁ₐ or R₄ₐ is hydrogen; or R₁ₐ and R₄ₐ are each hydrogen.

26. The lipid compound according to claim 23, wherein t and s are both 0; or t is 0 and s is 1; or t is 1 and s is 0; preferably, t and s are both 0; R₁ₐ is Ht and R₄ₐ is hydrogen; or t and s are both 0; and R₁ₐ is hydrogen and R₄ₐ is Ht.

27. The lipid compound according to claim 23, wherein the fragment 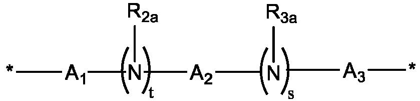 is
• A₁-A₃, wherein A₁ and A₃ are each independently a C₁-C₆ divalent aliphatic group (e.g., a C₁-C₄ divalent aliphatic group, e.g., a C₁-C₆ divalent alkyl, preferably a C₁-C₄ divalent alkyl); or
• A₁-(NR₂ₐ)-A₃, wherein A₁ and A₃ are each independently a C₁-C₆ divalent aliphatic group (e.g., a C₁-C₄ divalent aliphatic group, e.g., a C₁-C₆ divalent alkyl, preferably a C₁-C₄ divalent alkyl), and R₂ₐ is hydrogen, a monovalent aliphatic group, or a monovalent heteroaliphatic group; preferably hydrogen, a monovalent C₁₋₆ alkyl, or a monovalent C₁₋₆ heteroalkyl, e.g., amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆ alkylthio C₁₋₆ alkyl, or mercapto C₁₋₆ alkyl; or
• A₁-(NR₂ₐ)-A₂-(NR_{2b})-A₃, wherein A₁, A₂, and A₃ are each independently a C₁-C₆ divalent aliphatic group (e.g., a C₁-C₄ divalent aliphatic group, e.g., a C₁-C₆ divalent alkyl, preferably a C₁-C₄ divalent alkyl); and R₂ₐ and R_{2b} are each independently hydrogen, a monovalent aliphatic group, or a monovalent heteroaliphatic group; preferably hydrogen, a monovalent C₁₋₆ alkyl, or a monovalent C₁₋₆ heteroalkyl, e.g., amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆ alkylthio C₁₋₆ alkyl, or mercapto C₁₋₆ alkyl; or
• A₁-A₂-A₃, wherein A₁ and A₃ are each independently a C₁-C₆ divalent aliphatic group (e.g., a C₁-C₄ divalent aliphatic group, e.g., a C₁-C₆ divalent alkyl, preferably a C₁-C₄ divalent alkyl); and A₂ is a divalent cyclic heteroaliphatic group, such as a 3- to 7-membered monocyclic heteroaliphatic group containing 1-3 heteroatoms selected from oxygen, nitrogen and sulfur, preferably divalent piperazinyl;
wherein the asterisk * represents the connection of the terminal N atom in formula (I) or (I-1) to (I-7);
preferably, the fragment 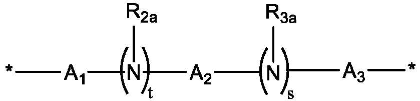 is
• -(CH₂)ₘ₁-, wherein m1 is an integer of 2-12, preferably 2-10 or 3-8; or an integer of 3; or
• -(CH₂)ₘ₂-(NR₂ₐ)-(CH₂)ₘ₃-, wherein m2 and m3 are each independently an integer of 1-6; preferably an integer of 2-6, or 2-4, or 2, or 3; R₂ₐ is hydrogen, a monovalent C₁₋₆ alkyl, amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆ alkylthio C₁₋₆ alkyl, or mercapto C₁₋₆ alkyl; or
• -(CH₂)ₘ₂-(NR₂ₐ)-(CH₂)ₘ₄-(NR_{2b})-(CH₂)ₘ₃, wherein m2, m3, and m4 are each independently an integer of 1-6; preferably an integer of 2-6, or 2-4, or 2, or 3; R₂ₐ and R_{2b} are each independently hydrogen, a monovalent C₁₋₆ alkyl, amino C₁₋₆ alkyl, di(C₁₋₆ alkyl)aminoalkyl, mono(C₁₋₆ alkyl)aminoalkyl, C₁₋₆ alkoxy C₁₋₆ alkyl, hydroxy C₁₋₆ alkyl, C₁₋₆ alkylthio C₁₋₆ alkyl, or mercapto C₁₋₆ alkyl; or
• -(CH₂)ₘ₂-A₂-(CH₂)ₘ₃, wherein m2 and m3 are each independently an integer of 1-6; preferably an integer of 2-6, or 2-4, or 2, or 3; and A₂ is a divalent cyclic heteroaliphatic group, such as a 3- to 7-membered monocyclic heteroaliphatic group containing 1-3 heteroatoms selected from oxygen, nitrogen and sulfur, preferably divalent piperazinyl;
wherein the asterisk * represents the connection of the terminal N atom in formula (I) or (I-1) to (I-7).

28. The lipid compound according to claim 23, wherein R₁ is a C₁₋₆ divalent aliphatic group or a C₁₋₆ divalent heteroaliphatic group.

29. The lipid compound according to claim 23, wherein X is 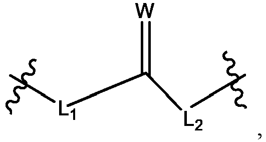 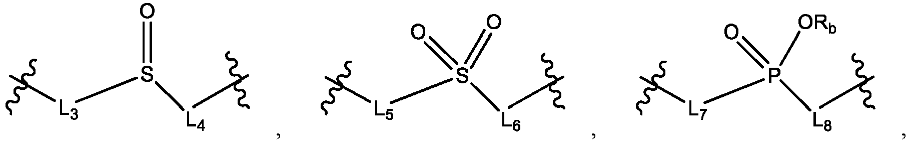 or 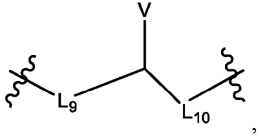 wherein each variable is as defined in formula (I).

30. The lipid compound according to claim 23, wherein X is 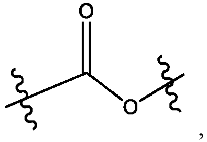 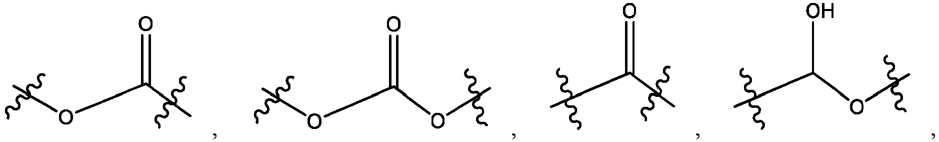 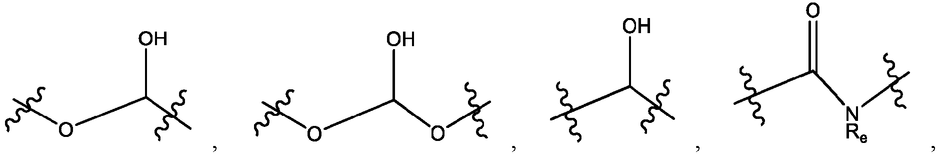 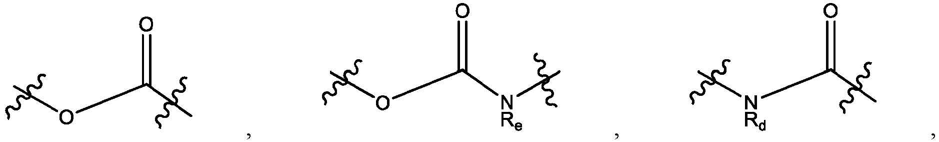 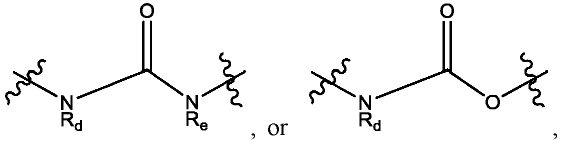 wherein R_{d} and Rₑ are as defined in formula (I).

31. The lipid compound according to claim 23, wherein Y and Z are both S; or Y is S and Z is O; or Y is O and Z is S; or Y and Z are both O.

32. The lipid compound according to claim 23, wherein each R₂ is independently at each occurrence a single bond, or a C₁₋₆ divalent aliphatic group, or a C₁₋₆ divalent heteroaliphatic group.

33. The lipid compound according to claim 23, wherein each R₃ is independently at each occurrence a single bond or a C₁₋₆ divalent aliphatic group.

34. The lipid compound according to claim 23, wherein each R4 is independently at each occurrence a hydrophobic group selected from long-chain alkyl, alkenyl, aryl, alkylaryl, arylalkyl, arylalkenyl, a cyclic group, an alicyclic group, and a polycyclic group, and optionally having at least one heteroatom selected from nitrogen, oxygen and sulfur.

35. The lipid compound according to claim 34, wherein each R₄ is independently at each occurrence C₈-C₃₀ alkyl, C₈-C₃₀ alkenyl, or C₈-C₃₀ alkynyl; or each R₄ is independently at each occurrence -(CH₂CH₂O)ₘ-C₈-C₃₀ alkyl, -(CH₂CH₂O)_{y}-C₈-C₃₀ alkenyl, or -(CH₂CH₂O)_{y}-C₈-C₃₀ alkynyl, in which y is 0, or 1, or 2, for example, R₄ is -CH₂CH₂O-C₈H₁₇, -CH₂CH₂O-C₁₀H₂₁, - CH₂CH₂O-C₁₂H₂₅, -CH₂CH₂O-C₁₄H₂₉, or -CH₂CH₂O-C₁₅H₃₁.

36. The lipid compound according to claim 23, wherein A₁ and A₃ are each independently a single bond or a divalent aliphatic group, and A₂ is a divalent aliphatic group or a divalent heteroaliphatic group.

37. The lipid compound according to claim 23, wherein the lipid compound is 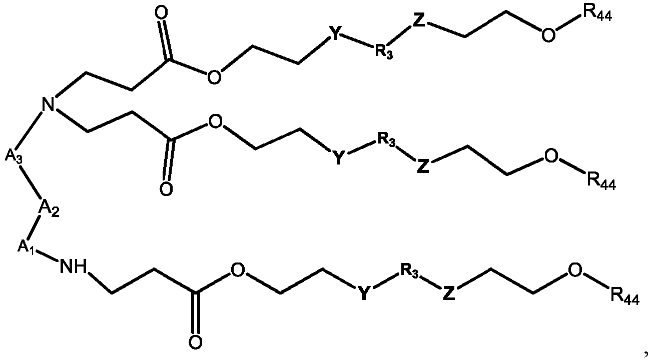 or 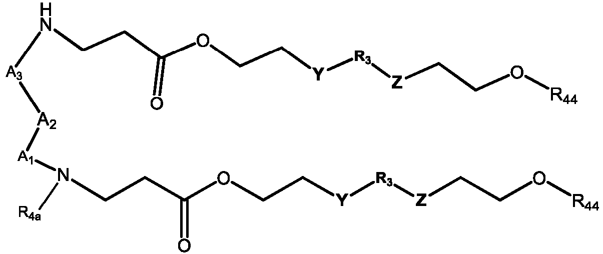 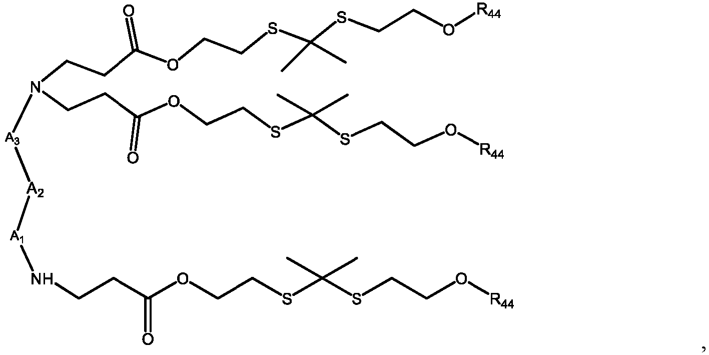 or 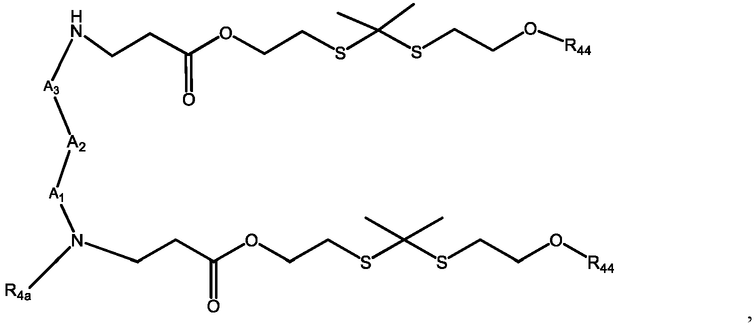 wherein A₁, A₂, A₃, and R₄ₐ are as defined in formula (I), and -(CH₂CH₂O)-R₄₄ is R₄ as defined in formula (I); Y and Z are both S; or Y is S and Z is O; or Y is O and Z is S; or Y and Z are both O; and each R₃ is independently at each occurrence a single bond, or 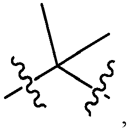 or methylene

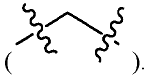

38. The lipid compound according to claim 37, wherein A₂ is divalent piperazinyl, and A₁ and A₃ are each independently C₁₋₄ divalent alkyl.

39. The lipid compound according to claim 23, wherein the lipid compound is 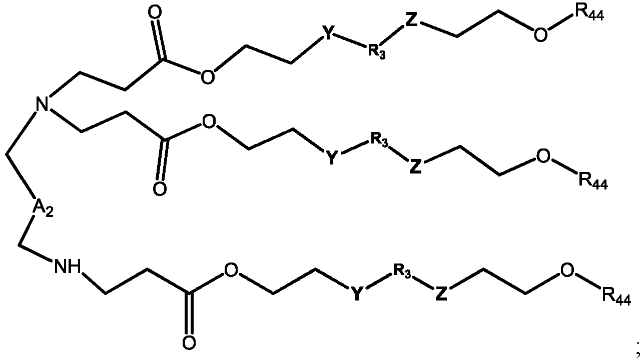 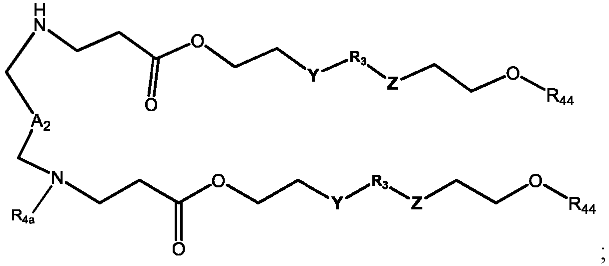 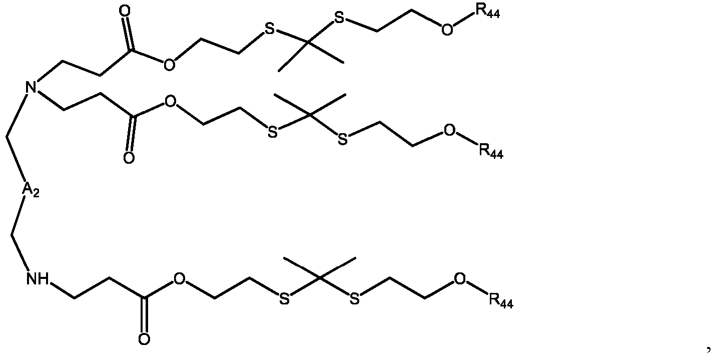 or 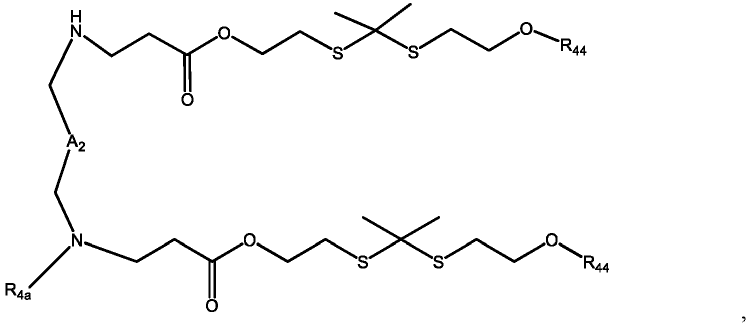 wherein A₂ and R₄ₐ are as defined in formula (I), and -(CH₂CH₂O)-R₄₄ is R₄ as defined in formula (I); Y and Z are both S; or Y is S and Z is O; or Y is O and Z is S; or Y and Z are both O; and each R₃ is independently at each occurrence a single bond, or 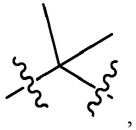 or methylene

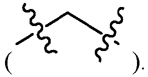

40. The lipid compound according to claim 39, wherein A₂ is divalent piperazinyl, and A₁ and A₃ are each independently C₁₋₄ divalent alkyl.

41. The lipid compound according to claim 1, wherein the lipid compound is 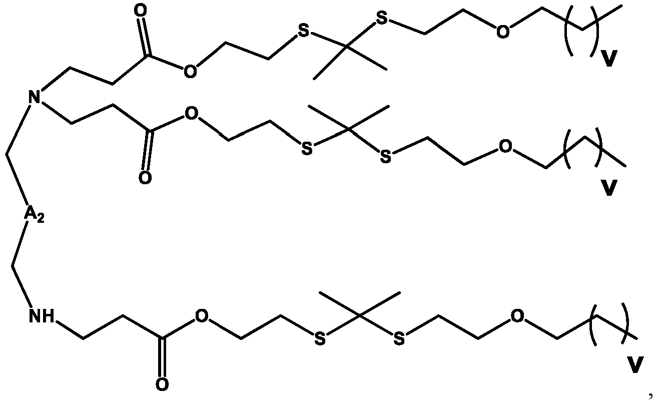 or 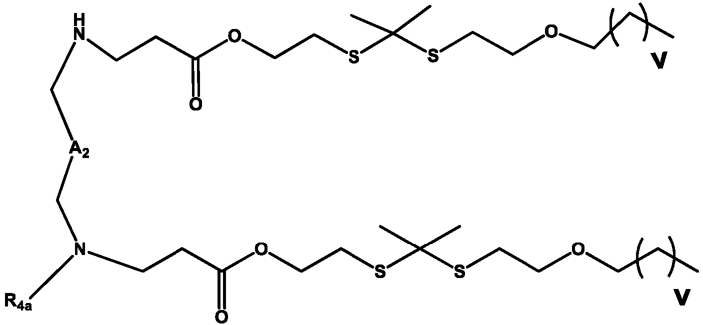 wherein v is 6-28; and A₂ and R₄ₐ are as defined in formula (I); or 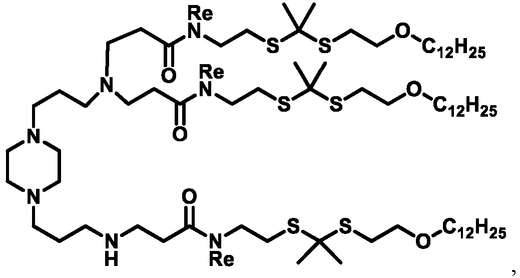 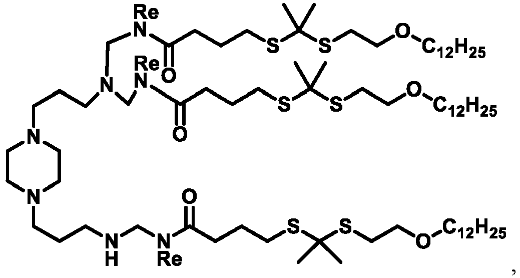 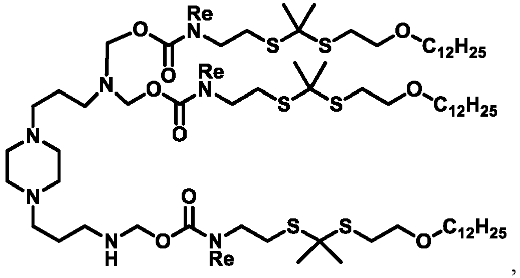 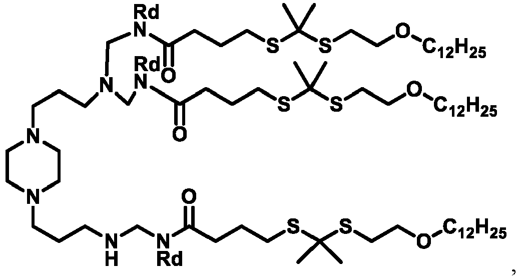 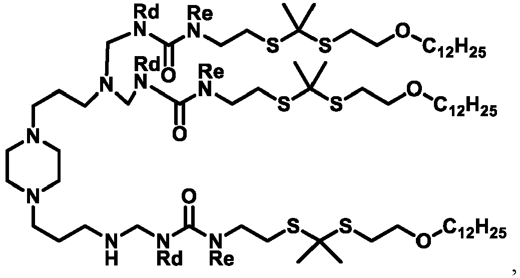 or 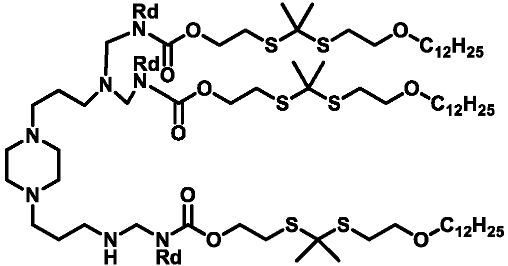 wherein R_{d} and Rₑ are as defined in formula (I).

42. The lipid compound according to claim 23, wherein the lipid compound is

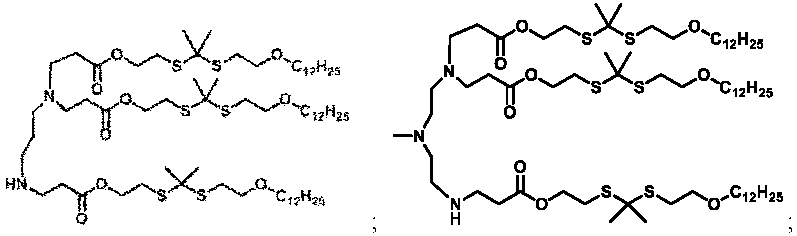

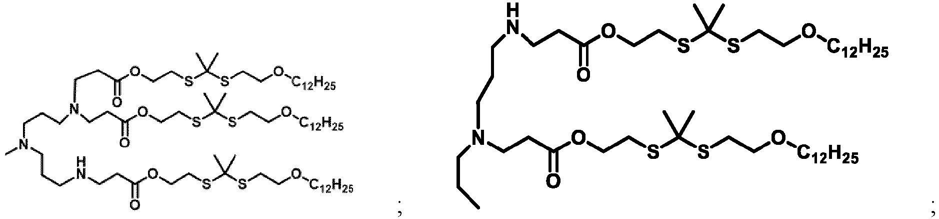

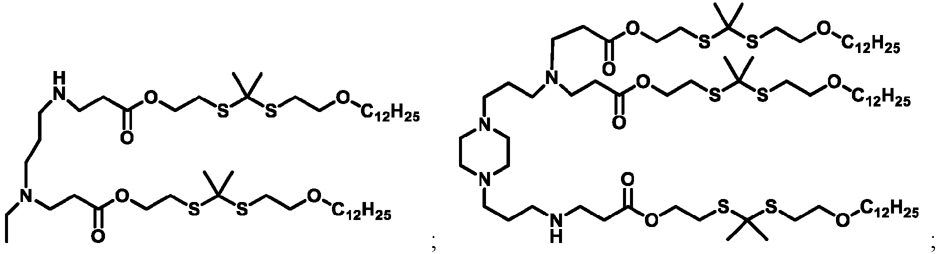

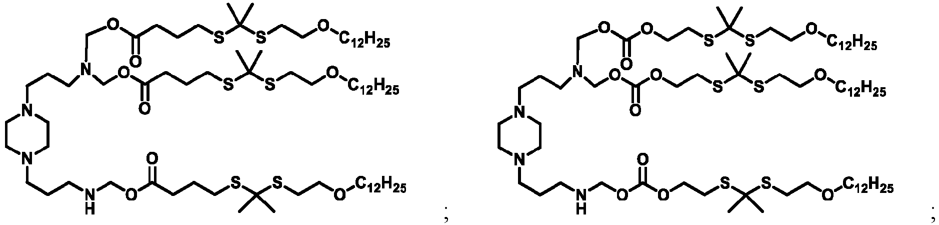

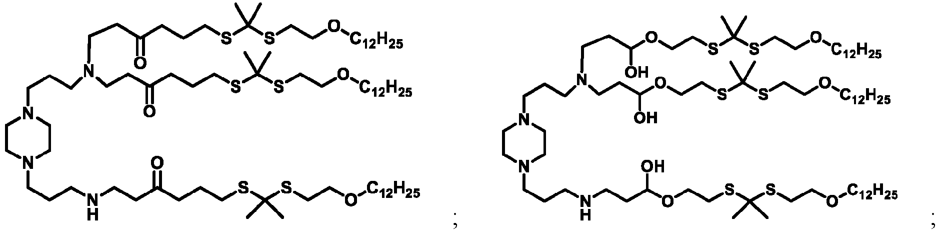

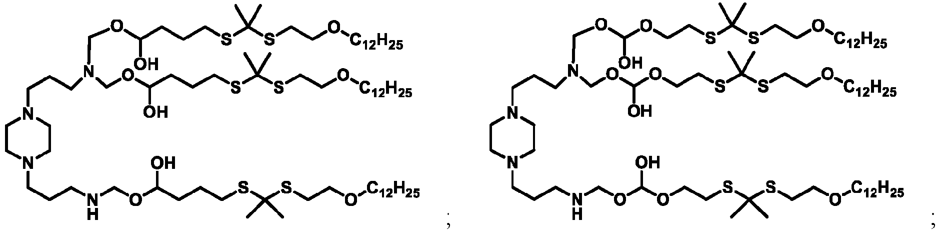

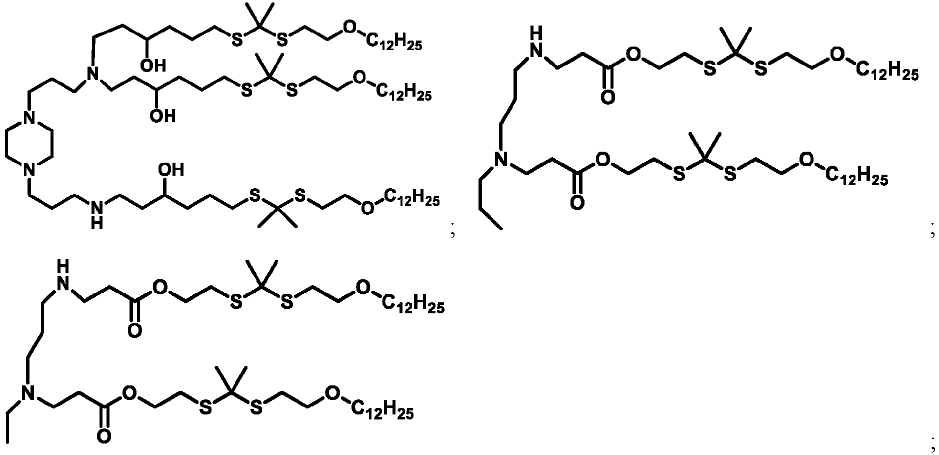

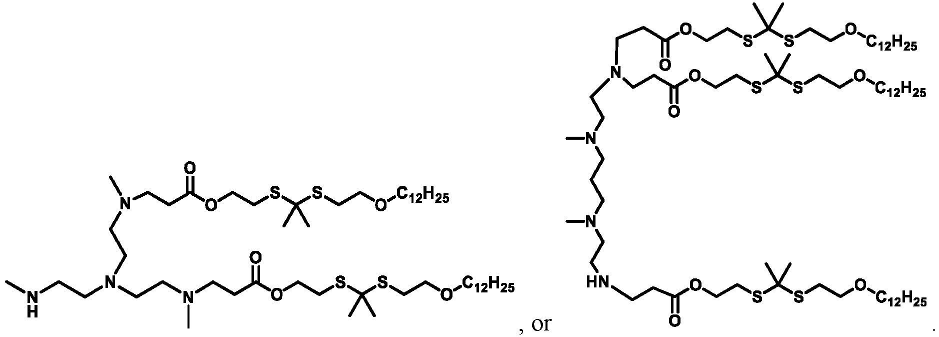

43. A method for preparing a lipid compound of formula (I) in which R₁ is ethylene and X is - C(O)-O-, the method comprising:
mixing and reacting an acrylic compound of formula (II), i.e., CH₂=CH-**X**-R₂-**Y**-R₃-**Z**-R₄ (II), in which each variable is as defined in formula (I), with a hydrophilic amine, wherein the hydrophilic amine contains at least one primary amino group (-NH₂) or at least two secondary amino groups (-NH-), and the molar ratio of the acrylic compound of formula (II) to the hydrophilic amine is less than the number of moles of free hydrogen atoms on amino groups in the hydrophilic amine, but greater than 1; and then optionally purifying the product by chromatography to obtain the desired lipid compound of formula (I),
wherein the variables R₂, **X, Y,** R₃, **Z**, and R₄ are as defined in formula (I).

44. A method for preparing a lipid compound of formula (I) in which R₁ is ethylene and X is - C(O)-O-, the method comprising:
mixing and reacting a ketal-containing acrylate CH₂=CH-C(O)O-R₂-**Y**-R₃-**Z**-R₄, in which each variable is as defined in formula (I), with a hydrophilic amine, wherein the hydrophilic amine contains at least one primary amino group (-NH₂) or at least two secondary amino groups (-NH-), and the molar ratio of the ketal-containing acrylate to the hydrophilic amine is less than the number of moles of free hydrogen atoms on amino groups in the hydrophilic amine, but greater than 1; and then optionally purifying the product by chromatography to obtain the desired lipid compound of formula (I),
wherein the variables R₂, **Y,** R₃, **Z**, and R₄ are as defined in formula (I).

45. The method according to claim 43 or 44, wherein the molar ratio of the ketal-containing acrylate to the hydrophilic amine is 1 or 2 less than the number of moles of free hydrogen atoms on amino groups in the hydrophilic amine, but greater than 1.

46. The method according to claim 43 or 44, wherein the molar ratio of the ketal-containing acrylate to a hydrophilic amine containing 2 free hydrogen atoms on amino groups is 1.1-1.8; or the molar ratio of the ketal-containing acrylate to a hydrophilic amine containing 3 free hydrogen atoms on amino groups is 1.1-2.7; or the molar ratio of the ketal-containing acrylate to a hydrophilic amine containing 4 free hydrogen atoms on amino groups is 1.1-3.5.

47. The method according to claim 43 or 44, wherein the pKa value of the hydrophilic amine is greater than 4.

48. The method according to claim 43 or 44, wherein the hydrophilic amine in method (a) or the unprotected hydrophilic amine in method (b) is selected from 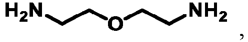 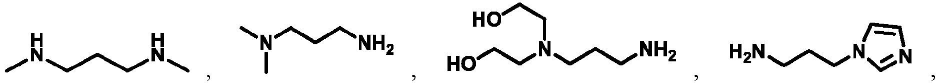 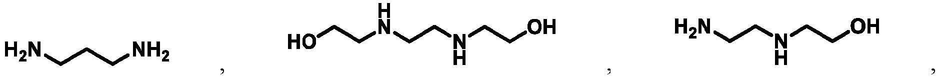 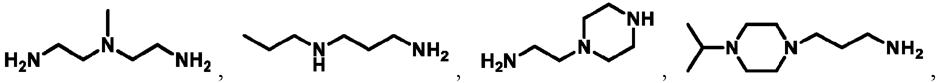 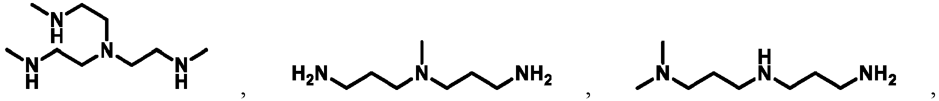 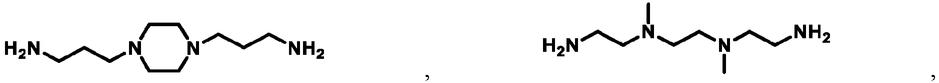 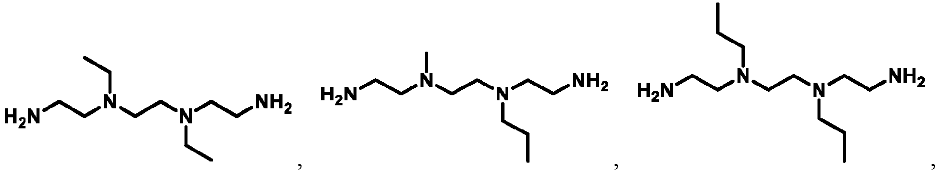 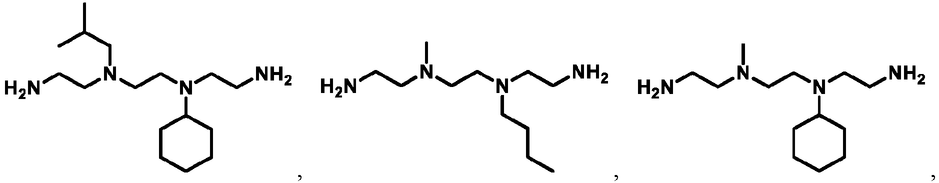 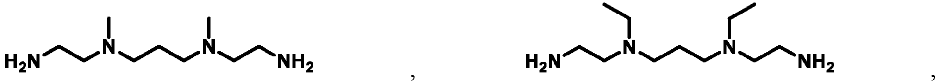 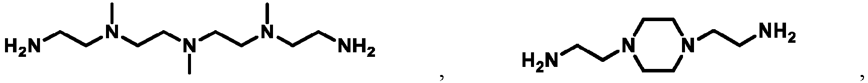 or

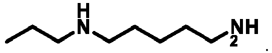

49. The method according to claim 43 or 44, wherein the ketal-containing acrylate CH2=CH-C(O)O-R2-Y-R3-Z-R4 is 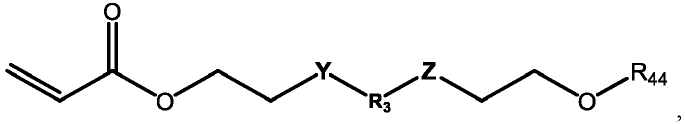 and the remaining variables are as defined in formula (I).

50. The method according to claim 43 or 44, wherein the ketal-containing acrylate is 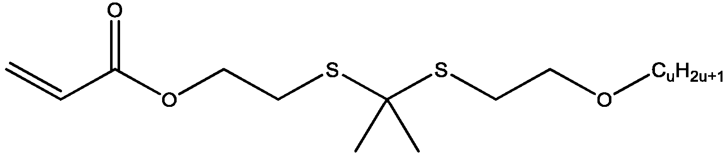 or 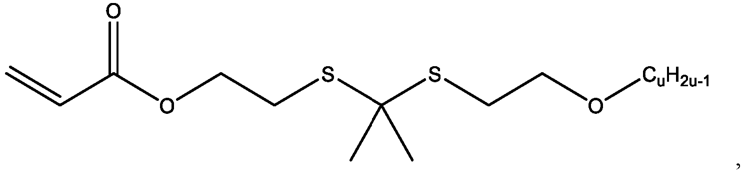 wherein CᵤH₂ᵤ₊₁ represents alkyl, CᵤH₂ᵤ₋₁ represents alkenyl, and u is 8-30.

51. A pharmaceutical composition comprising a pharmaceutical carrier and a nanocomplex, wherein the nanocomplex comprises the lipid compound according to any one of claims 23-42 and a pharmaceutically active molecule.

52. The pharmaceutical composition according to claim 51, wherein the pharmaceutically active molecule is a small molecule drug, a protein, a peptide, a nucleic acid, a saccharide, or a combination thereof.

53. The pharmaceutical composition according to claim 51, wherein the nanocomplex further comprises other lipids.
